# EUROPÄISCHE PATENTSCHRIFT

(11) **EP 1 250 430 B1**
(45) Veröffentlichungstag und Bekanntmachung des Hinweises auf die Patenterteilung: **11.05.2005**
(21) Anmeldenummer: 01903644.1
(22) Anmeldetag: 12.01.2001
(51) Int. Cl.: C12N 15/12, C12N 5/10, C12N 15/11, C07K 14/47, C12Q 1/68, A61K 48/00, A61K 35/14, C12N 15/85

(54) **REGULATORISCHE SEQUENZ ZUR SPEZIFISCHEN EXPRESSION IN DENDRITISCHEN ZELLEN UND DEREN ANWENDUNGEN**
REGULATORY SEQUENCE FOR THE SPECIFIC EXPRESSION IN DENDRITIC CELLS AND USES THEREOF
SEQUENCE REGULATRICE POUR L'EXPRESSION SPECIFIQUE DANS DES CELLULES DENDRITIQUES ET SES UTILISATIONS

(30) Priorität: 13.01.2000 DE 10001169; 02.03.2000 DE 10010188
(43) Veröffentlichungstag der Anmeldung: 23.10.2002
(73) Patentinhaber: Reske-Kunz, Angelika, 55270 Essenheim (DE); Ross, Xiaolan, 55131 Mainz (DE); Ross, Ralf, 55131 Mainz (DE); Bros, Matthias, 55131 Mainz (DE)
(72) Erfinder: Reske-Kunz, Angelika, 55270 Essenheim (DE); Ross, Xiaolan, 55131 Mainz (DE); Ross, Ralf, 55131 Mainz (DE); Bros, Matthias, 55131 Mainz (DE)
(74) Vertreter: VOSSIUS & PARTNER
(86) Internationale Anmeldenummer: PCT/EP2001/000362
(87) Internationale Veröffentlichungsnummer: WO 2001/051631

(56) Entgegenhaltungen:
- DATABASE EMBL [Online] Accession Number AC006483, 1. Februar 1999 (1999-02-01) WATERSTON R. H. : "Homo sapiens BAC clone CTB-161C1." XP002173793
- DATABASE EMBL [Online] Accession Number U90355, 1997 TUBB B. E. ET AL.: "Mus musculus fascin (Fscn1) gene." XP002173794 in der Anmeldung erwähnt
- SONDERBYE L. ET AL.: "SELECTIVE EXPRESSION OF HUMAN FASCIN (P55) BY DENDRITIC LEUKOCYTES" ADVANCES IN EXPERIMENTAL MEDICINE AND BIOLOGY, Bd. 417, 1997, Seiten 41-46, XP001010579 ISSN: 0065-2598
- MORITA A. ET AL.: "ROLES OF LANGERHANS CELLS IN GENETIC IMMUNIZATION" JOURNAL OF DERMATOLOGICAL SCIENCE, Bd. 20, Nr. 1, Mai 1999 (1999-05), Seiten 39-52, XP000982406 ISSN: 0923-1811
- RIEDINGER M. ET AL.: "TARGETED EXPRESSION OF MHC CLASS II GENES TO DENDRITIC CELLS IN VIVO" IMMUNOLOGY LETTERS, Bd. 57, Nr. 1 - 03, 1997, Seiten 155-158, XP000982398 ISSN: 0165-2478
- SCHOOTEN VAN W. C. A. ET AL.: "BIOLOGICAL PROPERTIES OF DENDRITIC CELLS: IMPLICATIONS TO THEIR USEIN THE TREATMENT OF CANCER" MOLECULAR MEDICINE TODAY, Bd. 3, Nr. 6, 1997, Seiten 254-260, XP001010564 ISSN: 1357-4310

## Beschreibung

Die vorliegende Erfindung betrifft regulatorische Sequenzen, die eine spezifische Expression in dendritischen Zellen vermitteln, rekombinante Nucleinsäuremoleküle, Vektoren, Wirtszellen sowie Verfahren zur Herstellung der Wirtszellen. Ferner betrifft die Erfindung Verwendungen der regulatorischen Sequenzen sowie Arzneimittel.

Dendritische Zellen (DC) spielen als Antigen-präsentierende Zellen (APC) eine Schlüsselrolle bei der Mobilisierung der spezifischen Immunabwehr. Als einzige Zellen sind dendritische Zellen in der Lage, ruhende, für die Abwehr in Bereitschaft stehende, sogenannte naive T-Lymphocyten effizient zu aktivieren. Dabei können sie sowohl CD4⁺ T-Helferzellen als auch CD8⁺ cytotoxische T-Zellen induzieren. Dendritische Zellen steuern daher gleichermaßen die humorale Antikörperdominierte wie die zelluläre Immunantwort. Für eine effiziente Immunabwehr von bakteriellen, viralen und parasitären Krankheitserregern sowie von Tumorzellen sind dendritische Zellen unabdingbar. Auch an pathologischen Entgleisungen des Immunsystems wie Autoimmunkrankheiten und Allergien sind dendritische Zellen ursächlich beteiligt. Es ist daher ein herausragendes medizinisches Anliegen, die Funktionen von dendritischen Zellen therapeutisch nutzbar zu machen.
Dendritische Zellen in unterschiedlichen Differenzierungsstadien besitzen unterschiedliche Funktionskompetenz. Drei diskrete Reifephasen lassen sich aus funktioneller Sicht definieren. 1.) Eine Überwachungsfunktion wird von jungen, unreifen dendritischen Zellen ausgeübt. Junge dendritische Zellen sind in nahezu allen Organen des Körpers und besonders in den Epithelien, die den Körper nach außen abgrenzen, als Wächterzellen strategisch positioniert. Ihre Aufgabe besteht in der Aufnahme von Fremdstoffen (Antigenen) in löslicher oder partikulärer Form, in der Antigenprozessierung und der Peptidbeladung von MHC-Molekülen. 2.) Als Peptidtransporter wandern die dendritischen Zellen in der sich anschließenden migratorischen Phase in die drainierenden Lymphknoten, um sich dort in den T-Zellarealen anzusiedeln. 3.) In diesem Bereich kommt das immunogene Potential der dendritischen Zellen zum Tragen. Die dendritischen Zellen präsentieren das prozessierte Peptid zusammen mit MHC-Molekülen T-Lymphocyten mit entsprechender Rezeptorspezifität. Dabei handelt es sich um naive T-Lymphocyten, d.h. um Zellen, die zuvor noch nicht mit dem Antigen in Kontakt gekommen waren und daher eine hohe Aktivierungsschwelle besitzen. Die direkte Zell-Zell-Interaktion zwischen den dendritischen Zellen und den T-Lymphocyten resultiert in der Aktivierung, der Expansion und damit der funktionellen Rekrutierung der peptidspezifischen T-Lymphocyten.
Dendritische Zellen sind aufgrund ihrer zentralen Funktion bei der Vermittlung von Immunantworten ein wesentlicher Ansatzpunkt für Schutzimpfungen, ein Aspekt, der im folgenden etwas ausführlicher diskutiert werden soll.
Die klassische Schutzimpfung, die vor allem auf der Verabreichung von attenuierten Erregern basiert, ist mit einer Reihe von Nachteilen verbunden. Dies betrifft beispielsweise das potentielle Risiko einer Infektion, falls der Erreger seine Human-Pathogenität wiedererlangen sollte (McKee, Am.J.Trop.Med.Hyg. 36 (1987), 435-442), Probleme in Zusammenhang mit der Produktion und Aufbewahrung der Vakzine (Rabinovich, Science 265 (1994), 1401; Fynan, Proc. Nat. Acad, Sci. USA 90 (1993), 11478) sowie eine mögliche Behinderung der Antigenpräsentation durch immunmodulatorische Eigenschaften des Erregers (Levine et al., in New Generation Vaccine, Woodrow, G. and Levine, M.M., eds., Marcel Decker, New York, (1990), 269-287). In Anbetracht dessen, kristallisiert sich immer mehr heraus, daß die Technik der DNA-Vakzinierung hierzu eine vielversprechende Alternative darstellt. Bei der DNA-Vakzinierung, die erstmals von Ulmer (Science 259 (1993), 1745-1749) gezeigt wurde, wird "nackte" DNA in den Körper injiziert oder anderweitig appliziert, die eine protektive. Immunantwort auslöst (für eine Übersicht siehe Lai und Bennet, Crit. Rev. Immunol. 18 (1998), 449-484). Die DNA enthält Sequenzen, die für ein Antigen eines Erregers oder Tumors oder für ein Allergen codieren und die unter der Regulation eines ubiquitär funktionierenden Promotors stehen. Die applizierte DNA wird von Zellen des umliegenden Gewebes aufgenommen, das Antigen wird exprimiert und über MHC-Proteine an der Zelloberfläche präsentiert.
Im Verlauf der Entwicklung von DNA-Vakzinierungsstrategien stellte sich zunehmend heraus, daß hierbei eine gezielte Adressierung von dendritischen Zellen vielversprechende Vorteile bieten könnte. Das läßt sich beispielsweise aus Immunisierungsversuchen ableiten, bei denen dendritische Zellen in vitro mit Antigen beladen und reinjeziert wurden (WO 94/02156). So erzeugten etwa murine dendritische Zellen, die in vitro mit synthetischen Peptiden (Mayordomo et al., Nat. Med. 1 (1995), 1297-1302; Celluzzi et al., J. Exp. Med. (1996), 283-87), Säureeluierten Peptiden von Tumorzellen (Zitvogel et al., J. Exp. Med. 183 (1996), 87-97) und sogar intakten Tumorproteinen (Paglia et al., J. Exp. Med. 183 (1996), 317-22) gepulst wurden, in vivo protektive Antworten cytotoxischer T-Lymphocyten (CTL) gegen den Tumor. Humane dendritische Zellen, die aus peripheren, mononucleären Blutzellen (PBMC) gesunder Donoren gezogen wurden, können ebenfalls CTL-Antworten gegen Tumor-Antigene erzeugen (van Elsas et al., Eur. J. Immunol. 26 (1996), 1683-1689; Falo et al., Nature Med. 1 (1995), 649-653) und in vitro kultivierte dendritische Zellen wurden bereits erfolgreich in klinischen Studien für die Therapie von Tumoren eingesetzt (Hsu et al., Nature Med. 2 (1996), 540-544). Auch dendritische Zellen und Zellinien dieses Zelltyps, die in vitro mit Antigen-codierenden Expressionsplasmiden transfiziert wurden, können nach adoptivem Transfer in vivo Antigen-spezifische Immunantworten induzieren (Manickan et al., J. Leucocyte Biol. 61 (1997), 125-132; Timares-Lebow et al., J. Invest. Dermatol. 109 (1997), 266; Tüting et al., Eur. J. Immunol. 27 (1997), 2702-2707). Dendritische Zellen, in die mRNA aus Tumorzellen eingeführt wurde, wurden ebenfalls bereits erfolgreich für Vakzinierungen eingesetzt (Boczkowski et al., J. Exp. Med. 184 (1996), 465-72). Aus diesen und anderen Beobachtungen lassen sich folgende Vorteile von DNA-Vakzinierungen unter gezielter Antigenexpression in dendritischen Zellen ableiten. So ist beispielsweise bekannt, daß die Präsentation eines Antigens durch nicht professionelle APCs, welche nicht die notwendigen costimulatorischen Signale für eine effiziente T-Zell Stimulation zur Verfügung stellen, zu einer mangelhaften Reaktivität bzw. Anergie der T-Zellen führt. Die Restriktion der Expression des Antigens auf reife dendritische Zellen könnte daher zu einer verstärkten Immunantwort führen. Desweiteren könnte es beispielsweise zusätzlich zur Expression des Antigens möglich sein, durch Co-Transfektion weitere, immunmodulatorische Proteine in DC zu exprimieren und dadurch regulatorisch in die Immunantwort einzugreifen. Die Beschränkung der Expression dieser Mediatoren (z.B. Cytokine, costimulatorische Membranproteine) auf transfizierte dendritische Zellen würde es erlauben, sehr spezifisch die erwünschte Immunantwort zu beeinflussen, ohne die bei systemischer Gabe dieser Mediatoren zu erwartenden Nebenwirkungen befürchten zu müssen.
Daraus ergibt sich ein Bedarf an Promotoren oder regulatorischen Sequenzen, die eine spezifische Expression in dendritischen Zellen vermitteln. Damit ließen sich beispielsweise DNA-Vakzinierungskonstrukte herstellen, die nach breitgestreuter Verabreichung, wie beispielsweise durch intramuskuläre Injektion oder bioballistischen Transfer in die Haut, nur in dendritischen Zellen, die in den peripheren Geweben relativ schwach repräsentiert sind, das Antigen oder immunmodulatorische Proteine exprimieren. Es besteht zumindest ein Bedarf an Promotoren oder regulatorischen Sequenzen, die in solchen Geweben eine spezifische Expression für dendritische Zellen vermitteln, in denen die Antigenbeladung der dendritischen Zellen stattfindet. Dies sind in erster Linie das Biutsystem, das Hautgewebe, mukosale Gewebe und die Muskeln. Weitere mögliche Anwendungen solcher Promotoren oder regulatorischer Sequenzen sind in vitro-Transfektionen von heterogenen Zellpopulationen, wobei das Antigen exklusiv in reifen dendritischen Zellen exprimiert würde.

Im Stand der Technik ist bereits ein Promotor mit Spezifität für dendritische Zellen beschrieben worden (Brocker, J. Exp. Med. 185 (1997), 541-550). Dieser Promotor wurde aus dem Maus-CD11c-Gen isoliert und konnte bei einem Transgen (MHC-Klasse II-I-E-Protein) in transgenen Mäusen eine spezifische Expression vermitteln. Jedoch erfüllt der in dieser Veröffentlichung präsentierte Promotor in einigen Punkten nicht die Anforderungen des oben skizzierten Promotors zur spezifischen Expression in dendritischen Zellen.
So konnte die Spezifität für dendritische Zellen nur für Milz- und Thymusgewebe gezeigt werden. Dagegen exprimierte dieser Promotor das Transgen auch in einem Teil der peritonealen Makrophagen. Desweiteren ist bekannt, daß CD11c nur in einer Subpopulation der dendritischen Zellen exprimiert wird (Rich et al., Poster Nr. D6, 5^{th} International Symposium on Dendritic Cells in Fundamental and Clinical Immunology, Pittsburgh, Penn. U.S.A., 23.-28. Sept. 1998). Schwerwiegender ist jedoch, daß die gezeigte Promotoraktivität sich auf das Maussystem bezieht. Da der gesuchte Promotor für humanmedizinische Anwendungen zur Verfügung stehen soll, bietet der CD11c-Promotor keine Lösung der Problemstellung, da nicht zu erwarten ist, daß der Promotor im Mensch dieselbe Gewebe- oder Zelltypspezifität besitzt wie in der Maus. Ein Beispiel, das eine unterschiedliche Zelltypspezifität zwischen Maus und Mensch zeigt, ist das Chemokingen DC/B-CK. In der Maus wird dieses Gen nur in dendritischen Zellen und aktivierten B-Zellen aber nicht in Makrophagen exprimiert (Ross, J. Invest. Dermatol. 113 (1999), 991-998). Dagegen ist das homologe menschliche Gen in Makrophagen exprimiert (Godiska, J. Exp. Med. 185 (1997), 1595).

Ferner wurde von Tubb et al. bereits eine ca. 3,5 kb lange genomische 5'-flankierende Sequenz des Maus-Fascingens unter der Genbank/EMBL-Hinterlegungsnummer U90355 veröffentlicht. Zwar deutet einiges darauf hin, daß Fascin vorwiegend in dendritischen Zellen exprimiert wird, jedoch ist fraglich, ob diese Sequenz einen Promotor enthält, der in der Lage ist, einem Transgen eine spezifische Expression in dendritischen Zellen zu vermitteln. Bis auf die rohe Sequenz liegen jedenfalls keinerlei Informationen hierzu vor. Zusammenfassend läßt sich also feststellen, daß bisher kein Promotor vorliegt, der im Menschen eine für dendritische Zellen spezifische Expression vermittelt.

Daher besteht nach wie vor der Bedarf an solchen Promotoren oder regulatorischen Sequenzen, um gezielte Immunisierungen oder Immuntherapien durchführen zu können, die auf dendritische Zellen fokussiert sind.

Der vorliegenden Erfindung liegt somit die Aufgabe zugrunde, eine regulatorische Sequenz bereitzustellen, die in Mensch eine spezifische Expression in dendritischen Zellen vermittelt.

Diese Aufgabe wird erfindungsgemäß durch die Bereitstellung der in den Patentansprüchen charakterisierten Ausführungsformen gelöst.

Somit betrifft die vorliegende Erfindung regulatorische Sequenzen ausgewählt aus der Gruppe bestehend aus:
(a) der regulatorischen Sequenz, die unter SEQ ID NO:72 von Positon 1 bis 3069 oder die unter SEQ ID NO:1 angegeben ist;
(b) der regulatorischen Sequenz, die in der Insertion des Klons DSM13274 enthalten ist und durch Amplifikation unter Verwendung eines Paars von Oligonucleotiden erhältlich ist, die beispielsweise die unter SEQ ID NO: 36 und 37 angegebenen Sequenzen besitzen;
(c) der regulatorischen Sequenz aus SEQ ID NO:72 von Position 1136 bis 3069, 1451 bis 3069, 1621 bis 3069, 1830 bis 3069, 2127 bis 3069, 2410 bis 3069 oder 2700 bis 3069 oder ausgewählt aus der Gruppe bestehend aus: SEQ ID NOs:2 bis 8;
(d) der regulatorischen Sequenz, die in der Insertion des Klons DSM13274 enthalten ist und durch Amplifikation unter Verwendung eines Paars von Oligonucleotiden erhältlich ist, wobei die Sequenzen der Oligonucleotide beispielsweise angegeben sind unter SEQ ID-Nummern, ausgewählt aus der Gruppe von Paaren bestehend aus: 38 und 37; 39 und 37; 40 und 37; 41 und 37; 42 und 37; 43 und 37; und 44 und 37;
(e) regulatorischen Sequenzen, die zumindest ein funktionaler Teil einer unter (a) bis (d) genannten Sequenz sind und eine für dendritische Zellen spezifische Expression bewirken; und
(f) regulatorischen Sequenzen, die mit der unter (a) angegebenen regulatorischen Sequenz hybridisieren, zu der unter (a) angegebenen Sequenz eine Sequenzidentität von mindestens 50% aufweisen und eine für dendritische Zellen spezifische Expression bewirken.

Die regulatorischen Sequenzen der vorliegenden Erfindung verleihen Nucleotidsequenzen, die von ihnen kontrolliert werden, eine für dendritische Zellen spezifische Expression.
Der Begriff "regulatorische Sequenz" bezieht sich auf Nucleotidsequenzen, die das Expressionslevel eines Gens beeinflussen, beispielsweise indem sie der Expression Gewebe- oder Zellspezifität verleihen. In diesem Sinne werden beispielsweise unter regulatorischen Sequenzen Elemente, im folgenden auch als regulatorische Elemente bezeichnet, verstanden, die einem Minimalpromotor zusätzliche Expressionseigenschaften verleihen, die über die Minimalpromotoren charakterisierende basale, konstitutive Expression hinausgehen. Der Begriff "Minimalpromotor" bezieht sich im Zusammenhang der Erfindung auf Nucleotidsequenzen, die für die Initiation der Transkription, d.h. für das Binden der RNA-Polymerase, notwendig sind, und beispielsweise die TATA-Box beinhalten. Ferner fallen unter den Begriff "regulatorische Sequenz" auch Sequenzen, die außerhalb des 5'-flankierenden Promotorbereichs liegen. Solche Sequenzen sind in beiden Orientierungen funktional und sind in der Position weniger festgelegt als Promotoren, vorzugsweise liegen sie innerhalb des Bereichs der im Rahmen der vorliegenden Erfindung offenbarten nicht-translatierten Sequenzen des humanen Fascingens (SEQ ID NO: 1 bis 20 oder die nicht-translatierten Sequenzen von SEQ ID NO:72). Zu solchen Sequenzelementen zählen Enhancer und Silencer, die die Expression eines Gens hoch- bzw. herunterregulieren können. Enhancer oder Silencer sind häufig in Introns oder im 3'-flankierenden Bereich eines Gens lokalisiert. Die regulatorische Sequenz kann auch ein Promotor sein, der im Sinne der Erfindung dadurch charakterisiert ist, daß er sämtliche Funktionen eines Promotors ausübt, d.h. Initiation der RNA-Polymerisation, Vermittlung einer bestimmten Expressionstärke und Regulation der Expression, vorzugsweise in Abhängigkeit des Zelltyps, besonders bevorzugt spezifisch für dendritische Zellen. Die in den SEQ ID NO: 1 bis 8 oder den oben genannten Abschnitten von SEQ ID NO:72 dargestellten Sequenzen sind regulatorische Sequenzen, die gleichzeitig der Definition eines Promotors entsprechen.

"Für dendritische Zellen spezifische Expression" bedeutet im Rahmen der vorliegenden Erfindung eine Expression ausschließlich in dendritischen Zellen, sofern Zellen des Hautgewebes, vorzugsweise der Epidermis, der mukosalen Gewebe, des Lymph- und Blutsystems und des Muskels betrachtet werden, vorzugsweise sofern solche Zellen mit einem Expressionskonstrukt transfiziert werden, das eine erfindungsgemäße regulatorische Sequenz enthält. Für Blutzellen aus Personen, die mit dem Epstein-Barr-Virus (EBV) infiziert sind, bezieht sich die für dendritische Zellen spezifische Expression zusätzlich auf B-Lymphocyten. Dabei können die erfindungsgemäßen regulatorischen Sequenzen sehr wohl auch in Zellen anderer Gewebetypen, wie z.B. Neuronen, Gliazellen oder Fibroblasten, eine Expression vermitteln, sofern sie in Haut, vorzugsweise in der Epidermis, in den mukosalen Geweben, im Blut und im Muskel spezifisch eine Expression nur in dendritischen Zellen gewährleisten. Vorzugsweise erfolgen Applikationen der erfindungsgemäßen regulatorischen Sequenzen so, daß eine Expression nur in dendritischen Zellen gewährleistet ist. Dies ist beispielsweise für die Haut, vorzugsweise für die Epidermis, für die muskosalen Gewebe, das Blut oder den Muskel der Fall.
"Dendritische Zellen" sind Antigen-präsentierende Zellen, die als einzige in der Lage sind, naive T-Zellen effizient zu aktivieren. Zu den dendritischen Zellen zählen auch die Langerhanszellen des Hautgewebes, welche unreife dendritische Zellen repräsentieren.
Die Spezifität der erfindungsgemäßen regulatorischen Sequenzen ist aus dem Expressionsverhalten des Fascingens, von dem die regulatorischen Sequenzen abgeleitet wurden, ersichtlich. So ist beispielsweise in der Haut, vorzugsweise der Epidermis, keine Fascinexpression detektiert worden, auch nicht in Langerhans-Zellen, da diese Zellen in der Haut unreif sind. Fascinexpression wurde allerdings in fortgeschritteneren Reifungsstadien der Langerhans-Zellen nachgewiesen (Ross, J. Immunol. 160 (1998), 3776-3782). In Blutzellen, zumindest von Personen, die nicht mit dem Epstein-Barr-Virus (EBV) infiziert sind, läßt sich Fascinexpression ausschließlich in dendritischen Zellen nachweisen (Mosialos, Am. J. Pathol. 148 (1996), 593-600). Zusätzlich wurde Fascinexpression in EBV-transfizierten B-Lymphocyten detektiert (Mosialos, J. Virol. 68 (1994), 7320). Im Muskel ist auf RNA-Ebene eine sehr geringe Fascin-Expression nachzuweisen, die auf die Präsenz von dendritischen Zellen zurückzuführen sein könnte (Mosialos, J. Virol. 68 (1994), 7320).

Die erfindungsgemäßen regulatorischen Sequenzen sind durch die unter SEQ ID NO: 1 bis 8 angegebenen Nucleotidsequenzen oder die oben genannten entsprechenden Abschnitte von SEQ ID NO:72 offenbart.

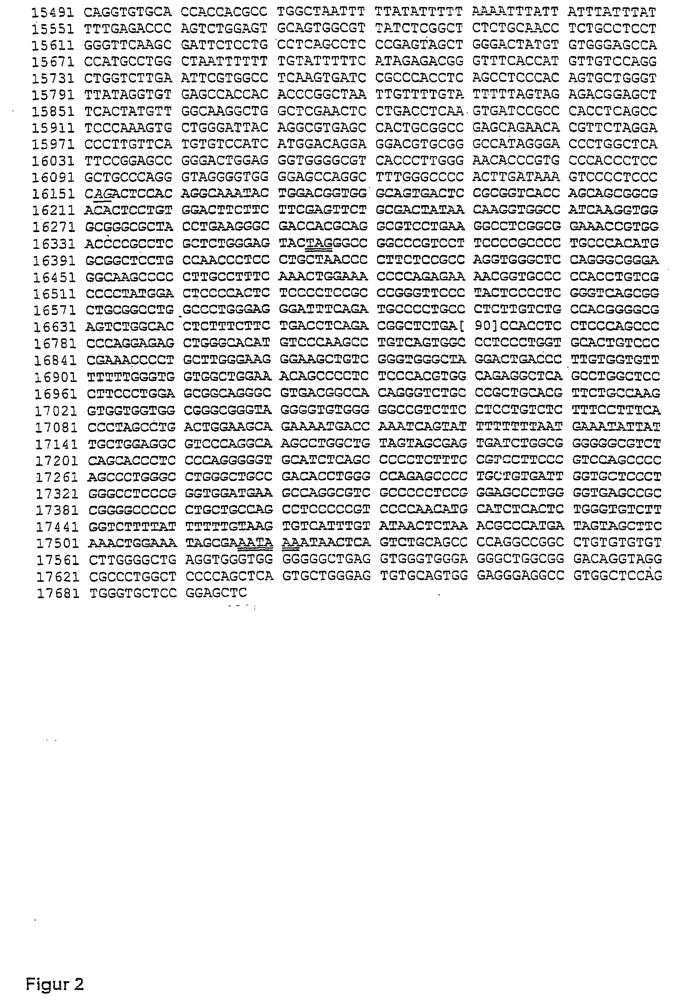

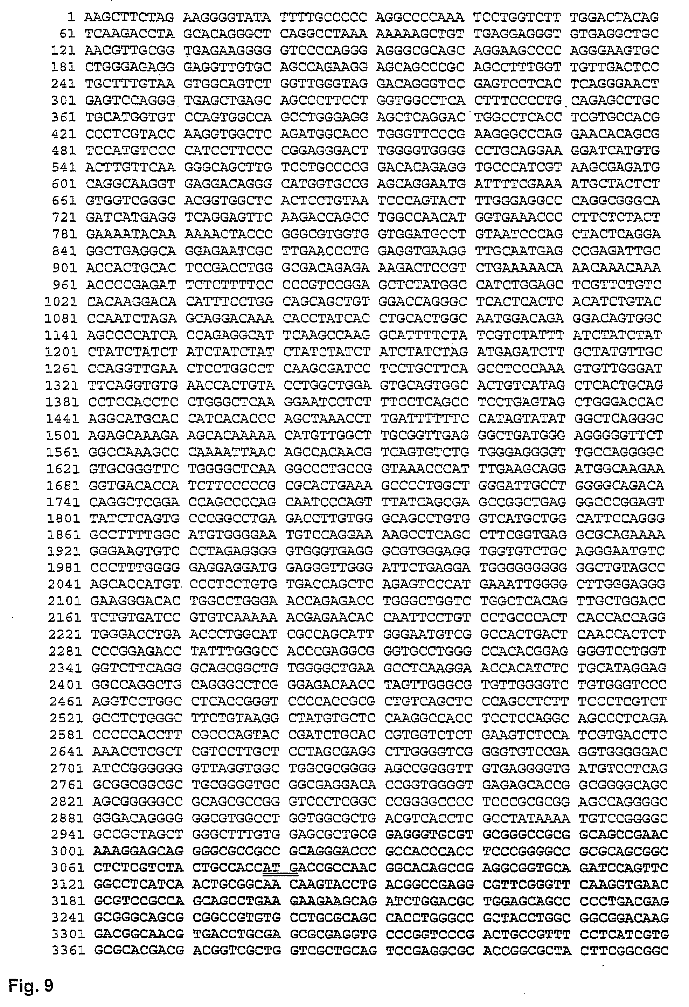

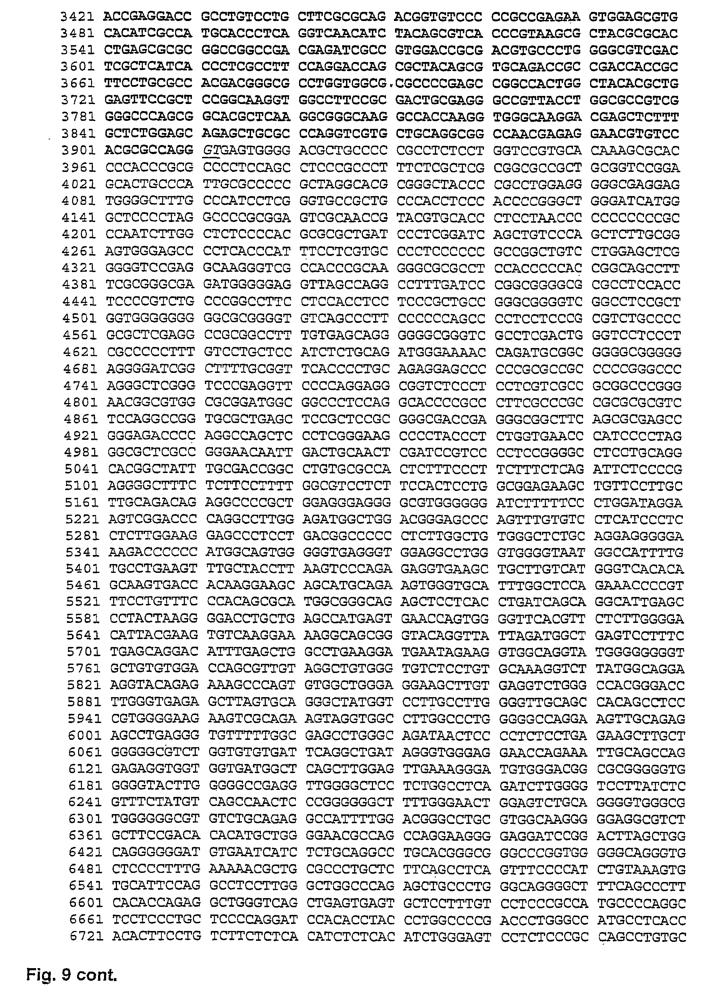

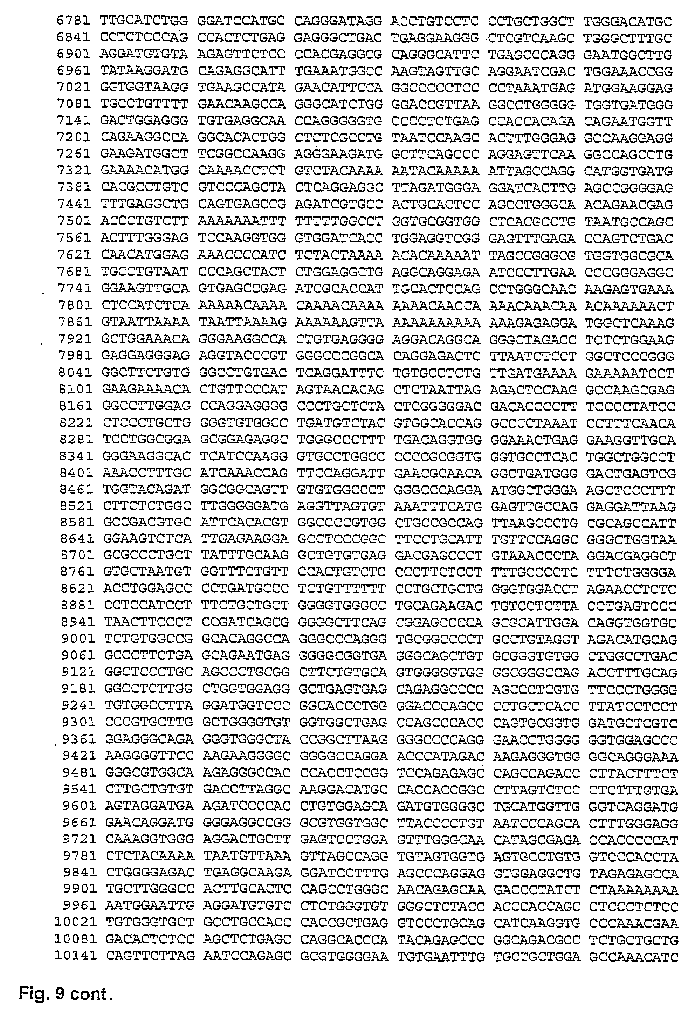

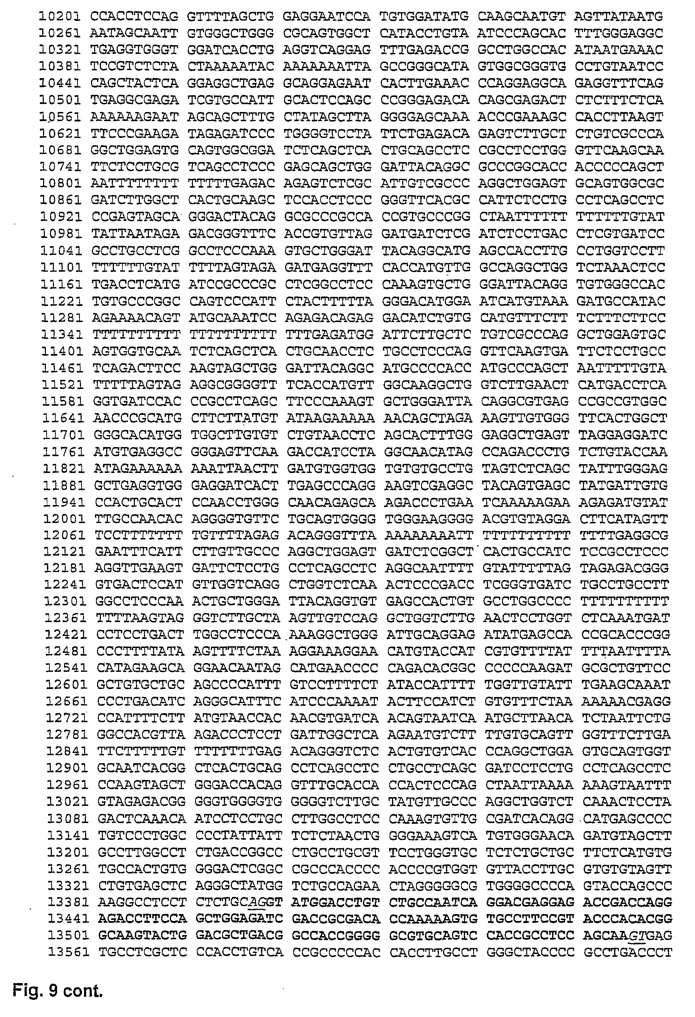

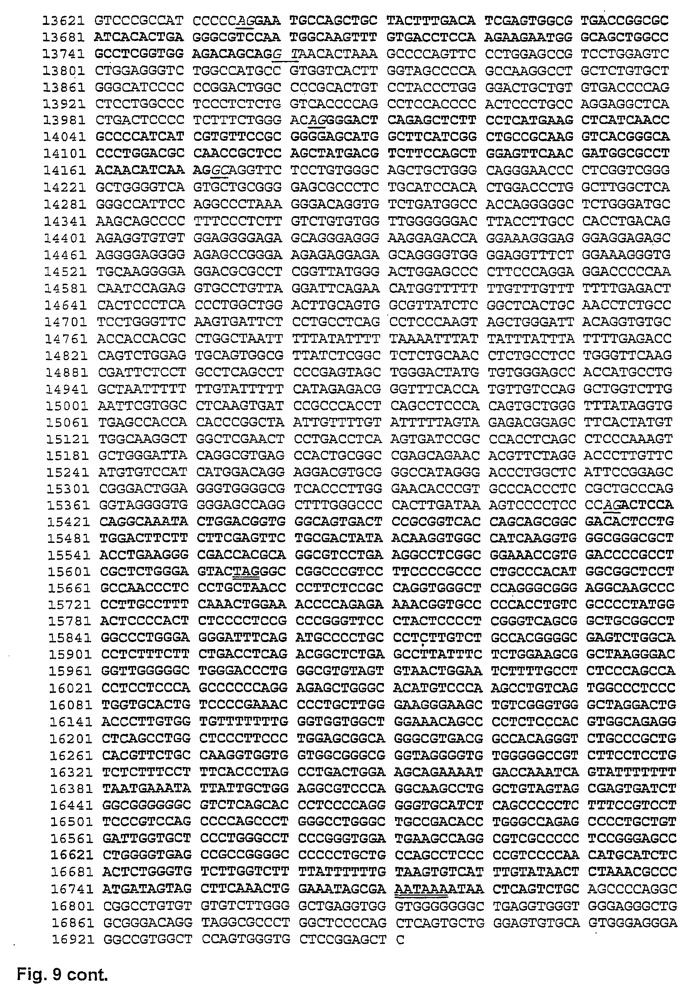

Die erfindungsgemäßen regulatorischen Sequenzen wurden durch DNA-Sequenzierung des humanen Fascin-Gens bereitgestellt, dessen Gesamtsequenz unter SEQ ID NO:72 sowie in Figur 9 dargestellt ist. Die Ergebnisse einer vorläufigen DNA-Sequenzierung dieses Gens erbrachten die unter SEQ ID NOs:10 bis 15 und 33 bis 35 sowie in Figur 2 angegebenen partiellen Nucleotidsequenzen. Die im Rahmen der Erfindung offenbarten regulatorischen Sequenzen SEQ ID NOs:1 bis 20 sind diesen vorläufigen Sequenzen entnommen. Unterschiede zwischen der neueren Gesamtsequenz und den älteren Teilsequenzen sollten im allgemeinen auf Ungenauigkeit bei der vorläufigen DNA-Sequenzierung zurückzuführen sein.

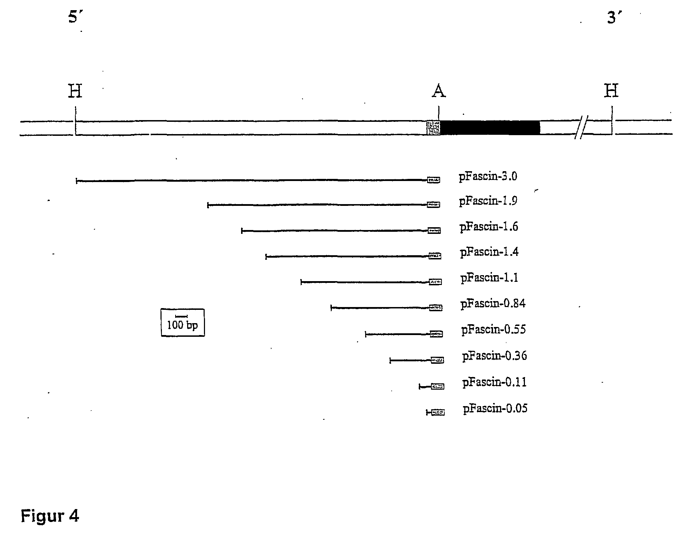

Gleichwohl sind die regulatorischen Sequenzen offenbart durch den hinterlegten Klon DSM13274 und die Angabe von Oligonucleotiden, mit denen die jeweiligen Teilsequenzen der Insertion, beispielsweise durch PCR, amplifiziert werden können. Der Klon DSM13274 ist der PAC-Klon RPCIP704C24766Q3/4, der unter der Hinterlegungsnummer DSM13274 bei der Deutschen Sammlung von Mikroorganismen und Zellkulturen, Braunschweig, am 1. Februar 2000 hinterlegt wurde. Mit Hilfe von Oligonucleotiden, beispielsweise mit solchen, deren Sequenzen unter SEQ ID NO: 36 und 37 angegeben sind, läßt sich der vollständige Promotor des humanen Fascingens amplifizieren. Mit Hilfe von Oligonucleotidpaaren, beispielsweise mit solchen, deren Sequenzen unter SEQ ID NO: 38 und 37; 39 und 37; 40 und 37; 41 und 37; 42 und 37; 43 und 37; oder 44 und 37 angegeben sind, lassen sich 5'-deletierte Fragmente des Fascinpromotors (s. Fig. 4) amplifizieren, die eine für dendritische Zellen spezifische Expression erlauben. Auf der Basis der unter SEQ ID NOs:1 bis 8 sowie 72 angegebenen Nucleotidsequenzen lassen sich weitere Oligonucleotidpaare ableiten, die es dem Fachmann erlauben, aus dem hinterlegten Klon die entsprechenden Promotorfragmente zu amplifizieren.
Bei der Bereitstellung der erfindungsgemäßen regulatorischen Sequenzen aus dem Fascinpromoter durch Amplifikation aus dem Klon DSM13274 kann die Spezifität der PCR-Reaktion erhöht werden, indem man ihr eine zusätzliche PCR-Reaktion voranstellt. Bei einer solchen "nested PCR" wird zunächst ein größerer Bereich des Fascingenlocus amplifiziert, der die gewünschten regulatorischen Sequenzen enthält, beispielsweise unter Verwendung von Oligonucleotiden, deren Sequenzen unter SEQ ID NO: 69 und 70 angegeben sind. Das Produkt der ersten PCR-Reaktion kann dann als Matrize für eine zweite PCR-Reaktion dienen, bei der mit Hilfe eines der oben angegebenen Oligonucleotidpaare eine der erfindungsgemäßen regulatorischen Sequenzen gewonnen werden kann.
Des weiteren kann die Sequenz der Promotorfragmente durch direkte Sequenzierung mit dem hinterlegten Klon als Matrize ermittelt werden. Der Fachmann kann hierzu aus den unter SEQ ID NOs:1 bis 8 sowie 72 angegebenen Nucleotidsequenzen Sequenzierungsprimer ableiten.

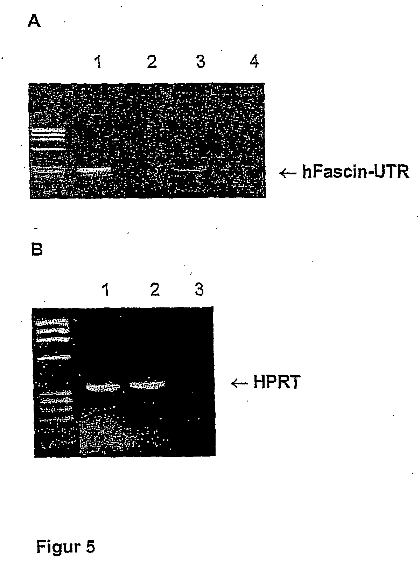

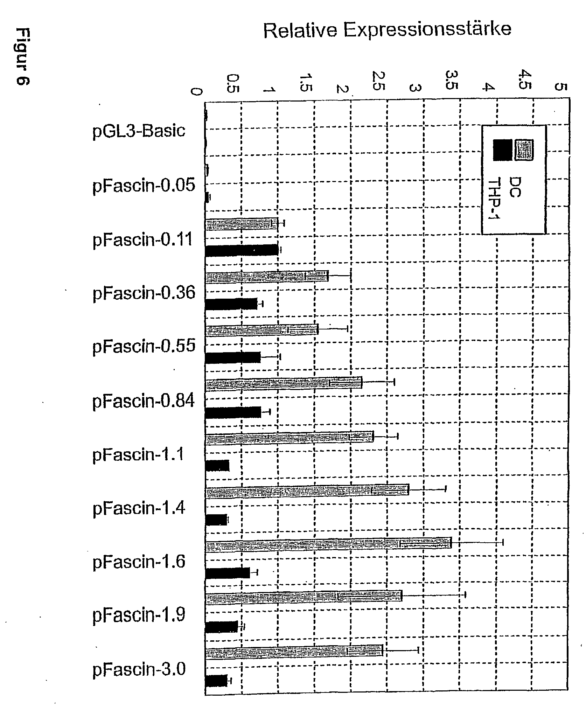

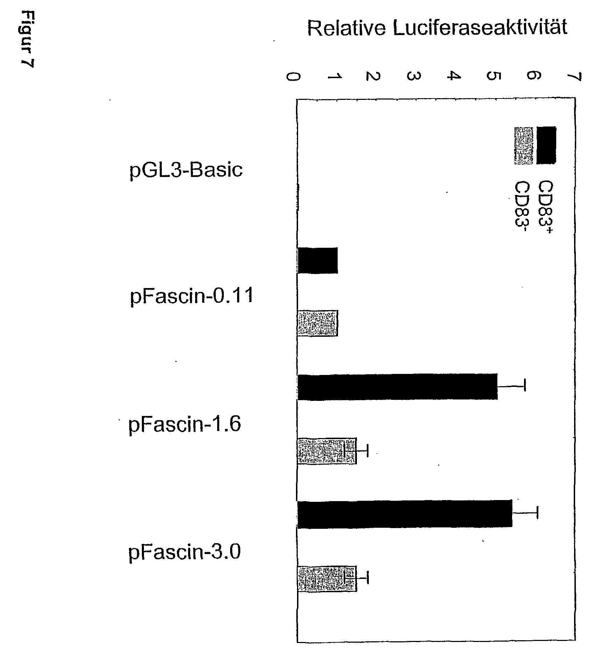

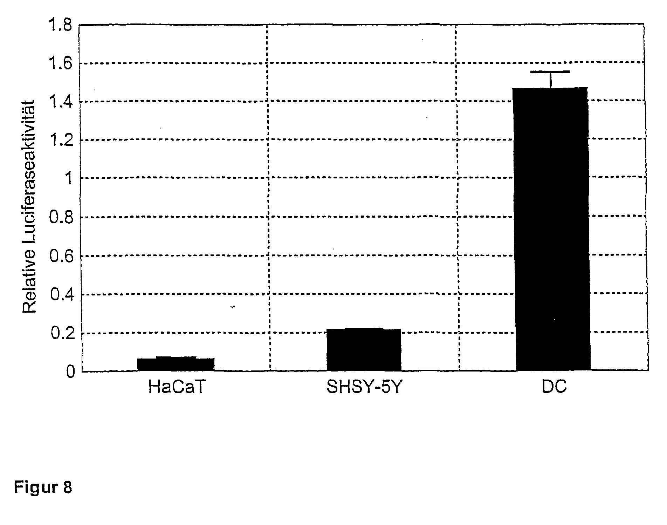

Die Erfindung basiert auf der Beobachtung, daß ein etwa 3.0 kb umfassendes Promotorfragment des menschlichen Fascingens (pFascin-3.0, Fig. 4, SEQ ID NO: 1 bzw. Position 1 bis 3069 in SEQ ID NO:72), das vor die codierende Region eines Reportergens (Photinus-Luciferase) cloniert wurde, in transfizierten, kultivierten dendritischen Zellen zur Expression des Reportergens führte (siehe Beispiel 2). Desweiteren konnte gezeigt werden, daß diese Expression spezifisch ist für dendritische Zellen, da die Expression in THP-1-Zellen (menschliche Monocytenzellinie, die endogen kein Fascin exprimiert, siehe Fig. 5) um etwa das 8-fache niedriger war. Weitere Expressionsstudien zeigten, daß der Fascinpromoter pFascin-3.0 sowie ein Subfragment davon (pFascin-1.6) in der Lage ist, Spezifität für die ausgereiften dendritischen Zellen (CD83⁺-Fraktion) zu vermitteln (Beispiel 2 und Figur 7). Die Stärke des Fascinpromoters wurde durch Cotransfektionsversuche mit einem Reportergenkonstrukt, das den unspezifischen, stark exprimierenden Promotor des Haushaltsgens EF1α enthielt, bestimmt (Beispiel 2 und Figur 8). Bemerkenswerterweise übertraf der Fascinpromoter die Genexpression des EF1α-Konstrukts in reifen dendritischen Zellen um etwa das 1½-fache.
Zur weiteren funktionellen Charakterisierung des Promotors wurden 5'-Deletionen des 3,0 kb-Fragments erzeugt und diese ebenfalls in Reportergen-Assays funktionell analysiert (siehe Beispiel 2, Fig. 4 und 6). Diese Analysen zeigten überraschendenderweise, daß ein 211 bp-Promotorfragment (pFascin-0.11, SEQ ID NO: 21 bzw. Position 2859 bis 3069 in SEQ ID NO:72) in beiden untersuchten Zelltypen die gleiche Expressionsstärke aufwies. Ein um 56 bp weiter 5'-deletiertes Fragment (pFascin 0.05, SEQ ID NO: 22 bzw. Position 2915 bis 3069 in SEQ ID NO:72), das noch die TATA-Box enthält, zeigte dagegen keine Expression, die nennenswert über die Negativkontrolle hinausging. pFascin-0.11 ist demnach ein Minimalpromotor, der eine basale Grundtranskription vermittelt, die keine Spezifität für dendritische Zellen gegenüber Monocyten ausübt.
Überraschenderweise zeigte sich, daß offensichtlich in weiter distal gelegenen Regionen Elemente lokalisiert sind, die einerseits die Transkription in dendritischen Zellen erhöhen (maximal 3,3-fach gegenüber dem Minimalpromotor) und andererseits Elemente vorhanden sind, die die Transkription in THP-1-Zellen vermindern (maximal um das 3-fache). Das Promotorkonstrukt pFascin-1.4 (SEQ ID NO. 4 bzw. Position 1621 bis 3069 in SEQ ID NO:72) zeigte die höchste Spezifität mit einer ca. 10-fach höheren Expression in dendritischen Zellen als in THP-1-Zellen. Diese Beobachtungen lassen den Schluß zu, daß die Promotorsequenz regulatorische Elemente enthält, die in der Lage sind, einem Minimalpromotor, wie z.B. dem in pFascin-0.11 enthaltenen Promotorfragment, Zelltypspezifität zu verleihen.
Somit betrifft die Erfindung auch funktionale Teile der Promotorsequenzen SEQ ID NO: 1 bis 8 bzw. Position 1 bis 3069 in SEQ ID NO:72, die, beispielsweise in Kombination mit einem Minimalpromotor, eine für dendritische Zellen spezifische Expression vermitteln. Beispiele für Minimalpromotoren sind der SV40- oder der Thymidinkinase-Minimalpromotor.
Darüberhinaus können die erfindungsgemäßen regulatorischen Sequenzen, die funktionale Teile des Fascinpromotors darstellen, dazu eingesetzt werden, das Expressionsverhalten bestehender heterologer Promotoren zu modifizieren. Beispielsweise kann ein Promotor, der eine von ihm kontrollierte Nucleotidsequenz konstitutiv exprimiert oder eine bestimmte Spezifität, wie z.B. Induzierbarkeit oder Entwicklungsspezifität aufweist, durch Integration eines oder mehrerer regulatorischer Sequenzen der Erfindung zusätzlich Spezifität für dendritische Zellen verliehen bekommen.
Es war bereits bekannt, daß das Protein Fascin, das Actin-Filamente kreuzvernetzt und in dendritischen Zellen auf diese Weise an der Ausbildung der Dendritenstruktur beteiligt ist, von dendritischen Zellen sowohl des Menschen (Ross, J. Invest. Dermatol. 115 (2000), 658-663) als auch der Maus (Ross, J. Immunol. 160 (1998), 3776-3782) stark exprimiert wird. Ferner war bekannt, daß Epidermalzellen in der unbehandelten Haut (zumindest bei Mäusen) keine Expression zeigen. Erst nach Aktivierung exprimieren die ausgereiften Langerhanszellen, die dendritischen Zellen der Epidermis, Fascin. Es wurde auch gezeigt, daß in humanen Blutzellen Fascin außer in dendritischen Zellen ebenfalls nicht exprimiert wird (Mosialos, Am. J. Pathol. 148 (1996), 593-600). Bisher war es jedoch nicht gelungen, die Sequenzen bereitzustellen, die die beschriebene Spezifität der Fascin-Expression bewirken.

Der Nachweis der Spezifität für dendritische Zellen dieser funktionalen Teile kann unter anderem durch das in Beispiel 2 beschriebene Verfahren erfolgen. Die Isolierung von Teilsequenzen aus einer der oben beschriebenen Promotorsequenzen kann nach den, dem Fachmann bekannten molekularbiologischen Standardmethoden erfolgen, beispielsweise nach Sambrook et al. (Molecular Cloning: A Laboratory Manual, 2. Ausgabe, Cold Spring Harbor Laboratory Press, Cold Spring Harbor NY (1989)). Diese Quelle kann auch für alle weiteren in der vorliegenden Beschreibung aufgeführten molekularbiologischen Techniken zu Rate gezogen werden. Zur Testung der isolierten Fragmente auf Spezifität für dendritische Zellen kann beispielsweise das unter Beispiel 2 beschriebene Verfahren verwendet werden. Hierzu werden die Fragmente stromaufwärts eines Minimalpromotors, wie oben definiert, cloniert und anschließend in transienten Assays die Expression eines Reportergens in dendritischen Zellen und in nicht-Fascin-exprimierenden Zellen (z.B. THP-1) gemessen. Spezifische Expression in dendritischen Zellen im Sinne der Erfindung liegt vor, wenn der Expressionslevel im Vergleich zu den nicht-Fascin-exprimierenden Zellen mindestens um das 5-fache, vorzugsweise mindestens um das 8-fache, besonders bevorzugt mindestens um das 10-fache, insbesondere bevorzugt mindestens um das 15-fache und am meisten bevorzugt um mindestens das 20-fache erhöht ist.
Ein weiterer Aspekt der Erfindung betrifft regulatorische Sequenzen, die mit einer der oben definierten erfindungsgemäßen regulatorischen Sequenzen, vorzugsweise dem komplementären Strang davon, hybridisieren, eine Sequenzidentität zu diesen von mindestens 50% aufweisen und eine für dendritische Zellen spezifische Expression einer von ihnen kontrollierten Nucleotidsequenz bewirken.

Bei diesen hybridisierenden Sequenzen kann es sich sowohl um Promotoren, wie weiter oben definiert, als auch um regulatorische Elemente handeln, die Minimalpromotoren Spezifität für dendritische Zellen verleihen können.

Der verwendete Begriff "hybridisieren" bezieht sich auf konventionelle Hybridisierungsbedingungen, vorzugsweise auf Hybridisierungsbedingungen, bei denen als Lösung 5xSSPE, 1% SDS, 1xDenhardts-Lösung verwendet wird und/oder die Hybridisierungstemperaturen zwischen 35°C und 70°C, vorzugsweise bei 65°C liegen. Nach der Hybridisierung wird vorzugsweise zuerst mit 2xSSC, 1% SDS und danach mit 0,2XSSC bei Temperaturen zwischen 35°C und 70°C, vorzugsweise bei 65°C gewaschen (zur Definition von SSPE, SSC und Denhardts-Lösung siehe Sambrook et al., a.a.O.). Besonders bevorzugt sind stringente Hybridisierungsbedingungen, wie sie beispielsweise in Sambrook et al., s.o. beschrieben sind. Besonders bevorzugte stringente Hybridisierungsbedingungen liegen beispielsweise vor, wenn Hybridisierung und Waschen wie oben angegeben bei 65°C erfolgt. Weniger bevorzugt sind nicht-stringente Hybridisierungsbedingungen wie beispielsweise mit Hybridisierung und Waschen bei 45°C, noch weniger bevorzugt bei 35°C.
Derartige regulatorische Sequenzen weisen eine Homologie, bestimmt durch Sequenzidentität, vorzugsweise über die gesamte Länge der verglichenen Sequenzen, von mindestens 50%, bevorzugt von mindestens 60%, besonders bevorzugt von mindestens 70%, vorteilhafterweise von mindestens 80%, vorzugsweise von mindestens 90% und insbesondere bevorzugt mindestens 95% zu der unter SEQ ID NO. 1 oder der von Position 1 bis 3069 in SEQ ID NO:72 gezeigten Sequenz auf. Vorzugsweise sind die hybridisierenden Sequenzen Fragmente mit einer Länge von mindestens 500 Nucleotiden, die zu der unter SEQ ID NO: 1 oder der von Position 1 bis 3069 in SEQ ID NO:72 gezeigten Sequenz eine Identität von mindestens 70%, vorzugsweise von mindestens 80%, besonders bevorzugt von mindestens 90% und insbesondere bevorzugt von mindestens 95% aufweisen. Wenn zwei zu vergleichende Sequenzen eine unterschiedliche Länge aufweisen, bezieht sich die Sequenzidentität vorzugsweise auf den prozentualen Anteil der Nucleotidreste der kürzeren Sequenz, die identisch sind mit den Nucleotidresten der längeren Sequenz. Die Sequenzidentität kann konventionell durch Verwendung von Computerprogrammen wie z. B. das Bestfit-Programm (Wisconsin Sequence Analysis Package, Version 8 for Unix, Genetics Computer Group, University Research Park, 575 Science Drive Madison, WI 53711) bestimmt werden. Bestfit nützt den lokalen Homologiealgorithmus von Smith und Waterman, Advances in Applied Mathematics 2 (1981), 482-489, um das Segment mit höchster Sequenzidentität zwischen zwei Sequenzen zu finden. Bei der Anwendung von Bestfit oder einem anderen Sequenz-Alignment-Programm zur Bestimmung, ob eine bestimmte Sequenz beispielsweise zu 95% identisch ist mit einer Referenzsequenz der vorliegenden Erfindung, werden die Parameter vorzugsweise so eingestellt, daß der Prozentanteil der Identität über die gesamte Länge der Referenzsequenz berechnet wird und daß Homologielücken ("gaps") von bis zu 5% der Gesamtzahl der Nucleotide in der Referenzsequenz erlaubt sind. Bei der Verwendung von Bestfit werden die sogenannten optionalen Parameter vorzugsweise bei ihren voreingestellten ("default") Werten belassen. Die Abweichungen, die bei dem Vergleich einer gegebenen Sequenz mit den oben beschriebenen Sequenzen der Erfindung auftreten, können beispielsweise durch Addition, Deletion, Substitution, Insertion oder Rekombination verursacht sein. Vorzugsweise kann ein derartiger Sequenzvergleich auch mit dem Programm DNASIS (Version 6.0, Hitachi Software Engineering Co. Ltd., 1984, 1990) durchgeführt werden. Hierbei sollten ebenfalls die "default"-Parametereinstellungen (Cut off Score: 16, Ktup: 6) verwendet werden.
Ob hybridisierende Sequenzen eine für dendritische Zellen spezifische Expression vermitteln, kann beispielsweise ebenfalls nach den in Beispiel 2 beschriebenen Techniken festgestellt werden.

Die erfindungsgemäßen regulatorischen Sequenzen ermöglichen eine für dendritische Zellen spezifische Expression von Nucleotidsequenzen, die von Ihnen kontrolliert werden. Durch ihre Eigenschaft, als einzige Subpopulation der Antigen-präsentierenden Zellen (APC) naive T-Zellen effizient aktivieren zu können, nehmen dendritische Zellen eine zentrale Stellung bei der Vermittlung und Modulation von Immunantworten ein. Die Bereitstellung der erfindungsgemäßen regulatorischen Sequenzen eröffnet nun die Möglichkeit, dendritische Zellen gezielter als bisher zu nutzen und dabei Nachteile von im Stand der Technik beschriebenen Methoden zu überwinden. Dies bezieht sich im wesentlichen auf die Techniken der DNA-Vakzinierung und der in vitro-Transformation von dendritischen Zellen, unter anderem zum Zwecke der Gentherapie.
Daß sich dendritische Zellen als Ansatzpunkte für DNA-Vakzinierungen eignen, ist bekannt. So zeigten beispielsweise kinetische Studien, bei denen das immunisierte Gewebe zu unterschiedlichen Zeiten nach der Immunisierung entfernt wurde, daß direkt transfizierte dendritische Zellen, die kurze Zeit nach Immunisierung in die Lymphknoten wanderten, von ausschlaggebenderer Bedeutung für eine erfolgreiche Immunisierung sind (Torres et al., J. Immunol. 158 (1997), 4529-4532; Klinman et al., J. Immunol. 160 (1998), 2388-2392). Ferner konnte gezeigt werden, daß dendritische Zellen, die nach Injektion von DNA aus dem transfizierten Gewebe auswanderten, das Antigen in immunogener Form trugen und in der Lage waren, Immunantworten gegen das Plasmid-codierte Protein hervorzurufen (Casares et al., J. Exp. Med. 186 (1997), 1481-1486).
DNA-Vakzinierungen werden primär durch Injektion, beispielsweise durch intramuskuläre oder intradermale Injektion vorgenommen, können aber z.B. auch durch Beschuß der Haut mit Partikeln, an die Expressionsplasmide gebunden sind, verabreicht werden. Ebenso können Expressionsplasmide oral oder sublingual gegeben werden oder auf die Schleimhäute des Respirationstraktes durch nasale oder intratracheale Applikation aufgebracht werden. Die Induktion von humoralen und zellulären Immunantworten durch Applikation von Antigen-codierender DNA in die Haut wird nach sehr gutem Erfolg im Tiermodell bereits am Menschen erprobt. Bisher werden Promotoren verwendet, die zur Expression des codierten Proteins in allen Hautzellen also auch beispielsweise in Keratinocyten führen. Dabei ist die epidermale Subpopulation der dendritischen Zellen, die Langerhans-Zellen, der eigentliche Adressat solcher Vakzinierungen. So zeigten Arbeiten an Knochenmarks-chimären Mäusen, daß die MHC-Restriktion der T-Zellantwort nach DNA-Immunisierung allein vom MHC-Haplotyp der Knochenmarkszellen, also den professionellen APCs (dendritischen Zellen) abhängig ist und nicht vom MHC-Haplotyp der Myocyten oder Keratinocyten (Fu et al., Mol. Med. 3 (1997), 362-371). Diese Zellen sind folglich selbst nicht in der Lage, eine Immunantwort zu induzieren. Dennoch könnte die Expression des Antigens in diesen Zellen zu einer durch dendritische Zellen vermittelten Immunisierung beitragen, wenn das sekretierte Antigen von dendritischen Zellen aufgenommen wird. Das Antigen könnte dann von der dendritischen Zelle prozessiert und über MHC-Moleküle präsentiert werden. Solche exogen aufgenommenen Antigene werden über MHC-Klasse II-Proteine präsentiert. Im Sinne einer effektiven DNA-Vakzinierungsstrategie ist jedoch eine Antigenpräsentation über MHC-Klasse I-Proteine wünschenswert, da diese die zelluläre lmmunabwehr induziert, während MHC-Klasse II-Präsentation eine humorale Immunantwort hervorruft. Dies ist im Gegensatz zu herkömmlichen DNA-Vakzinierungsmethoden durch die regulatorischen Sequenzen der vorliegenden Erfindung nun besser realisierbar. Im Gegensatz zur Aufnahme exogener Antigene führt die intracelluläre Expression eines Antigens in dendritischen Zellen zur Präsentation antigener Peptide über MHC-Klasse I-Moleküle. Durch die gezielte Adressierung von Antigen-exprimierenden Vektoren an dendritische Zellen wird die gestreute Expression von mittransfizierten umliegenden Zellen, wie z.B. Keratinocyten, und damit auch die exogene Aufnahme von exprimiertem Antigen via Endocytose durch dendritische Zellen weitgehend ausgeschlossen. Somit ist zu erwarten, daß durch Vakzinierung mit DNA, die spezifisch in dendritischen Zellen exprimiert wird, eine Stärkung der zellulären Immunantwort unter Beteiligung cytotoxischer T-Zellen erreicht wird.
Ferner ist bekannt, daß die Präsentation eines Antigens durch nicht-professionelle APCs, welche nicht die notwendigen co-stimulatorischen Signale für eine effiziente T-Zell-Stimulation zur Verfügung stellen, zu einer mangelhaften Reaktivität bzw. Anergie der T-Zellen führt. Mit Hilfe der erfindungsgemäßen regulatorischen Sequenzen ist es nun möglich, derartige Effekte weitgehend auszuschalten, indem die Expression des Antigens nach DNA-Vakzinierung auf reife dendritische Zellen beschränkt wird. Dies führt zu einer in der Summe verstärkten Immunantwort.
Eine weitere wichtige Anwendung der erfindungsgemäßen regulatorischen Sequenzen liegt in der DNA-Vakzinierung mit Konstrukten, die immunmodulatorische Proteine (z.B. Cytokine, co-stimulierende Membranproteine) exprimieren. Zusätzlich zur Expression von Antigenen kann durch Co-Transfektion von Konstrukten, die immunmodulatorische Proteine codieren, regulatorisch in die Immunantwort eingegriffen werden. Die Beschränkung dieser Mediatoren auf transfizierte dendritische Zellen erlaubt es, sehr spezifisch die erwünschte Immunantwort zu beeinflussen, ohne die bei systemischer Gabe der Mediatoren zu erwartenden Nebenwirkungen befürchten zu müssen.
Auch die Transfektion von dendritischen Zellen in vitro, unter anderem zu anschließenden therapeutischen Anwendungen, kann durch die regulatorischen Sequenzen der Erfindung entscheidend verbessert werden. Wie oben beschrieben, existieren im Stand der Technik bereits vielfältige Methoden zur Verwendung von in vitro-gereiften dendritischen Zellen zu therapeutischen Zwecken, die inzwischen bis hin zu klinischen Tests reichen. Für viele dieser Methoden und Anwendungen ist es jedoch notwendig, durch aufwendige Aufreinigungsprozeduren zu präparierende, möglichst homogene Populationen von dendritischen Zellen einzusetzen, um den bekannten Nachteilen der Transfizierung von Mischpopulationen zu entgehen.

Dendritische Zellen sind nur zu 1% oder weniger in der peripheren Blutleukocytenpopulation vertreten. Zu diesen Nachteilen zählt beispielsweise der oben im Zusammenhang mit DNA-Vakzinierung diskutierte Effekt der mangelnden Reaktivität oder Anergie von T-Zellen in Folge von Antigenpräsentation durch nicht-professionelle APCs.
Ein Beispiel für Therapieansätze mit in vitro-transfizierten dendritischen Zellen bezieht sich auf die Expression von Tumor- oder Pathogen-abgeleiteten Antigenen in dendritischen Zeiten, die zur anschließenden Therapie in Patienten eingesetzt werden können.
Ein weiteres Beispiel für eine Therapieform durch gezielte Expression in dendritischen Zellen bezieht sich auf dendritische Zellen, die ein relevantes Antigen (z.B. Autoantigen oder Transplantationsantigen) präsentieren und mit dem IL-10 Gen oder einem anderen immunsupprimierenden Gen effizient transfiziert werden. Dies kann zur Induktion einer gezielten Anergie in den korrespondierenden T-Zellen eingesetzt werden. Auf diese Weise läßt sich beispielsweise Autoimmunität oder Transplantatabstoßung therapieren.
Ebenso ermöglichen die erfindungsgemäßen regulatorischen Sequenzen, Gene für regulatorisch eingreifende Proteine (z.B. Cytokine, co-stimulatorische Moleküle) spezifisch in dendritischen Zellen zur Expression zu bringen und somit die Qualität der Immunantwort durch Selektion der jeweiligen Klassen von T-Helferzellen bzw. cytotoxischen T-Zellen (Th1/Th2, Tc1/Tc2) und die Stärke der Immunantwort zu beeinflussen.
Der Vorteil der erfindungsgemäßen regulatorischen Sequenzen gegenüber z.B. dem konstitutiv exprimierenden CMV-Promotor liegt darin, daß die gesamte Leukocytenpopulation (bei EBV-negativen Personen), bzw. die B-Zell-depletierte Leukocytenpopulation (bei EBV-infizierten Personen) oder angereicherte frisch isolierte oder kultivierte dendritische Zellen in geeigneten Reifestadien eingesetzt werden können, und somit die zeitaufwendige und kostenintensive Aufreinigung von dendritischen Zellen umgangen werden kann.

In einer weiteren Ausführungsform der Erfindung sind die oben beschriebenen regulatorischen Sequenzen kombiniert mit mindestens einer Nucleotidsequenz, die
(a) ausgewählt ist aus der Gruppe bestehend aus: den Abschnitten von SEQ ID NO: 72 von Position 3911 bis 13398, 13556 bis 13637, 13760 bis 14004, 14173 bis 15414 und 16791 bis 16951 und SEQ ID NOs: 9 bis 20, oder Teilen davon; oder
(b) in der Insertion des Klons DSM13274 enthalten ist und durch Amplifikation unter Verwendung eines Paars von Oligonucleotiden erhältlich ist, wobei die Sequenzen der Oligonucleotide beispielsweise angegeben sind unter den SEQ ID-Nummern, ausgewählt aus der Gruppe von Paaren bestehend aus: 45 und 46; 47 und 48; 49 und 50; 51 und 52; 53 und 54; 55 und 56; 57 und 58; 59 und 60; 61 und 62; 63 und 64; 65 und 66; 67 und 68; und 45 und 60.

Nach den Ergebnissen aus Beispiel 2 ist die Reporterexpression in THP-1 durch die getesteten regulatorischen Sequenzen zwar deutlich vermindert gegenüber der basalen Expression, vor allem maximal ca. 10-fach niedriger als in dendritischen Zellen. In Anbetracht dessen, daß in THP-1 endogen keine Fascinexpression nachzuweisen ist (Fig. 5), ergibt sich die Frage, warum die Expression in THP-1 in transienten Expressionsassays nicht weit niedriger ausfällt. Offensichtlich reicht die Kontrolle des Fascinpromotors allein nicht aus für die völlige Repression der Fascinexpression. Demnach ist zu erwarten, daß ein weiteres kontrollierendes Element beispielsweise ein Silencer, im Fascingen die Transkription in nicht-dendritischen Zellen reprimiert. Bevorzugte Aufenthaltsorte für Silencer sind Introns oder die 3'-genflankierende Region. Somit umfaßt die Erfindung auch solche regulatorischen Sequenzen, die neben den oben beschriebenen Sequenzen zusätzlich mit einer oder mehreren der folgenden Sequenzen kombiniert ist: Intronsequenzen des Fascingens (die Abschnitte von SEQ ID NO: 72 von Position 3911 bis 13398, 13556 bis 13637, 13760 bis 14004 und 14173 bis 15414 bzw. SEQ ID NOs: 9 bis 19), die 161 bp umfassende 3'-genflankierende Region (Position 16791 bis 16951 in SEQ ID NO:72 bzw. SEQ ID NO: 20) und jeweils Teile davon. Vorzugsweise vermitteln die in dieser Ausführungsform gekennzeichneten regulatorischen Sequenzen Nucleotidsequenzen, die von ihnen kontrolliert werden, eine Expression, die noch spezifischer ist als die der regulatorischen Sequenzen, die nicht mit Intronsequenzen und/oder 3'-flankierenden Sequenzen kombiniert sind. "Noch spezifischer" bedeutet, daß die Expression durch die Kombination mit regulatorischen Sequenzen der vorliegenden Ausführungsform zwischen dendritischen Zellen und nicht-Fascin-exprimierenden Zellen um einen Faktor divergiert, der größer ist als mit denselben regulatorischen Sequenzen ohne eine Sequenz aus den Intron- und 3'-flankierenden Sequenzen in SEQ ID NO:72 bzw. SEQ ID NOs:9 bis 20. Dieser Faktor ist vorzugsweise größer als 10, besonders bevorzugt größer als 15, insbesondere bevorzugt größer als 20, am meisten bevorzugt größer als 30. Vorzugsweise liegt die Expression einer von diesen regulatorischen Sequenzen kontrollierten Nucleotidsequenzen in nicht-Fascin-exprimierenden Zellen unter der Nachweisgrenze.
"Kombiniert" bedeutet in diesem Zusammenhang, daß eine oder mehrere Sequenzen aus den Intron- und 3'-flankierenden Sequenzen in SEQ ID NO:72 bzw. SEQ ID NOs:9 bis 20 oder Teile davon nach den gängigen molekularbiologischen Techniken in die Nähe einer unter SEQ ID NOs: 1 bis 8 gezeigten Sequenz bzw. der oben genannten entsprechenden Abschnitte von SEQ ID NO:72, einem funktionalen Teil davon oder einer Sequenz, die eine der vorgenannten Sequenzen hybridisiert, cloniert wird. "In die Nähe" heißt, daß die Sequenz(en) aus den Intron- und 3'-flankierenden Sequenzen in SEQ ID NO:72 bzw. SEQ ID NOs: 9 bis 20 oder Teilen davon direkt oder in einem gewissen Abstand zu den vorgenannten regulatorischen Sequenzen stromabwärts oder stromaufwärts oder intermittierend cloniert wird, vorzugsweise erfolgt die Clonierung jedoch stromabwärts, da die vorgenannten regulatorischen Sequenzen (Promotoren oder funktionale Teile davon) bekanntermaßen für ihre Funktionsweise, d.h. zum Binden von RNA-Polymerase oder Transkriptionsfaktoren, relativ definierte Distanzen zum Transkriptionsstartpunkt bzw. zur TATA-Box benötigen.
Unter "in einem gewissen Abstand" wird eine Distanz verstanden, die dazu geeignet ist, daß Silencer oder Enhancer ihre Funktion ausüben können. Die Formulierung "Teil davon" ist zu verstehen als eine Sequenz innerhalb einer in SEQ ID NOs: 9 bis 20 gezeigten Sequenz bzw. einer Intron- oder 3'-flankierenden Sequenz in SEQ ID NO: 72, die geeignet ist, eine Silencer- oder Enhancer-Funktion auszuüben. Derartige Sequenzen können durch eine experimentelle Vorgehensweise, die zu der in Beispiel 2 analog ist, d.h. durch funktionelle Analysen von Reportergen-Konstrukten, eingegrenzt werden. Hierzu kann man beispielsweise eine isolierte Teilsequenz einer Sequenz aus SEQ ID NOs:9 bis 20 bzw. einer Intron- oder 3'-flankierenden Sequenz in SEQ ID NO: 72 in ein funktionales Promotor-Reportergen-Konstrukt, wie beispielsweise pFascin-3.0 clonieren, vorzugsweise direkt an das 5'-Ende des Promotorfragments angrenzend, oder in ein Intron des Reportergens, und anschließend dieses Konstrukt und zum Vergleich dasselbe Konstrukt ohne diese Teilsequenz in einem transienten Expressionsassay in dendritischen Zellen, vorzugsweise reifen dendritischen Zellen, sowie in nicht-Fascin-exprimierenden Zellen analysieren. Ergibt sich hierbei für das Konstrukt mit dem Teilstück eine größere Differenz der Expressionslevel zwischen dendritischen Zellen und nicht-Fascin-exprimierenden Zellen als für das Konstrukt ohne das Teilstück, so umfaßt dieses Teilstück ein funktionales Silencer- oder Enhancer-Element. Weitere Techniken zur Eingrenzung solcher Elemente stehen dem Fachmann zur Verfügung. Die Ausführungsform umfaßt die oben beschriebenen regulatorischen Sequenzen, die kombiniert sind mit mindestens einer Nucleotidsequenz, die durch Amplifikation, beispielsweise mittels PCR, aus der Insertion des hinterlegten Klons DSM13274 bereitgestellt werden kann, oder Teilen davon. Zur Amplifikation können beispielsweise Paare von Oligonucleotiden verwendet werden, deren Sequenzen unter folgenden SEQ ID-Nummern angegeben sind: 45 und 46; 47 und 48; 49 und 50; 51 und 52; 53 und 54; 55 und 56; 57 und 58; 59 und 60; 61 und 62; 63 und 64; 65 und 66 (Intronsequenzen); sowie 67 und 68 (3'-genflankierende Region). Weitere Oligonucleotidpaare lassen sich zu diesem Zweck von den unter SEQ ID NO: 9 bis 20 angegebenen Nucleotidsequenzen ableiten.
Des weiteren kann die Sequenz dieser Fragmente durch direkte Sequenzierung mit dem hinterlegten Klon als Matrize ermittelt werden. Der Fachmann kann hierzu aus den unter SEQ ID NO: 9 bis 20 angegebenen Nucleotidsequenzen bzw. den in SEQ ID NO:72 enthaltenen Intron- oder 3'-flankierenden Sequenzen Sequenzierungsprimer ableiten.
Ebenso betrifft die vorliegende Ausführungsform die Kombination der oben definierten regulatorischen Sequenzen mit dem gesamten Intron 1 des Fascingens oder Teilen davon. Die Nucleotidsequenz des Introns 1 ist in dem hinterlegten Klon DSM13274 enthalten und kann durch Amplifikation bereitgestellt werden, wobei hierzu ein Oligonucleotidepaar mit Sequenzen, die beispielsweise unter SEQ ID NO: 45 und 60 angegeben sind, verwendet werden kann.

Auch hier kann die Spezifität von PCR-Reaktionen, die der Bereitstellung von Sequenzen aus dem Klon DSM13274 dienen, die im Rahmen der vorliegenden Ausführungsform mit einer der oben beschriebenen regulatorischen Sequenzen kombiniert werden können, dadurch erhöht werden, daß man ihr eine zusätzliche PCR-Reaktion voranstellt. Bei einer solchen "nested PCR" wird zunächst ein größerer Bereich des Fascingenlocus amplifiziert, der die gewünschten Sequenzen enthält, beispielsweise unter Verwendung von Oligonucleotiden, deren Sequenzen unter SEQ ID NO: 71 und 68 angegeben sind. Das Produkt der ersten PCR-Reaktion kann dann als Matrize für eine zweite PCR-Reaktion dienen, bei der mit Hilfe eines der oben angegebenen Oligonucleotidpaare eine Intron- oder 3'-genflankierte Sequenz zur Kombination mit den oben beschriebenen regulatorischen Sequenzen gewonnen werden kann.
Die Kombination der durch Amplifikation aus dem Klon DSM13274 gewonnenen Sequenzen mit den oben definierten regulatorischen (Promoter-)sequenzen kann entsprechend der für die im Sequenzprotokoll offenbarten Intron- und 3'-flankierenden Sequenzen angegebenen Anleitung erfolgen.

In einer besonders bevorzugten Ausführungsform stammen die regulatorischen Sequenzen aus Mensch.

Vorzugsweise handelt sich bei den erfindungsgemäßen regulatorischen Sequenzen um DNA- oder RNA-Moleküle, wobei es sich bei den DNA-Molekülen vorzugsweise um genomische DNA handelt.

Eine weitere bevorzugte Ausführungsform der Erfindung bezieht sich auf rekombinante Nucleinsäuremoleküle, die die erfindungsgemäßen regulatorischen Sequenzen enthalten in Kombination mit einer Nucleotidsequenz, wobei die Nucleotidsequenz aus einer anderen Spezies stammt als die regulatorische Sequenz, die Nucleotidsequenz natürlicherweise im Vergleich zur regulatorischen Sequenz an einer anderen Position innerhalb des Genoms lokalisiert ist oder die Nucleotidsequenz im Vergleich zur natürlichen Vorlage mutiert oder chemisch modifiziert ist.
Der Begriff "rekombinantes Nucleinsäuremolekül" bezieht sich auf Nucleinsäuremoleküle, die einem unterschiedlichen genetischen Kontext entstammen und durch molekularbiologische Methoden kombiniert wurden. "Unterschiedlicher genetischer Kontext" bezieht sich hierbei auf Genome aus unterschiedlichen Spezies, Varietäten oder Individuen oder unterschiedlichen Positionen innerhalb eines Genoms. Neben natürlichen Sequenzen können rekombinante Nucleinsäuremoleküle auch Sequenzen enthalten, die im Vergleich zu ihrer natürlichen Vorlage mutiert oder chemisch modifiziert sind.
An den erfindungsgemäßen rekombinanten Nucleinsäuremolekülen liegen eine oder mehrere der oben beschriebenen regulatorischen Sequenzen in Kombination mit Sequenzen aus einem anderen genetischen Kontext vor. Ein Beispiel für ein rekombinantes Nucleinsäuremolekül enthält eine oder mehrere erfindungsgemäße regulatorische Sequenzen in Kombination mit einem Minimalpromotor, der einem anderen Gen als dem humanen Fascingen entnommen wurde. Die regulatorischen Sequenzen sind hierbei regulatorische Promotorelemente, die dem Gesamtpromotor eine für dendritische Zellen spezifische Expression verleihen.
Desweiteren können die rekombinanten Nucleinsäuremoleküle neben einem Promotor, der eine oder mehrere erfindungsgemäße regulatorische Sequenzen enthält, stromabwärts davon eine Polylinkersequenz mit einer oder mehreren Restriktionserkennungsstellen enthalten, in die durch dem Fachmann bekannte Verfahren Nucleotidsequenzen cloniert werden können, die dadurch unter die Expressionskontrolle des Promotors gelangen. Vorzugsweise liegt dieser Polylinker in einer Region, die direkt hinter dem durch den Promotor definierten Transkriptionsstartpunkt liegt.
Ferner kann das erfindungsgemäße rekombinante Nucleinsäuremolekül stromabwärts des Polylinkers ein Transkriptions-Terminationssignal enthalten. Beispiele für geeignete Terminationssignale sind im Stand der Technik beschrieben. Dabei kann es sich beispielsweise um das Thymidinkinase-Polyadenylierungssignal handeln. Die hier beschriebenen rekombinanten Nucleinsäuremoleküle, die vorzugsweise eine zu exprimierende Nucleotidsequenz enthalten, können direkt für Anwendungen im Sinne der Erfindung, wie beispielsweise DNA-Vakzinierungen eingesetzt werden. Die erfindungsgemäßen rekombinanten Nucleinsäuremoleküle können beispielsweise durch gängige in vitro-Amplifikationstechniken, wie zum Beispiel PCR, vermehrt werden. Sie können allerdings auch konventionell in einem Vektor in vivo vermehrt werden und nach Nucleinsäurepräparation und anschließender Heraustrennung aus dem Vektor, wie beispielsweise durch Restriktionsspaltung, für Anwendungen, in denen beispielsweise linearisierte Expressionseinheiten benötigt werden, zur Verfügung gestellt werden.

Rekombinante Nucleinsäuremoleküle, die vorzugsweise eine zu exprimierende Nucleotidsequenz enthalten, können auch Expressionseinheiten darstellen, die oft auch als Expressionskassetten bezeichnet werden, die leicht in verschiedene Standardvektoren cloniert werden können und so je nach Vektor verschiedene Funktionen ausüben können.
Für Anwendungen der erfindungsgemäßen regulatorischen Sequenzen bei EBV-infizierten Personen kann die Expression in B-Zellen durch die Kombination der regulatorischen Sequenzen mit bekannten Silencer-Elementen für B-Zellen unterdrückt werden. Andererseits kann es auch durchaus von Vorteil sein, wenn EBV-infizierte B-Zellen ebenfalls die von den regulatorischen Sequenzen kontrollierte Nucleotidsequenz exprimieren, da EBV-transfizierte B-Zellen gute Antigen-präsentierende Zellen für aktivierte T-Zellen darstellen und somit eine Amplifikation der Immunantwort bewirken können.

Eine weitere bevorzugte Ausführungsform der Erfindung betrifft Vektoren, die die erfindungsgemäße regulatorische Sequenz oder das erfindungsgemäße rekombinante Nucleinsäuremolekül enthalten.
Der Begriff "Vektor" betrifft zirkuläre oder lineare Nucleinsäuremoleküle, die sich autonom in Wirtszellen, in die sie eingeführt werden, replizieren können. Die Vektoren können die oben gekennzeichneten rekombinanten Nucleinsäuremoleküle in ihrer vollen Länge enthalten oder können auch neben den erfindungsgemäßen regulatorischen Sequenzen, die für die rekombinanten Nucleinsäuremoleküle beschriebenen Bestandteile, wie beispielsweise Minimalpromotor, Polylinker und/oder Terminationssignal, enthalten.
Die erfindungsgemäßen Vektoren können zur Replikation in prokaryontischen und/oder eukaryontischen Wirtszellen geeignet sein und enthalten einen entsprechenden Replikationsursprung (origin of replication). Vorzugsweise eignen sich die Vektoren zur Replikation in Säugerzellen, besonders bevorzugt in menschlichen Zellen.
Vorzugsweise enthalten die erfindungsgemäßen Vektoren einen Selektionsmarker. Beispiele für Selektionsmarkergene sind dem Fachmann bekannt. Selektionsmarkergene, die sich zur Selektion in eukaryontischen Wirtszellen eignen, sind beispielsweise Gene für die Dihydrofolat-Reductase, G418 oder Neomycin-Resistenz.
Vorzugsweise handelt es sich bei den erfindungsgemäßen Vektoren um Expressionsvektoren zur Expression in eukaryontischen Zellen. Solche Vektoren lassen sich auch ausgehend von bekannten Expressionsvektoren konstruieren, indem dessen Promotor bzw. die nicht zu einem Minimalpromotor gehörenden Sequenzen davon durch die regulatorischen Sequenzen der Erfindung ersetzt oder um regulatorische Sequenzen (regulatorische Elemente) ergänzt werden. Beispiele für Expressionsvektoren, die sich auf diese Weise modifizieren lassen, sind pcDV1 (Pharmacia), pRC/CMV, pcDNA1 oder pcDNA3 (Invitrogen).

Eine weitere bevorzugte Ausführungsform der Erfindung betrifft die oben beschriebenen rekombinanten Nucleinsäuremoleküle oder Vektoren, die zusätzlich eine zu exprimierende Nucleotidsequenz enthalten, wobei die Expression der Nucleotidsequenz von der regulatorischen Sequenz oder von einem Promoter, der die regulatorische Sequenz enthält, kontrolliert wird.
Die "zu exprimierenden Nucleotidsequenzen" codieren entweder ein Protein oder (Poly)peptid oder RNA-Moleküle, die ihre Funktion auf der RNA-Ebene entfalten. Nucleotidsequenzen, die ein Protein, Polypeptid oder Peptid codieren, umfassen eine codierende Region, die gekennzeichnet ist durch ein Startcodon (ATG), eine Folge von Aminosäure-codierenden Basentripletts und ein Stopcodon (TGA, TAG oder TAA), wenn es sich um DNA handelt. Bei RNAs ist das Thymidin (T) durch Uracil (U) ersetzt. Bei degenerierten Aminosäurecodons können die Basentripletts entsprechend der Codon-Usage der Zielzellen angepaßt werden, mit Techniken, die dem Stand der Technik entnommen werden können. Beispiele für Nucleotidsequenzen, die RNA-Moleküle exprimieren, sind Antisense-RNA oder Ribozyme.

Weiterhin betrifft die Erfindung die oben genannten rekombinanten Nucleinsäuremoleküle oder Vektoren, wobei die zu exprimierende Nucleotidsequenz ein Antigen codiert.
Der Begriff "Antigen" betrifft im Zusammenhang mit der vorliegenden Erfindung Moleküle, die von einem Organismus als fremd erkannt werden, und dadurch eine spezifische Immunantwort auslösen. Antigene werden natürlicherweise von Antigen-präsentierenden Zellen (APC) endocytiert und zusammen mit Histokompatibilitätsantigenen (MHC) der Klasse II an der Zelloberfläche präsentiert.
Bei den Antigenen, die von der vorliegenden Ausführungsform betroffen sind, handelt es sich um Proteine, Polypeptide oder Peptide. Antigene, die von einem Expressionsvektor in einer dendritischen Zelle exprimiert werden, werden durch MHC-I-Proteine präsentiert. Dabei werden die synthetisierten Proteine durch Proteasomen in Peptide gespalten. Die Peptide werden bevorzugt durch TAP1/TAP2-Komplexe in das endoplasmatische Retikulum transloziert und binden dort an MHC-Klasse-I-Moleküle. Ein Teil der Peptide bindet aber auch auf unbekanntem Weg an MHC-Klasse-II-Moleküle.

In einer bevorzugten Ausführungsform der rekombinanten Nucleinsäuremoleküle oder Vektoren sind die Antigene tumor- oder pathogenspezifisch.
Vorzugsweise sind die Antigene, die von den rekombinanten Nucleinsäuremoleküfen oder Vektoren spezifisch in dendritischen Zellen exprimiert werden, Proteine von Pathogenen, wie zum Beispiel Viren (z. B. HIV), Bakterien, Pilzen oder Parasiten, die in Patienten eine Immunantwort auslösen. Unter pathogenspezifischen Antigenen sind allerdings auch Proteine oder (Poly)Peptide eingeschlossen, die zumindest eine antigene Determinante (Epitop) eines Pathogens enthalten.
Tumorspezifische Antigene sind Proteine oder (Poly)Peptide von Tumorzellen, die eine spezifische Immunantwort auslösen können. Auch hierbei sind (Poly)Peptide eingeschlossen, die mindestens ein Epitop eines tumorspezifischen Antigens enthalten.
Zusätzlich kann es sich bei dem Antigen um Proteine der Alzheimer-Plaques (oder zumindest Epitope daraus) handeln, die an der Alzheimer-Krankheit ursächlich beteiltigt sind.

In einer weiteren Ausführungsform der erfindungsgemäßen rekombinanten Nucleinsäuremoleküle oder Vektoren ist das Antigen ein Autoantigen oder ein Transplantationsantigen.
Der Begriff "Autoantigen" betrifft körpereigene Antigene eines Patienten, die beispielsweise in Folge einer Störung der Selbsterkennung oder von Regulationsmechanismen des Immunsystems unter Bildung von Autoantikörpern eine Autoimmunerkrankung verursachen. Geeignete Autoantigene können beispielsweise bei einer vorliegenden Autoimmunerkrankung durch Identifizierung von Autoantikörpern im Patienten bestimmt werden.
Bei "Transplantationsantigenen" handelt es sich um Histokompatibilitätsantigene (MHC) der Klasse I und II, die durch allogene Transplantate in einen Organismus eingeführt werden und dort eine Immunreaktion (Transplantatabstoßung) auslösen.
Die erfindungsgemäßen rekombinanten Nucleinsäuremoleküle oder Vektoren, die spezifisch in Zellen Autoantigene oder Transplantationsantigene exprimieren können, können dazu verwendet werden, die gegen diese Antigene gerichtete Immunreaktion zu hemmen. Hierzu können beispielsweise dendritische Zellen, die diese Antigene exprimieren, in vitro mit einem weiteren Expressionsvektor transfiziert werden, der ein immunregulatorisches Molekül (z. B. IL-10), exprimiert, das in der Lage ist, Immunreaktionen zu hemmen. Durch Verabreichung derart transformierter dendritischer Zellen kann gezielt eine Anergie von T-Zellen, die Autoantigen- bzw. Transplantationsantigen-spezifisch sind, erzeugt werden, und so die pathologische Immunantwort behandelt werden.

In einer weiteren bevorzugten Ausführungsform der rekombinanten Nucleinsäuremoleküle oder Vektoren sind die Antigene Allergene.
Im Zusammenhang mit der vorliegenden Ausführungsform handelt es sich bei "Allergenen" um Proteine oder (Poly)Peptide, die in Organismen eine allergische Reaktion auslösen können, oder zumindest um ein Epitop aus einem solchen Protein oder (Poly)Peptid.
Dendritische Zellen, die intrazellulär ein Allergen exprimieren, können beispielsweise eine Allergen-spezifische cytotoxische T-Zellantwort induzieren, woraufhin cytotoxische T-Zellen Allergen-präsentierende Zellen töten können. Auf diese Weise ist es möglich, die Aktivierung von Allergen-spezifischen T-Helferzellen und somit die Aktivierung von IgE-Antikörper-produzierenden B-Zellen zu verhindern.
Desweiteren können dendritische Zellen mit Expressionsvektoren cotransfiziert werden, die ein Allergen und ein immunregulatorisches Molekül, das in der Lage ist, eine Immunantwort zu hemmen (z.B. IL-10), exprimieren. Alternativ können dendritische Zellen mit Expressionsvektoren cotransfiziert werden, die ein Allergen und eine Antisense-RNA exprimieren, wobei letztere die Expression eines co-stimulatorischen Moleküls (wie z.B. der B7-Familie) inhibiert. In beiden Fällen kann dadurch eine Anergie in den Allergen-spezifischen Zellen induziert werden.

In einer weiteren bevorzugten Ausführungsform der erfindungsgemäßen rekombinanten Nucleinsäuremoleküle oder Vektoren codieren die zu exprimierenden Nucleotidsequenzen ein Protein, das eine Immunantwort reguliert.
Durch die spezifische Adressierbarkeit von dendritischen Zellen durch die erfindungsgemäßen regulatorischen Sequenzen, besteht zusätzlich zur Antigen-Präsentation die Möglichkeit, eine Immunantwort zielgerichtet zu steuern, indem man Proteine in dendritischen Zellen exprimiert, die eine Immunantwort regulieren. Verabreichungen von rekombinanten Nucleinsäuren oder Vektoren, die solche Proteine exprimieren, können erfolgen, indem in vitro dendritische Zellen transfiziert werden, und die Zellen anschließend in eine Person eingebracht werden oder indem Expressionskonstrukte für diese Proteine direkt verabreicht werden, beispielsweise durch Injektion. Immunregulatorische Proteine sollten vorzugsweise in dendritischen Zellen exprimiert werden, die mit einem Antigen beladen sind, vorzugsweise indem die Zellen das Antigen selbst exprimieren, beispielsweise durch einen erfindungsgemäßen Vektor, damit zielgerichtet die Immunantwort reguliert wird, die durch das Antigen hervorgerufen wird.
Beispiele für immunregulatorische Proteine sind Cytokine, zu denen die Lymphokine, Monokine, Interleukine, Chemokine, koloniestimulierenden Faktoren, Interferone und transformierende Wachstumsfaktoren, wie zum Beispiel TGF-β, gerechnet werden. Weitere immunregulatorische Proteine sind co-stimulierende Moleküle. Nucleotidsequenzen, die die immunregulatorischen Proteine codieren, sind im Stand der Technik beschrieben und können der Literatur entnommen werden.

Besonders bevorzugt ist dabei eine Ausführungsform der rekombinanten Nucleinsäuremoleküle oder Vektoren, wobei das Protein, das eine Immunantwort reguliert, ein Cytokin oder ein co-stimulierendes Molekül ist.
"Cytokine" sind definiert als von verschiedenen Zellarten gebildete und sezernierte Substanzen, die als interzelluläre Mediatoren zur Steuerung der Aktivität von anderen Zellen beitragen. Für die Anwendungen, die sich durch die erfindungsgemäßen regulatorischen Sequenzen eröffnen, sind vor allem von Bedeutung die Interleukine, Interferone, Chemokine, koloniestimulierenden Faktoren und transformierenden Wachstumsfaktoren.
Unter "co-stimulierenden Faktoren" sind Moleküle zu verstehen, die von professionellen Antigen-präsentierenden Zellen wie den dendritischen Zellen membrangebunden exprimiert werden oder von diesen Zellen sekretiert werden und die für die effiziente Aktivierung von T-Lymphocyten benötigt werden.
Nucleotidsequenzen, die Cytokine oder co-stimulierende Moleküle codieren, sind dem Fachmann bekannt. Beispielsweise sind Aminosäuresequenzen von Cytokinen in "The Cytokine Handbook" (A.W. Thomson, ed. Academic Press, San Diego, CA, 1998) und von co-stimulatorischen Molekülen in "The Leucocyte Antigen Facts Book" (A.N. Barclay, M.H. Brown, S.K.A. Law, A.J. McKnight, M.G. Tomlinson, P.A. van der Merwe, eds., Academic Press, San Diego, CA, 1997) veröffentlicht. Entsprechende codierende Nucleotidsequenzen können den öffentlich zugänglichen Datenbanken entnommen werden. Vorzugsweise werden zur Konstruktion der erfindungsgemäßen rekombinanten Nucleinsäuremoleküle und Vektoren dieser und der nachfolgenden Ausführungsformen Nucleotidsequenzen verwendet, die humanen Ursprungs sind. Durch die Wahl von geeigneten Cytokinen oder co-stimulierenden Molekülen zur spezifischen Expression in dendritischen Zellen, kann die Immunantwort entweder gesteigert oder gehemmt werden.

In einer besonders bevorzugten Ausführungsform exprimieren die erfindungsgemäßen rekombinanten Nucleinsäuremoleküle oder Vektoren Proteine, die eine immunantwort regulieren, wobei die Regulation eine Hemmung ist.
Diese Ausführungsform der Erfindung kann dazu verwendet werden, dendritische Zellen, die ein Antigen tragen, das eine unerwünschte Immunantwort hervorruft (z.B. Autoantigene oder Transplantationsantigene), mit rekombinanten Nucleinsäuremolekülen oder Vektoren zu transfizieren, die ein Protein exprimieren, das die Immunantwort hemmt. Dies kann zur Induktion einer gezielten Anergie in den korrespondieren T-Zellen eingesetzt werden.
Vorzugsweise tragen die dendritischen Zellen das Antigen, weil sie mit einem erfindungsgemäßen Nucleinsäuremolekül oder Vektor, das/der das Antigen exprimiert, transfiziert wurden. Cytokine mit einer derart hemmenden Aktivität sind in der Literatur beschrieben. Hierzu zählt beispielsweise das Interleukin IL-10 oder der transformierende Wachstumsfaktor TGF-β.
Somit betrifft eine besonders bevorzugte Ausführungsform rekombinante Nucleinsäuremoleküle oder Vektoren, die das Protein IL-10 oder TGF-β exprimieren.

In einer weiteren bevorzugten Ausführungsform exprimieren die erfindungsgemäßen rekombinanten Nucleinsäuremoleküle oder Vektoren Proteine, die eine Immunantwort regulieren, wobei die Regulation eine Steigerung der Immunantwort ist.
Eine gezielte Steigerung der Immunantwort kann durch Verabreichung von rekombinanten Nucleinsäuremolekülen oder Vektoren erreicht werden, die geeignete Cytokine oder co-stimulierende Moleküle exprimieren. Unter Cytokinen sind beispielsweise für die Interleukine IL-2, IL-4, IL-12, IL-15, IL-18, für die Interferone IFN-gamma und IFN-alpha, für die Chemokine DC-CK1 und MDC sowie für den Granulocyten/Monocyten koloniestimulierenden Faktor (GM-CSF) immunstimulierende Wirkungen beschrieben worden. Nucleinsäuremoleküle, die diese Faktoren codieren, sind dem Stand der Technik zu entnehmen, wie beispielsweise in den oben genannten Quellen.

Somit betrifft eine besonders bevorzugte Ausführungsform rekombinante Nucleinsäuremoleküle oder Vektoren, die die Proteine IL-2, IL-4, IL-12, IL-15, IL-18, IFN-gamma, IFN-alpha, DC-CK1, MDC oder GM-CSF exprimieren.

Eine weitere besonders bevorzugte Ausführungsform betrifft rekombinante Nucleinsäuremoleküle oder Vektoren, die ein co-stimulatorisches Molekül, vorzugsweise ein Mitglied der B7-Familie, ICOS-Ligand oder CD40, exprimieren.
Proteine der B7-Familie, ICOS-Ligand und CD40 sind co-stimulatorische Moleküle, die sich zu einer Steigerung der Immunantwort in dem oben ausgeführten Sinne eignen. Nucleotidsequenzen, die diese co-stimulatorischen Faktoren codieren sind ebenfalls in der Literatur beschrieben (siehe oben).

In einer weiteren besonders bevorzugten Ausführungsform der erfindungsgemäßen rekombinanten Nucleinsäuremoleküle oder Vektoren codiert die zu exprimierende Nucleotidsequenz ein Apoptose-induzierendes Molekül.
Der Begriff "Apoptose" bezeichnet programmierten Zelltod, der durch exogene Signale induziert werden kann. Die rekombinanten Nucleinsäuremoleküle oder Vektoren der vorliegenden Ausführungsform können dazu verwendet werden, in Antigen-beladene dendritische Zellen transfiziert zu werden, um T-Zellen, die gegen das Antigen spezifisch sind, zum Absterben zu bringen. Auf diese Weise kann die Zahl solcher T-Zellen herabgesetzt werden und eine unerwünschte Immunreaktion, beispielsweise gegen Autoantigene oder Transplantationsantigene, abgeschwächt werden.
Im Stand der Technik sind einige Proteine beschrieben, die membrangebunden sind, aber auch teilweise sezerniert werden können und in nächster Nachbarschaft in Zellen das Selbstmordprogramm auslösen können. Darunter fallen beispielsweise die Proteine der TNF-Superfamilie.

Somit betrifft eine besonders bevorzugte Ausführungsform der Erfindung rekombinante Nucleinsäuremoleküle oder Vektoren, die ein Apoptose-induzierendes Molekül exprimieren, wobei das Apoptose-induzierende Molekül zur TNF-Superfamilie gehört.

In einer weiteren besonders bevorzugten Ausführungsform der erfindungsgemäßen rekombinanten Nucleinsäuremoleküle oder Vektoren ist die zu exprimierende Nucleotidsequenz eine Antisense-Sequenz oder exprimiert ein Ribozym.
Die Antisense-Sequenzen und Ribozyme sind Moleküle, deren Expression auf der Ebene der RNA stattfindet. Bei "Antisense-Sequenzen" handelt es sich um Sequenzen, die komplementär sind zu einer in der Zielzelle vorhandenen mRNA oder einem Teil davon, wobei der Teil die codierende Region, 5'- und/oder 3'-nichttranslatierte Region umfassen kann. Antisense-RNAs, d.h. die Transkripte der Antisense-Sequenz, sind in der Lage, an die komplementäre mRNA in vivo zu hybridisieren und auf diese Weise deren Translation zu inhibieren.
"Ribozyme" sind katalytische RNA-Moleküle. Vorzugsweise handelt es sich im Zusammenhang mit der vorliegenden Erfindung um Ribozyme, die spezifisch an eine mRNA binden können, um diese durch eine katalytische Aktivität für eine erfolgreiche Translation unzugänglich zu machen, vorzugsweise durch hydrolytische Spaltung. Anleitungen zur Auswahl von geeigneten Antisense-Sequenzen sowie zur Konstruktion von Ribozymen mit gewünschter Sequenzspezifizität sind in der Literatur beschrieben und können beispielsweise Antisense: "From Technology to Therapy" (Schlingensiepen, R., Brysch, W., Schlingensiepen, K.-H., eds., Blackwell Science Ltd. Oxford, 1997) bzw. Rossi (AIDS Research and Human Retroviruses 8 (1992), 183) entnommen werden.

Eine besonders bevorzugte Ausführungsform betrifft die vorgenannten rekombinanten Nucleinsäuremoleküle oder Vektoren, wobei die Antisense-Sequenz oder das Ribozym spezifisch ist für eine mRNA, die ein Cytokin oder ein co-stimulatorisches Molekül codiert.
Die rekombinanten Nucleinsäuremoleküle oder Vektoren der vorliegenden Ausführungsform können dazu verwendet werden, in Antigen-beladenen dendritischen Zellen gezielt die Expressionen eines Cytokins oder co-stimulatorischen Moleküls zu inhibieren. Hierzu können Antigen-beladene dendritische Zellen in vitro transfiziert werden, wobei die Antigen-Beladung vorzugsweise durch Co-Transfektion mit einem erfindungsgemäßen rekombinanten Nucleinsäuremolekül oder Vektor, der ein Antigen codiert, durchgeführt wird. Durch die gezielte Inhibition eines Cytokins oder co-stimulatorischen Moleküls läßt sich die Immunantwort gegen das Antigen modulieren. Weiterhin können die rekombinanten Nucleinsäuremoleküle oder Vektoren der vorliegenden Ausführungsform auch in vivo appliziert werden, um eine allgemeine Expression spezifisch für dendritische Zellen auszulösen. Durch eine solche Vorgehensweise ist es möglich, die allgemeine Immunsituation eines Patienten zu modulieren.

In einer weiteren besonders bevorzugten Ausführungsform der erfindungsgemäßen rekombinanten Nucleinsäuremoleküle oder Vektoren codiert die Nucleotidsequenz einen Transkriptionsfaktor.
Vorzugsweise handelt es sich bei den Transkriptionsfaktoren um solche, die die endogene Expression von Cytokinen oder co-stimulatorischen Molekülen in dendritischen Zellen induzieren. Die Expression eines Transkriptionsfaktors kann dabei die Induktion von mehreren Genen für Cytokine oder co-stimulatorische Faktoren gleichzeitig bewirken.
Die vorliegende Ausführungsform der rekombinanten Nucleinsäuremoleküle oder Vektoren kann allerdings auch dazu verwendet werden, die endogene Expression von Cytokinen oder co-stimulatorischen Molekülen in dendritischen Zellen zu inhibieren. Es ist bekannt, daß Transkriptionsfaktoren in Kombination mit anderen (endogenen) Transkriptionsfaktoren eine Repressoraktivität besitzen können.

In einer besonders bevorzugten Ausführungsform der oben beschriebenen rekombinanten Nucleinsäuremoleküle oder Vektoren enthalten die rekombinanten Nucleinsäuremoleküle oder Vektoren neben der einen zu exprimieren Nucleotidsequenz eine zweite zu exprimierende Nucleotidsequenz, wobei eine Nucleotidsequenz ein wie oben definiertes Antigen codiert und die zweite Nucleotidsequenz ein Protein, das eine Immunantwort reguliert. Die Immunantwort kann dadurch reguliert werden, daß die zweite Nucleotidsequenz ein Cytokin, ein co-stimulatorisches Molekül, ein Apoptose-induzierendes Molekül, einen Transkriptionsfaktor, eine Antisense-Sequenz oder ein Ribozym exprimiert. Dabei können diese zwei Nucleotidsequenzen in einem Leserahmen hintereinander geschaltet, d. h. translational fusioniert, sein (falls beide Nucleotidsequenzen Protein-codierend sind). Dabei können diese codierenden Regionen direkt aneinander grenzen oder durch einen Spacer getrennt sein. Durch einen Spacer wird die Tertiärstruktur der beiden Proteine voneinander räumlich getrennt, um zu verhindern, daß ihre Tertiärstrukturen negativ interagieren. Vorzugsweise hat der Spacer jedoch die Funktion, als Angriffsstelle für eine Protease zu fungieren, vorzugsweise für eine endogene Protease der transfizierten Zelle, so daß die exprimierten Proteine in vivo getrennt werden. Alternativ kann der Spacer eine IRES-Sequenz (internal ribosomal entry site) enthalten. Dies erlaubt die Transkription beider Gene unter der Kontrolle eines einzigen Promotors, wobei deren Translation getrennt erfolgt.
Andererseits können die zwei Nucleotidsequenzen auch unabhängig voneinander transkriptionell codiert werden. Hierzu steht jede Nucleotidsequenz unter der Kontrolle eines eigenen Promotors, wobei mindestens ein Promotor, vorzugsweise beide Promotoren die regulatorischen Sequenzen der vorliegenden Erfindung umfaßt.
Mit dieser Ausführungsform ließen sich die besonderen Vorteile, die eine Co-Transfektion mit Expressionskonstrukten, die ein Antigen bzw. ein immunregulatorisches Protein codieren, auf die in-vivo-Situation übertragen. Nach den oben beschriebenen Ausführungsformen ist solch eine Co-Transfektion von dendritischen Zellen nur in vitro möglich.
Durch die Anwendung von rekombinanten Nucleinsäuremolekülen oder Vektoren der vorliegenden Ausführungsform ist es möglich, durch direkte Applikation in den Körper einerseits gezielt eine Antigen-Präsentation durch dendritische Zellen hervorzurufen, andererseits die induzierte Immunantwort durch die Aktivität eines Mediators zu regulieren, wobei Nebenwirkungen durch unspezifische Wirkungen beispielsweise des Mediators weitgehend ausgeschlossen sind. Diese Ausführungsform beinhaltet sämtliche Antigene und immunregulatorischen Proteine, die in den vorgenannten Ausführungsformen definiert wurden.

In einer besonders bevorzugten Ausführungsform sind die oben beschriebenen Vektoren Viren.
Im Stand der Technik ist eine Vielzahl von viralen Vektoren zur Transfektion von Säugerzellen ex vivo oder in vivo beschrieben. Dabei handelt es sich stets um Abkömmlinge von Säuger- bzw. human-pathogenen Viren, die durch genetische Modifizierung ihrer pathogenen Eigenschaften beraubt wurden. Zur Transfektion werden virale Vektoren nach dem Fachmann bekannten Methoden in vitro verpackt, d. h. mit viralen Hüllproteinen versehen. Es können DNA- sowie auch RNA-Viren verwendet werden. Beispiele für Viren zur Transfektion von Säuger-, vorzugsweise Humanzellen sind Herpesvirus, Retroviren, Adenoviren und Adeno-assoziierte Viren.

In einer weiteren Ausführungsform der Erfindung sind die oben beschriebenen Vektoren zur Gentherapie oder DNA-Vakzinierung geeignet.
Gentherapie und DNA-Vakzinierung basieren auf der Einführung von therapeutischen bzw. immunisierenden Genen in Zellen ex vivo oder in vivo. Geeignete Vektoren oder Vektorsysteme und Methoden zu deren Einsatz für Gentherapie oder DNA-Vakzinierung sind in der Literatur beschrieben und dem Fachmann bekannt, siehe beispielsweise Giordano, Nature Medicine 2 (1996), 534-539; Schaper, Circ. Res. 79 (1996), 911-919; Anderson, Science 256 (1992), 808-813, Isner, Lancet 348 (1996), 370-374; Muhlhauser, Circ. Res. 77 (1995), 1077-1086; Wang, Nature Medicine 2 (1996), 714-716; WO 94/29469; WO 97/00957; Schaper, Current Opinion in Biotechnology 7 (1996), 635-640; oder Verma, Nature 389 (1997), 239-242 und darin zitierte Referenzen. Die oben beschriebenen rekombinanten Nucleinsäuremoleküle oder Vektoren können zur direkten Einführung oder zur Einführung über Liposomen oder virale Vektoren, z. B. adenoviral oder retroviral, ausgelegt werden.

In einer weiteren bevorzugten Ausführungsform der erfindungsgemäßen rekombinanten Nucleinsäuremoleküle oder Vektoren ist die zu exprimierende Nucleotidsequenz ein Reportergen.
Beispiele für Reportergene, mit denen man vorzugsweise in eukaryontischen Zellen, die Expressionsaktivität von regulatorischen Sequenzen, vorzugsweise von Promotoren detektieren kann, sind in der Literatur beschrieben. Beispiele für Reportergene codieren Luciferase, grün-fluoreszierendes Protein, β-Galactosidase oder Chloramphenicol-Acetyltransferase.

Eine weitere Ausführungsform der vorliegenden Erfindung betrifft ein Verfahren zur Herstellung von genetisch modifizierten Wirtszellen, dadurch gekennzeichnet, daß man Wirtszellen mit einem der oben beschriebenen Vektoren transfiziert und die transfizierte Wirtszelle in einem Kulturmedium kultiviert.
Der Begriff "genetisch modifiziert" bedeutet, daß die Wirtszelle oder der Wirt zusätzlich zum natürlichen Genom ein Nucleinsäuremolekül oder einen Vektor der vorliegenden Erfindung enthält, der in die Wirtszelle oder den Wirt oder einen Vorgänger eingeführt wurde. Das Nucleinsäuremolekül oder der Vektor kann in der genetisch modifizierten Wirtszelle/Wirt entweder als ein unabhängiges Molekül außerhalb des Genoms, vorzugsweise als replizierbares Molekül, oder stabil in das Genom der Wirtszelle oder des Wirts integriert vorliegen.
Die Einführung eines Vektors in Wirtszellen kann durchgeführt werden nach bekannten Standardverfahren, wie zum Beispiel in Sambrook et al. (a.a.O.) beschrieben. Beispiele für anwendbare Transfektionstechniken sind Calciumphosphat-Transfektion, DEAE-Dextran-vermittelte Transfektion, Elektroporation, Transduktion, Infektion, Lipofektion oder biolistischer Transfer. Zur anschließenden Kultivierung kann man ebenfalls auf Standardverfahren zurückgreifen oder, bei der genetischen Modifizierung von dendritischen Zellen, vorzugsweise nach dem in den Beispielen und den darin zitierten Referenzen beschriebenen Verfahren.

In einer weiteren bevorzugten Ausführungsform betrifft die Erfindung Wirtszellen, die genetisch modifiziert sind mit einem rekombinanten Nucleinsäuremolekül oder einem Vektor der vorliegenden Erfindung oder erhältlich sind nach dem oben beschriebenen Verfahren.
Die Wirtszelle der vorliegenden Erfindung kann prinzipiell jede prokaryontische oder eukaryontische Zelle sein, und umfaßt unter anderem Säugerzellen, Pilzzellen, Pflanzenzellen, Insektenzellen oder Bakterienzellen. Geeignete bakterielle Zellen sind solche, die allgemeine Verwendung finden für Clonierungen, wie z. B. E.coli oder Bacillus subtilis.
Beispiele für Pilzzellen sind Hefezellen, vorzugsweise solche der Gattungen Saccharomyces oder Pichia, besonders bevorzugt von Saccharomyces cerevisiae oder Pichia pastoris. Unter geeigneten tierischen Zellen sind beispielsweise Insektenzellen, Vertebratenzellen, vorzugsweise Säugerzellen, wie z. B. CHO, Hela, NIH3T3, MOLT-4, Jurkat, K562, HepG2 oder PC12 zu zählen. Weitere geeignete Zellinien sind im Stand der Technik beschrieben und lassen sich beispielsweise von der Deutschen Sammlung für Mikroorganismen und Zellkulturen (DSMZ, Braunschweig) beziehen.

Besonders bevorzugt ist die Ausführungsform der Wirtszellen, die dendritische Zellen sind.

Dendritische Zellen sind der primäre Anwendungsort der erfindungsgemäßen regulatorischen Sequenzen, rekombinanten Nucleinsäuremoleküle oder Vektoren. Dendritische Zellen lassen sich beispielsweise aus peripheren Blutleukocyten und Langerhanszellen aus epidermalen Präparationen gewinnen, wobei es sich bei den isolierten Zellen auch um Vorläuferzellen handeln kann, die durch eine geeignete in vitro-Kultivierung zu dendritischen Zellen umgewandelt werden können. Methoden hierzu sind im Stand der Technik beschrieben und lassen sich beispielsweise auch aus den Beispielen wie auch aus Ross (J. Invest. Dermatol. 115 (2000), 658-663), Ross (J. Immunol. 160 (1998), 3776-3782) oder darin zitierten Referenzen entnehmen. Diese Quellen liefern ebenfalls Methoden zur Kultivierung von dendritischen Zellen.

Besonders bevorzugt sind im Rahmen der Erfindung Wirtszellen, die aus Mensch stammen.

Eine weitere bevorzugte Ausführungsform der Erfindung betrifft Nucleinsäurefragmente mit einer Länge von mindestens 15 Nucleotiden, die spezifisch mit einem Strang einer erfindungsgemäßen regulatorischen Sequenz unter stringenten Bedingungen hybridisieren, wobei die Sequenz des Nucleinsäurefragments zu der Sequenz unter SEQ ID NO:1 oder Position 1 bis 3069 von SEQ ID NO:72 eine Sequenzidentität von mindestens 50% aufweist. Hybridisierende Nucleotidsequenzen gemäß der vorliegenden Ausführungsform können beispielsweise als Sonden dienen, die beispielsweise zur Identifikation homologer Promotoren, vorzugsweise regulatorischer Sequenzen anderer Gene, die aufgrund bestimmter übereinstimmender Sequenzelemente ein den erfindungsgemäßen regulatorischen Sequenzen vergleichbares Expressionsmuster induzieren, beitragen. Des weiteren können diese Sequenzen zum Design geeigneter Oligonucleotide, beispielsweise als PCR-Primer, verwendet werden.
Der Begriff "Hybridisierung" ist weiter oben bereits definiert worden. Vorzugsweise hybridisieren die Nucleotidsequenzen unter stringenten Bedingungen. Die Fragmente haben eine Länge von mindestens 15 Nucleotiden, vorzugsweise von mindestens 20 Nucleotiden, besonders bevorzugt von mindestens 50 Nucleotiden, insbesondere bevorzugt von mindestens 100 Nucleotiden, vorteilhafterweise von mindestens 200 Nucleotiden und am meisten bevorzugt von mindestens 500 Nucleotiden.

Eine weitere bevorzugte Ausführungsform der Erfindung betrifft ein Verfahren zur Antigen-spezifischen Stimulierung von T-Zellen in vitro, enthaltend die Schritte:
(a) Transfektion von dendritischen Zellen mit einem Vektor, der eine Nucleotidsequenz enthält, die ein Antigen codiert, allein oder in Kombination mit einem Vektor, der eine Nucleotidsequenz enthält, die ein immunregulatorisches Protein codiert, wobei die Regulation vorzugsweise eine Steigerung der Immunantwort ist, und wobei die Nucleotidsequenz vorzugsweise ein Cytokin oder ein co-stimulatorisches Molekül, eine Antisense-Sequenz oder ein Ribozym exprimiert, oder mit einem Vektor, der sowohl ein Antigen als auch ein immunregulatorisches Protein codiert;
(b) Cokultivierung der durch Schritt (a) gewonnenen transfizierten dendritischen Zellen mit T-Zellen; und
(c) Detektion der Aktivierung der T-Zellen aus Schritt (b).

Die Verfahrensschritte sind im Prinzip im Stand der Technik beschrieben und lassen sich beispielsweise WO94/02156 entnehmen. Auf die Bereitstellung von dendritischen Zellen ist bereits weiter oben eingegangen worden. Ein Verfahren hierzu läßt sich den Beispielen im Anhang entnehmen. Die Präparation von T-Zellen ist nach Verfahren aus dem Stand der Technik vorzunehmen, wie beispielsweise in "Current Protocols in Immunology" (Coligan, J.E., Kruisbeek, A.M., Margulies, D.H., Shevach, E.M., Strober, W., eds., Greene Publishing Associates and Whiley-Interscience, New York, 1991, Vol. 1) ausgeführt. Der Vorteil des hier vorgestellten Verfahrens gegenüber dem Stand der Technik betrifft die Verwendung der erfindungsgemäßen Vektoren. Da diese die Expression in dendritischen Zellen in spezifischer Weise erlauben, lassen sich nun Zellpopulationen verarbeiten, die einen weitaus niedrigeren Aufreinigungsgrad für dendritische Zellen benötigen als für herkömmlichen Verfahren notwendig ist, ohne Nebenwirkungen oder Risiken durch die Transfektion von nicht-dendritischen Zellen befürchten zu müssen.
Bei den in Schritt (b) zur Co-Kultivierung eingesetzten T-Zellen kann es sich um naive oder aktivierte T-Zellen handeln. Die Detektion der Aktivierung der T-Zellen in Schritt (c) kann nach einem oder mehreren der folgenden Methoden erfolgen: Messung der Proliferation, Detektion der cytotoxischen Aktivität, Detektion der Cytokinproduktion, Detektion der metabolischen Aktivität und Detektion von Aktivierungsmarkern.

Eine weitere bevorzugte Ausführungsform betrifft ein Verfahren zur Herstellung eines Arzneimittels umfassend die Schritte (a) bis (c) des Verfahrens zur Antigen-spezifischen Stimulierung von T-Zellen in vitro und zusätzlich den Schritt
(d) Formulierung eines Arzneimittels durch Versetzen der durch den Schritt (c) gewonnenen stimulierten T-Zellen mit einem pharmazeutisch verträglichen Träger.

Zur Formulierung von Zellen zur Verabreichung als Arzneimittel werden die Zellen in einem pharmazeutisch verträglichen Trägermaterial suspendiert. Dies gilt für die stimulierten T-Zellen wie auch für die im folgenden beschriebenen dendritischen Zellen. Beispiele für Trägermaterial sind Wasser, Kochsalzlösung, Dextrose, Glycerin, etc. oder Kombinationen davon. Zusätzlich kann die zu verabreichende Zellsuspension weitere Stoffe wie Emulsionsmittel, pH-Puffer, Adjuvanzien, oder auch immunregulatorische Faktoren, wie Cytokine, enthalten.
T-Zellen, die nach dem oben beschriebenen Verfahren stimuliert wurden, können beispielsweise zur Bekämpfung von schweren Virusinfektionen, wie z.B. gezeigt für CMV bei immunschwachen Patienten (Walter, New Engl. J. Med. 333 (1995), 1038-1044), oder zur Induktion der immunabwehr von Metastasen (Nestle, Nature Med. 4 (1998), 328-332) eingesetzt werden.

Eine weitere Ausführungsform der Erfindung betrifft ein Verfahren zur Herstellung von T-Zell-stimulierenden dendritischen Zellen in vitro, enthaltend die Schritte:
(a) Transfektion von dendritischen Zellen mit einem Vektor, der eine Nucleotidsequenz enthält, die ein Antigen codiert, allein oder in Kombination mit einem Vektor, der eine Nucleotidsequenz enthält, die ein immunregulatorisches Protein, ein Apoptose-induzierendes Molekül, vorzugsweise zur TNF-Superfamilie gehörend, codiert oder eine Antisense-Sequenz oder ein Ribozym exprimiert, oder mit einem Vektor, der sowohl ein Antigen als auch ein immunregulatorisches Protein codiert; und
(b) Kultivierung der transfizierten dendritischen Zellen in einem geeigneten Medium und/oder Detektion der T-Zell-stimulierenden Aktivität. Eine weitere bevorzugte Ausführungsform betrifft ein Verfahren zur Herstellung eines Arzneimittels umfassend die Schritte (a) und (b) des Verfahrens zur Herstellung von T-Zellen stimulierenden dendritischen Zellen und zusätzlich den Schritt
(c) Formulierung eines Arzneimittels durch Versetzen der durch den Schritt (b) gewonnenen T-Zell-stimulierenden dendritischen Zellen mit einem pharmazeutisch verträglichen Träger.

Die durch dieses Verfahren gewonnenen dendritischen Zellen können als Arzneimittel Patienten verabreicht werden, um durch T-Zell-Aktivierung gezielte Immunantworten auszulösen. Die dendritischen Zellen können intradermal, subcutan, intravenös oder, im Falle einer Tumorbehandlung, in Regionen des Tumorwachstums oder in Lymphgefäße, die diese Regionen drainieren, injiziert werden.

In einer besonders bevorzugten Ausführungsform des oben beschriebenen Verfahrens stammen die dendritischen Zellen aus Mensch.

Eine weitere bevorzugte Ausführungsform der vorliegenden Erfindung betrifft Arzneimittel umfassend die rekombinanten Nucleinsäuremoleküle und die Vektoren der vorliegenden Erfindung, die Wirtszellen, Antigen-spezifisch stimulierte T-Zellen, erhältlich nach dem oben beschriebenen Verfahren, oder T-Zell-stimulierende dendritische Zellen, erhältlich nach dem oben beschriebenen Verfahren, und optional einen pharmazeutisch verträglichen Träger.

In einer besonders bevorzugten Ausführungsform der Erfindung ist das Arzneimittel ein Impfstoff.
Die regulatorischen Sequenzen, rekombinanten Nucleinsäuremoleküle, Vektoren oder Wirtszellen der Erfindung können verwendet werden zur Herstellung eines Impfstoffs, d. h. im Sinne der Erfindung eines DNA-Vakzins. Modi der Verabreichung von DNA-Vakzinen sind im Stand der Technik beschrieben und DNA-Vakzinierung wurde bereits erfolgreich eingesetzt, um Antitumorimmunantworten auszulösen (Tighe M. et al., Immunology Today 19 (1998), 89-97). Darüber hinaus konnte bereits gegen verschiedene Krankheitsformen ein Immunschutz durch Verabreichung von DNA-Nucleinsäuremolekülen erzielt werden (Fynan, Proc. Natl. Acad. Sci. U.S.A. 90 (1993), 11478-11482; Boyer, Nat. Med. 3 (1997), 526-532; Webster, Vaccine 12 (1994), 1495-1498; Montgomery et al., DNA Cell Biol. 12 (1993), 777-783; Barry, Nature 311 (1995), 632-635; Xu and Liew, Immunology 84 (1995), 173-176; Zhoug, Eur. J. Immunol. 26 (1996), 2749-2757; Luke, J. Inf. Dis. 175 (1997), 91-97; Mor, Biochem. Pharmacology 55 (1998), 1151-1153; Donelly, Annu. Rev. Immun. 15 (1997), 617-648; MacGregor, J. Infect. Dis. 178 (1998), 92-100).
Die erfindungsgemäßen Nucleinsäuremoleküle zur Verwendung als Impfstoff können in einer neutralen Form oder als Salz formuliert werden. Pharmazeutisch wirksame Salze sind dem Fachmann geläufig. Die Impfstoffe der Erfindung können, unter anderem, verwendet werden zur Behandlung und/oder Prävention von Infektionen und werden in Dosierungen verabreicht, die pharmakologisch wirksam sind zur Prophylaxe oder Behandlung.
Die im Sinne der Erfindung anzuwendenden Vakzinierungsprotokolle beinhalten aktive Immunisierung, wobei die Verabreichung von Nucleinsäuremolekülen, die gezielt in den dendritischen Zellen einer Person Antigene oder Allergene exprimieren, eine protektive Immunantwort auslösen sollen. Zusätzlich beinhalten die Vakzinierungsprotokolle die Kombination der Antigenexpression mit immunmodulatorischen Effekten durch zusätzliche Verabreichung von Nucleinsäuren, die entsprechende Mediatoren ebenfalls gezielt in dendritischen Zellen exprimieren oder auch weitere unterstützende medikamentöse Behandlung.
Weitere Strategien zur Erreichung eines Impfschutzes beinhalten die Verabreichung in vitro-transfizierter dendritischer Zellen, in vitro-aktivierter T-Zellen, jeweils nach den oben beschriebenen Verfahren. Methoden und Prinzipien sind dem Stand der Technik zu entnehmen und zum Beispiel beschrieben in Paul, "Fundamental Immunology" Raven Press, New York (1989) oder Morein, "Concepts in Vaccine Development", ed: S.H.E. Kaufmann, Walter de Gruyter, Berlin, New York (1996), 243-264.
Typischerweise werden Impfstoffe zur Injektion hergestellt als flüssige Lösung oder Suspension. Die Präparationen können emulgiert sein oder der Wirkstoff kann in Liposomen eingekapselt werden. Die Wirkstoffe werden oftmals mit Trägermaterial gemischt, die mit dem Wirkstoff kompatibel sind. Beispiele für Trägermaterial sind Wasser, Kochsalzlösung, Dextrose, Glycerin, Ethanol etc. oder Kombinationen davon. Der Impfstoff kann auch Hilfssubstanzen wie Emulsionsmittel, pH-Puffer und/oder Adjuvanzien enthalten.
Statt durch Injektion kann DNA durch biolistischen Transfer (US-Patent No. 5,100,702; Kalkbrenner, Meth. Mol. Biol. 83, 1996, 203-216) zur Vakzinierung verabreicht werden. Hierzu wird DNA, d. h. rekombinante Nucleinsäuremoleküle oder Vektoren der vorliegenden Erfindung an kleine Partikel, beispielsweise Goldpartikel oder Partikel aus biokompatiblem Material gebunden und beschleunigt durch Gasdruck in die Epidermis oder Dermis eingebracht. DNA kann ebenfalls oral oder sublingual verabreicht werden oder auf die Schleimhäute des Respirationstraktes durch nasale oder intratracheale Applikation aufgetragen werden (Beispiele hierzu in Etchart, J. Gen. Virol. 78 (1997), 1577-1580 oder McCluskie, Antisense and Nucleic Acid Drug Development 8 (1998), 401-414).

Eine weitere bevorzugte Ausführungsform der Erfindung betrifft die Verwendung der erfindungsgemäßen rekombinanten Nucfeinsäuremoleküle oder Vektoren, die eine Nucleotidsequenz exprimieren, die vorzugsweise ein Antigen codiert, das besonders bevorzugt tumor- oder pathogenspezifisch oder ein Allergen ist, wobei der Vektor vorzugsweise ein Virus ist oder zu Gentherapie oder DNA-Vakzinierung geeignet ist, allein oder in Kombination mit den erfindungsgemäßen rekombinanten Nucleinsäuremolekülen oder Vektoren, die ein immunmodulatorisches Protein exprimieren, das vorzugsweise ein Cytokin oder co-stimulatorisches Molekül ist, dessen Regulation vorzugsweise eine Steigerung der Immunantwort ist, oder die einen Transkriptionsfaktor exprimieren, sowie die Verwendung der erfindungsgemäßen rekombinanten Nucleinsäuremoleküle oder Vektoren, die sowohl ein Antigen als auch ein immunregulatorisches Protein codieren, der erfindungsgemäßen Wirtszellen, Antigen-spezifisch stimulierten T-Zellen, erhältlich nach dem oben beschriebenen Verfahren, oder T-Zell-stimulierenden dendritischen Zellen, erhältlich nach dem oben beschriebenen Verfahren, zur Herstellung eines Arzneimittels zur Impfung gegen Viren, Bakterien, Pilze, Parasiten, Tumoren, Allergene, Creutzfeldt-Jakob-Plaques oder Alzheimer-Plaques oder zur gentherapeutischen Behandlung von Tumoren oder viralen, bakteriellen oder parasitischen Infektionen oder von Allergien.

Eine weitere bevorzugte Ausführungsform der Erfindung betrifft die Verwendung der erfindungsgemäßen rekombinanten Nucleinsäuremoleküle oder Vektoren, die eine Nucleotidsequenz exprimieren, die vorzugsweise ein Antigen codiert, das besonders bevorzugt ein Autoantigen, Transplantationsantigen oder Allergen ist, wobei der Vektor vorzugsweise ein Virus ist oder zu Gentherapie oder DNA-Vakzinierung geeignet ist, allein oder in Kombination mit den erfindungsgemäßen rekombinanten Nucleinsäuremolekülen oder Vektoren, die ein immunregulatorisches Protein exprimieren, wobei die Regulation vorzugsweise eine Hemmung ist, oder die ein Apoptose-induzierendes Molekül, einen Transkriptionsfaktor, oder eine Antisense-Sequenz oder ein Ribozym exprimieren, sowie die Verwendung der erfindungsgemäßen rekombinanten Nucleinsäuremoleküle oder Vektoren, die sowohl ein Antigen als auch ein immunregulatorisches Protein codieren, oder der erfindungsgemäßen Wirtszellen zur Herstellung eines Arzneimittels zur Behandlung von Autoimmunerkrankungen, Transplantatabstoßung oder Allergien.

Eine weitere bevorzugte Ausführungsform der Erfindung betrifft die Verwendung der erfindungsgemäßen rekombinanten Nucleinsäuremoleküle oder Vektoren, die ein Apoptose-induzierendes Molekül codieren, das vorzugsweise zur TNF-Superfamilie gehört, wobei der Vektor vorzugsweise ein Virus ist oder zu DNA-Vakzinierung oder Gentherapie geeignet ist, zur Herstellung eines Arzneimittels zur Vermeidung der Abstoßung von Transplantaten und von Autoimmunreaktionen. Dentritische Zellen, welche in einer Blutprobe des Spenders eines Transplantates enthalten sind, exprimieren nach Aufnahme des Expressionsvektors das codierte Apoptose-induzierende Molekül. Nach Injektion in den Empfänger des Transplantates bringen diese dendritischen Zellen T-Zellen, die die Transplantationsantigene spezifisch erkennen, zum Absterben. Bei Autoimmunreaktionen bringen Autoantigen-beladene dendritische Zellen, welche nach Aufnahme des Expressionsvektors das codierte Apoptose-induzierende Molekül exprimieren, T-Zellen, die das Autoantigen spezifisch erkennen, zum Absterben.

Ferner betrifft die vorliegende Erfindung die Verwendung der erfindungsgemäßen regulatorischen Sequenzen, der rekombinanten Nucleinsäuremoleküle oder Vektoren zur Herstellung eines Arzneimittels zur Immuntherapie durch spezifische Expression von Antigenen oder immunregulatorischen Proteinen in dendritischen Zellen.

In einer weiteren bevorzugten Ausführungsform betrifft die vorliegende Erfindung die Verwendung der erfindungsgemäßen regulatorischen Sequenzen oder der rekombinanten Nucleinsäuremoleküle oder Vektoren, die vorzugsweise ein Reportergen exprimieren, zur Identifizierung und Isolierung von cis-Elementen aus der regulatorischen Sequenz, die eine für dendritische Zellen spezifische Expression vermitteln.

In einer weiteren bevorzugten Ausführungsform betrifft die vorliegende Erfindung die Verwendung der erfindungsgemäßen regulatorischen Sequenzen oder der rekombinanten Nucleinsäuremoleküle oder Vektoren, die vorzugsweise ein Reportergen exprimieren, zur Bestimmung des Reifegrads von dendritischen Zellen. Diese Ausführungsform läßt sich beispielsweise zur Bestimmung des Reifegrads von in vitro-kultivierten dendritischen Zellen, die in klinischen Studien eingesetzt werden sollen, nutzen.

Eine weitere Ausführungsform der Erfindung betrifft die Verwendung der erfindungsgemäßen regulatorischen Sequenzen oder der rekombinanten Nucleinsäuremoleküle oder Vektoren zur Identifizierung und Isolierung von Faktoren, die eine Expression spezifisch für dendritische Zellen vermitteln.

Ferner betrifft die vorliegende Erfindung die Verwendung der erfindungsgemäßen regulatorischen Sequenzen, die eine Nucleotidsequenz aus einer der unter SEQ ID NO: 1 bis 8 angegebenen Sequenzen bzw. einer entsprechenden Promotorsequenz aus SEQ ID NO:72 umfassen oder die eine Nucleotidsequenz umfassen, die in der Insertion des Klons DSM13274 enthalten ist und durch Amplifikation unter Verwendung eines Paares von Oligonucleotiden erhältlich ist, deren Sequenzen unter einem der folgenden Paare von SEQ ID-Nummern angegeben ist: 36 und 37; 38 und 37; 39 und 37; 40 und 37; 41 und 37; 42 und 37; 43 und 37; oder 44 und 37; Teilen davon oder Sequenzen, die mit den vorgenannten spezifisch hybridisieren, zur Herstellung eines Arzneimittels zur Immuntherapie durch Inhibierung der Primärstimulation von T-Zellen, wobei die regulatorische Sequenz oder Teile davon in dendritischen Zellen als Transkriptionsfaktor-Bindungsstellen dienen, die Transkriptionsfaktoren blockieren. Da die erfindungsgemäßen regulatorischen Sequenzen in ihrem Ursprungsgen eine Stadien-spezifische Expression in dendritischen Zellen vermitteln (Fascin wird nicht in unreifen dendritischen Zellen, wohl aber zunehmend in reiferen Stadien exprimiert), ist es möglich, durch Blockierung von Transkriptionsfaktoren, die die Stadienspezifität vermitteln, den Übergang von unreifen dendritischen Zellen in reifere Stadien zu inhibieren. Hierzu können die regulatorischen Sequenzen der Erfindung bzw. Teile davon, vorzugsweise in Form von Oligonucleotiden verwendet werden. Durch die Inhibition der Ausreifung der dendritischen Zellen wird indirekt die Primärstimulation von T-Zellen inhibiert. Dies kann beispielsweise bei Gewebetransplantationen zur Verhinderung von Abstoßungsreaktionen genutzt werden.

Diese und andere Ausführungsformen sind dem Fachmann offenbart und offensichtlich und umfaßt durch die Beschreibung und die Beispiele der vorliegenden Erfindung. Weiterführende Literatur kann zu einer der oben angeführten Methoden, Mittel und Verwendungen, die im Sinne der vorliegenden Erfindung angewendet werden können, dem Stand der Technik entnommen werden, z. B. aus öffentlichen Bibliotheken unter z. B. der Benutzung von elektronischen Hilfsmitteln. Zu diesem Zweck bieten sich unter anderem öffentliche Datenbanken an wie die "Medline", die über Internet zur Verfügung stehen, z. B. unter der Adresse http://www.ncbi.nlm.nih.gov/PubMed/medline.html. Weitere Datenbanken und Adressen sind dem Fachmann geläufig und können aus dem Internet entnommen werden, z. B. unter der Adresse http://www.lycos.com. Eine Übersicht über Quellen und Informationen zu Patenten bzw. Patentanmeldungen in der Biotechnologie ist in Berks, TIBTECH 12 (1994), 352-364 gegeben.

Die Figuren zeigen:

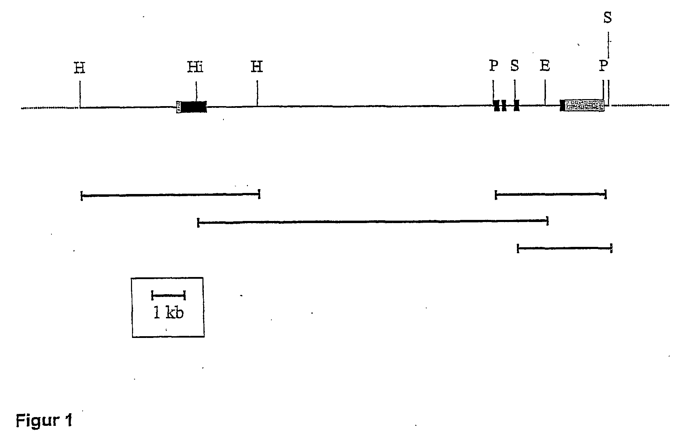

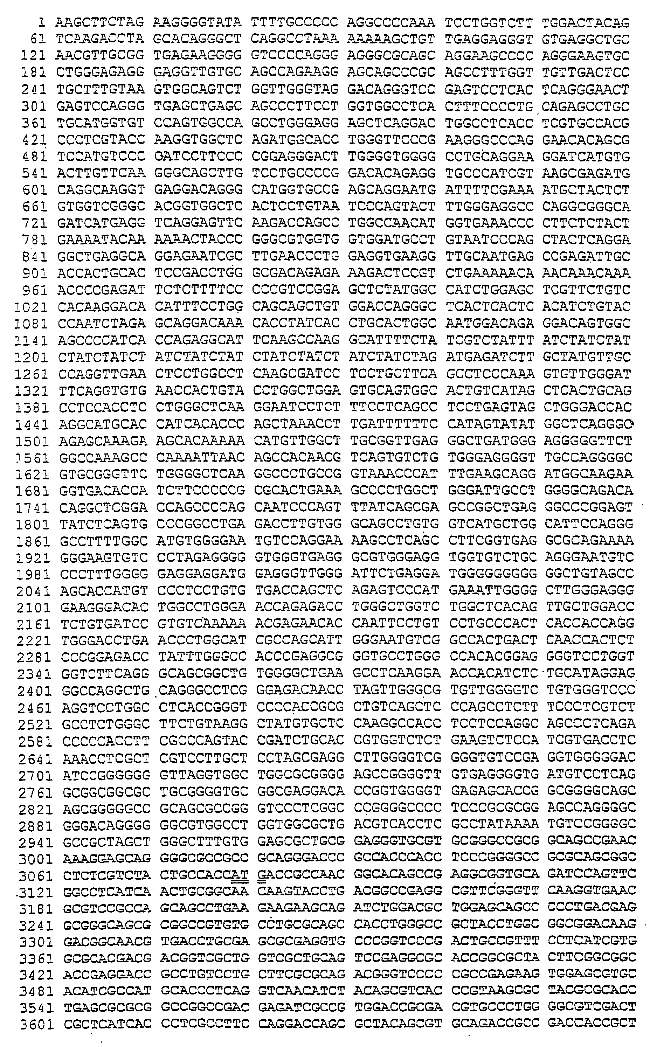

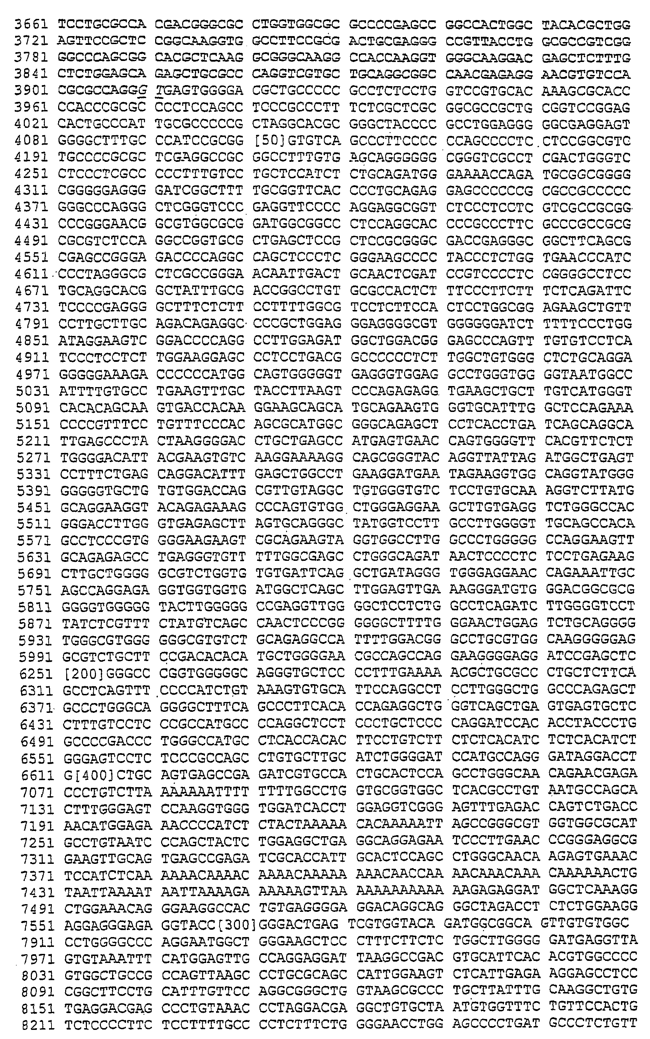

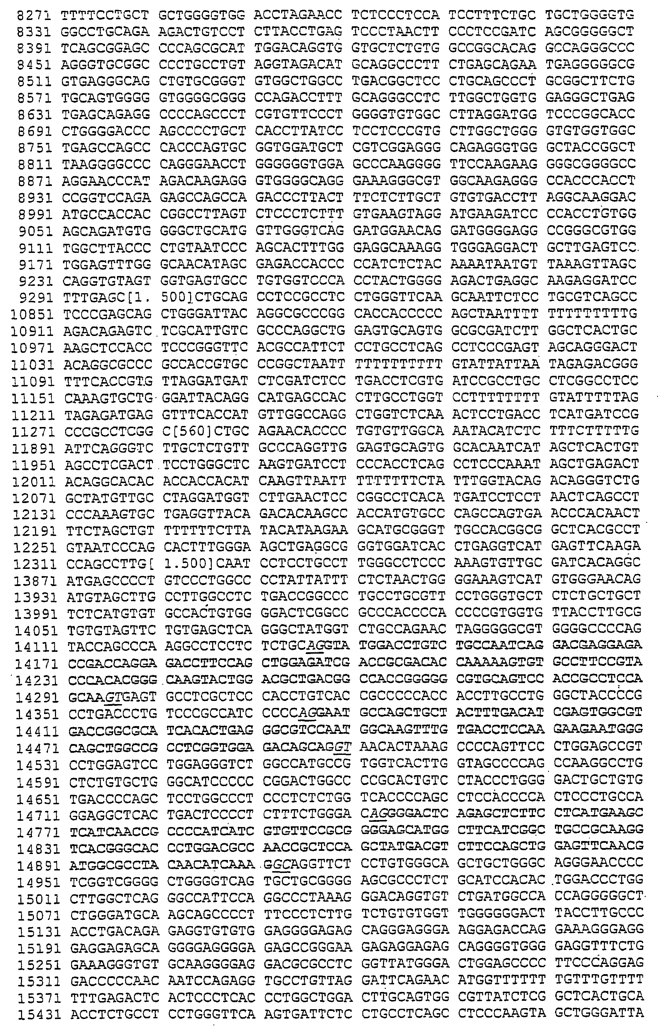

- **Figur 1:**: Genomische Organisation des humanen Fascingens. *Oben:* Schematische Wiedergabe des Genlocus. Das Gen erstreckt sich über etwa 13 kb und besteht aus fünf Exons (als Kästchen hervorgehoben, grau: untranslatierte, schwarz: proteincodierende Bereiche). Unten: Lage und Größe der aus dem PAC-Klon RPCIP704C24766Q3/4 subklonierten genomischen Fragmente, die für detailliertere Studien verwendet worden sind. Die für die jeweilige Subklonierung genutzten Restriktionsschnittstellen sind oben eingezeichnet. H: *Hind III*, Hi: *Hinc II*, P: *Pst I*, S: *Sac I,* E: *Eco RI*.
- **Figur 2:**: Nucleotidsequenz des humanen Fascingens. Exonsequenzen sind fett wiedergegeben. Das Start- und Stopcodon sowie das putative Polyadenylierungssignal sind jeweils doppelt unterstrichen. Exon-Intron-Spleißstellen sind kursiv und unterstrichen. (Die Länge der noch nicht sequenzierten Genabschnitte ist in eckigen Klammern angegeben. Für zwei Teilsequenzen in Intron 1 ist die korrekte Orientierung wegen der flankierenden Sequenzierlücken noch nicht bekannt.)

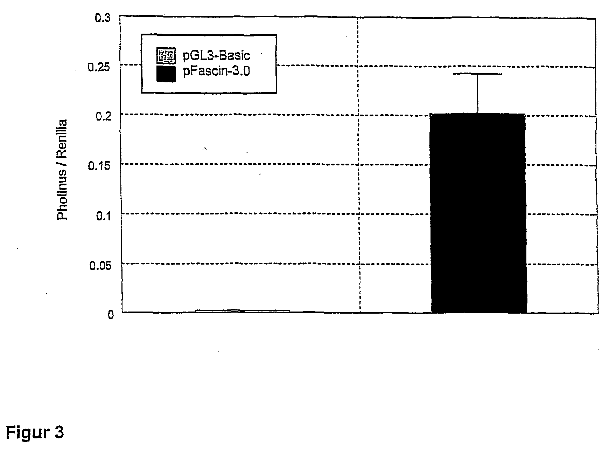

- **Figur 3:**: Expressionsstärke des humanen Fascinpromotors (pFascin-3.0) in dendritischen Zellen (DC), die aus CD14⁺ Vorläuferzellen des peripheren Blutes generiert wurden, im Vergleich zur Negativkontrolle (pGL3-Basic). Angegeben ist die normalisierte Expressionsstärke des getesteten Promoterfragments als Quotient aus den Luciferaseaktivitäten für das Testkonstrukt pFascin-3.0 (Photinus-Luciferase) und den konstitutiv exprimierten Coreporter pRL-CMV (Renilla-Luciferase). Angegeben ist der Mittelwert ± SEM (Standardfehler) für die in Triplikatansätzen getesteten Konstrukte.
- **Figur 4:**: Lage und Größe der getesteten Deletionskonstrukte des humanen Fascinpromotors im Vergleich zum Fascin-Gen (oben). Dargestellt sind der 5'-genflankierende genomische Bereich (ungefüllter Abschnitt), der transkribierte nichttranslatierte 5'-Genbereich (5'-UTR, grau hinterlegt) und der translatierte Abschnitt (schwarz hinterlegt) von Exon 1 sowie der flankierende Anteil von Intron 1. Der putative Promotorbereich wurde in den Vektor pGL3-Basic vor das promotorlose Reportergen Photinus-Luciferase cloniert (pFascin-3.0). 5'-verkürzte Deletionskonstrukte wurden durch gerichtete "nested deletion" bzw. Religationen hergestellt. Die jeweilige Größe der Promotorkonstrukte (abzüglich des 5'-UTR-Anteils) ist als Klonbezeichnung (in Kb) angegeben.
- **Figur 5:**: Nachweis der Abwesenheit endogener Fascinexpression in der humanen Monocytenlinie THP-1 mittels RT-PCR. **A**: Amplifiziert wurde ein 266 bp großes Fragment aus dem 3'-UTR der Fascin-cDNA (hFascin-UTR). *Spur 1*: Positivkontrolle (in pUC18 kloniertes PCR-Produkt hFascin-UTR); *2*: Negativkontrolle (H₂O); *3*: SHSY5Y (Neuroblastomlinie; exprimiert konstitutiv Fascin); *4*: THP-1. **B**: Für die in A dargestellte Fascin-PCR wurden die einzusetzenden cDNA-Mengen mittels HPRT-PCR standardisiert. *Spur 1:* THP-1; *2*: SHSY5Y; *3*: Negativkontrolle. In **A** und **B** ist in der jeweils ersten Spur der Molekulargewichtsmarker (φX174, *Hae III*-restringiert) aufgetragen.
- **Figur 6:**: Expressionsstärken der Deletionskonstrukte des humanen Fascinpromoters in DC und der Monocytenlinie THP-1. Die normalisierten Werte für die Reportergenexpression der einzelnen Promotorkonstrukte sind auf die Expressionsstärke des Minimalpromoters (pFascin-0.11) mit der Einheit eins normiert. Angegeben ist der Mittelwert ± SEM für die in Triplikatansätzen getesteten Konstrukte.
- **Figur 7:**: Vergleich der Aktivität des Fascinpromotors in CD83⁺ und CD83⁻ Zellen einer DC-Kultur. Die relative Expressionsstärke dreier Humanfascin-Promotorkonstrukte zunehmender Länge (*pFascin-0.11, pFascin-1.6, pFascin-3.0*) wurde vergleichend in der CD83-positiven und -negativen Zellfraktion einer DC-Kultur getestet. Angegeben ist die relative Expressionsstärke jedes Promotorkonstrukts im Vergleich zum kürzesten Konstrukt mit Basalaktivität (*pFascin-0.11*), dessen relative Luciferaseaktivität auf 1 normiert ist. Als Negativkontrolle diente der promotorlose Vektor pGL3-Basic (*pGL3-Basic*). Angegeben sind die Mittelwerte ± SEM von zwei Experimenten, die jeweils in Triplikaten durchgeführt wurden. Unterschiede in der Transfektionseffizienz wurden durch Cotransfektion mit pRL-CMV normalisiert.
- **Figur 8:**: Zelltypspezifische Aktivität des humanen Fascinpromotors. Die zelltypspezifische Aktivität des humanen Fascinpromotors (*pFascin-3.0*) ist relativ zur Expressionsstärke einer Positivkontrolle angegeben. Als Positivkontrolle wurde ein Luciferasekonstrukt eingesetzt, bei dem die Expression des Reporters Luciferase unter Kontrolle des Promotors des Haushaltsgens EF1α erfolgt, der in den untersuchten Zellen eine starke, Zelltyp-unabhängige Expression vermittelt. Beide Konstrukte wurden in humanen Zelltypen getestet: einer Fascin-negativen Keratinozytenlinie (*HaCaT*), einer Fascin-exprimierenden Neuroblastomlinie (*SHSY-5Y*) sowie in stark Fascin-positiven reifen Dendritischen Zellen (*DC*). Angegeben sind die Mittelwerte ± SEM von drei (*HaCaT*, *SHSY-5Y*) bzw. zwei (*DC*) Experimenten, die jeweils in Triplikaten durchgeführt wurden. Unterschiede in der Transfektionseffizienz wurden durch Cotransfektion mit pRL-CMV normalisiert.
- **Figur 9:**: Nucleotidsequenz des humanen Fascingens. Exonsequenzen sind fett wiedergegeben. Das Start- und Stopcodon sowie das putative Polyadenylierungssignal sind jeweils doppelt unterstrichen. Exon-Intron-Spleißstellen sind kursiv und unterstrichen.

Die nachfolgenden Beispiele erläutern die Erfindung.

### Methodisches Setup

### Zellkultur und Erzeugung von humanen dendritischen Zellen

Die humane Monozytenlinie THP-1 (Tsuchiya, Int. J. Cancer 26 (1980), 171-176) wurde in RPMI 1640 kultiviert, die humane Keratinozytenlinie HaCaT (Boukamp, J Cell Biol. 106 (1988), 761-771) in IMDM und die humane Neuroblastomlinie SHSY-5Y (Vinores, Cancer Res. 44 (1984),:2595-2599) in einer gleichvolumigen Mischung aus DMEM und Nut Mix F12. Die Kulturmedien wurden jeweils mit 10 % fötalem Kälberserum (FCS), 2 mM L-Glutamin, 1 mM Natriumpyruvat, 100 IU/ml Penicillin sowie 100 µg/ml Streptomycin ergänzt. Alle Kulturmedien und -zusätze wurden von Life Technologies (Eggenstein) bezogen.
Menschliche dendritische Zellen (DC) wurden hergestellt aus peripheren mononucleären Blutzellen (peripheral blood mononuclear cells, PBMC) wie beschrieben in Jonuleit (Eur. J. Immunol. 27 (1997), 3135-3142). Sämtliche Zentrifugationsschritte wurden bei Raumtemperatur ausgeführt. Etwa 80 ml *buffy coat*, das aus gesunden Blutspendern gewonnen wurde, wurde 1:3 mit Phosphatgepufferter Kochsalzlösung (PBS) verdünnt, die 2 mM EDTA und 0,4 IU/ml Natriumheparin enthielt. 15 ml Ficoll-Histopaque (Biochrom, Berlin) wurden mit dem doppelten Volumen des verdünnten buffy coats überschichtet. Nach Zentrifugation bei 1000 rpm über 20 min wurde ein 5 ml-Aliquot der oberen Phase (Serumfraktion) entfernt und Hitze-inaktiviert bei 56°C für 30 min. Die Serumfraktion wurde bei 3000 rpm für 5 min zentrifugiert und der Überstand wurde für nachfolgende Anwendungen als autologes Plasma bei 4°C aufbewahrt. Der Ficoll-Gradient wurde ein zweites Mal zentrifugiert (für 15 min bei 1500 rpm) und die PBMC-enthaltende Interphase wurde vorsichtig entfernt unter Verwendung einer Pasteur-Pipette. Die PMBC wurden mit 30 ml PBS, das 2 mM EDTA enthielt, verdünnt und dreimal gewaschen mit PBS/ 2 mM EDTA. Die Zellzahl wurde bestimmt und die Zellen wurden für eine Stunde bei 37°C in X-Vivo 15 (Bio-Whittaker, Walkersville, USA), das 3% des autologen Plasmas enthielt, in 6-Loch-Zellkultur Clusterplatten (Corning Costar, Bodenheim) in einem Volumen von 3 ml (5x10⁶ Zellen/ml) inkubiert. Daraufhin wurde der Überstand entfernt, der die nicht adhärenten Leukocyten enthielt, und ersetzt durch neues Kulturmedium, das durch 800 U/ml rekombinantes menschliches (rh) GM-CSF und 1000 U/ml rhIL-4 ergänzt wurde. Die Cytokinbehandlung induzierte die Reifung der ursprünglich adhärenten monocytischen Zellen zu unreifen dendritischen Zellen. An jedem zweiten Tag wurde 1 ml des Mediums ersetzt durch Medium, das mit 1600 U/ml rhGM-CSF und 1000 U/ml rhIL-4 ergänzt wurde. Am Tag 7 wurden die nicht adhärenten Zellen, die die unreifen dendritischen Zellen repräsentieren, geerntet und zweimal mit Kulturmedium gewaschen. Unreife dendritische Zellen wurden bei einer Dichte von 10⁶ Zellen/ml in 6-Loch-Platten in 3 ml Kulturmedium, das mit einer Mischung aus Cytokinen, die die weitere Reifung iniziieren, ergänzt wurde, angesetzt. Der Cytokincocktail enthielt rhGM-CSF (800 U/ml), rhIL-4 (500 U/ml), rhtL-1β (10 ng/ml), rhIL-6 (1000 U/ml), rhTNFα (10 ng/ml) und Prostaglandin E2 (1 µg/ml). Sämtliche Cytokine wurden erworben bei Strathmann Biotech (Hannover) außer rhGM-CSF (Sandoz, Nürnberg). Prostaglandin E2 wurde erworben bei Pharmacia & Upjohn (Erlangen). Nach 24 Stunden Inkubation wurden die reifen dendritischen Zellen zur Transfektion eingesetzt.

Alternativ wurden die dendritischen Zellen vor der Transfektion in eine CD83-positive und eine CD83-negative Fraktion aufgetrennt. Zur Erkennung des DC-Reifungsmarkers CD83 wurde der murine monoklonale Antikörper HB15e (BD Biosciences, Heidelberg) verwendet. Die CD83-positiven Zellen der DC-Kultur wurden mit antikörperbeschichteten paramagnetischen Kügelchen inkubiert, über magnetische Separation isoliert und nach DNase-vermitteltem Abdau der Kügelchen für die Transfektionen eingesetzt. Für die Zellseparation wurde das CELLection™ Pan Mouse IgG Kit (Dynal, Hamburg) nach den Vorgaben des Herstellers eingesetzt.

### Reverse-transcription PCR (RT-PCR)

Es wurde mRNA isoliert unter Verwendung des QuickPrep™ Micro mRNA Purification Kit (Amersham Pharmacia Biotech, Uppsala, Schweden). Das RNA-Pellet wurde in 5 µl Diethylpyrocarbonat-behandeltem Wasser gelöst. Die reverse Transkriptionsreaktion (RT) wurde mit 1 µl mRNA in einem Gesamtvolumen von 20 µl durchgeführt wie beschrieben in Ross (PCR Methods Applic. 4 (1995), 371-375). Die Amplifikations- und reversen Transkriptionsreaktionen wurden durchgeführt unter Verwendung eines Model 480 DNA Thermal Cycler (Perkin-Elmer, Foster City, USA). Sämtliche zur PCR eingesetzten genspezifischen Primer wurden von MWG-Biotech (Ebersberg) hergestellt. Die Menge und Qualität der erhaltenen cDNA wurde geprüft durch Amplifikation eines 366 bp-Fragments des housekeeping-Gens Hypoxanthin-Guanin-Phosphoribosyl-Transferase (HPRT) mit 1 µl der RT-Reaktion als Matrize. Es wurden die Primer HPRT-3 (5'-GCTGACCTGCTGGATTACAT-3'; SEQ ID NO: 23) und HPRT-4 (5'-CATTATAGTCAAGGGCATATCC-3'; SEQ ID NO: 24) verwendet. PCR-Denaturierung (94°C für 1 min) und Extensionsschritte (72°C für 1 min) waren konstant. Die Annealing-Temperatur wurde vermindert von 59°C auf 58°C, jeweils für zwei Zyklen, und auf 57°C für weitere 30 Zyklen (jeweils 1 min). Der finale Extensionsschritt (7 min bei 72°C) sicherte die komplette Doppelstrangpolymerisation und war Teil jeder PCR-Reaktion (siehe auch weiter unten). Zur relativen Quantifizierung des amplifizierten HPRT-cDNA-Fragments wurden Aliquots der PCR-Reaktionen einer Gelelektrophorese in 1,4%igem Agarosegel unterzogen und die Fluoreszenzintensitäten der Ethidiumbromidgefärbten PCR-Produkte wurden mit einem lmaging-System (Herolab, Wiesloch) verglichen. HPRT-standardisierte Mengen der cDNA wurden als Matrize für eine RT-PCR eingesetzt, bei der unter Verwendung der Fascin-spezifischen Primer hFascin-3 (5'-GGCAAGCCTGGCTGTAGTAG-3'; SEQ ID NO: 25) und hFascin-4 (5'-CCAGAGTGAGATGCATGTTGG-3'; SEQ ID NO: 26), ein 277 bp-Fragment des 3'-UTR der humanen Fascin-cDNA (SEQ ID NO: 27) amplifiziert wurde. PCR-Denaturierung (94°C), Annealing und Extensionsschritte (72°C) wurden jeweils für 1 min durchgeführt. Die Annealingtemperatur wurde von 66°C auf 65°C vermindert, jeweils für zwei Zyklen, und auf 64°C für die weiteren 30 Zyklen.

### Hybridisierungsproben

Eine genomische Bank wurde durchmustert mit der Sonde mFascin-ORF, die die ersten Zweidrittel (+73 bp bis +984 bp) des offenen Leserasters (ORF) der Maus-Fascin-cDNA (SEQ ID NO: 29) umfaßt. Innerhalb dieser Region zeigen Maus- und Mensch-Fascin-cDNA eine Homologie von 91% auf der Nucleotidebene (GenBank Hinterlegungsnummer U03057 und L33726). Die Sonde mFascin-ORF wurde hergestellt unter Verwendung der Primer allfas-1 (5'-GCCACCATGACCGCCAACGG-3'; SEQ ID NO: 31) und allfas-2 (5'-TGTGTCGCGGTCGATCTCCA-3'; SEQ ID NO: 32). Maus-cDNA aus dendritischen Zellen wurde zur Verwendung als Matrize Hitze-denaturiert (98°C für 5 min), und schnell auf Eis gekühlt vor dem Einsatz in der PCR. Während der PCR blieben die Denaturierungs- (95°C für 1 min) und Extensionsschritte (72°C für 2 min) konstant. Die Annealingschritte wurden für jeweils 1 min ausgeführt, wobei die Temperatur von 70°C auf 69°C und weiter auf 68°C vermindert wurde, jeweils für zwei Zyklen, und auf 67°C für weitere 30 Zyklen. Die Sonde wurde während der PCR markiert mit Digoxigenin (DIG)-11-dUTP unter Verwendung des PCR DIG Probe Synthesis Kits (Roche Molecular Biochemicals, Mannheim).
Genomische Restriktionsfragmente, die die weiter distal gelegenen Bereiche des Gens enthalten, wurden identifiziert durch die Probe hFascin-UTR, die den distalen Bereich der 3'-UTR des menschlichen Fascin-Gens (+2388 bis +2664 in SEQ ID NO: 27) abdeckt. Die cDNA von humanen dendritischen Zellen wurde verwendet als Matrize zur Amplifikation unter Verwendung der Primer hFascin-3 und hFascin-4 (siehe oben). Das PCR-Produkt wurde in *Hinc II*-restringierten pUC18 cloniert und sequenziert. Zur DIG-Markierungsreaktion wurde subcloniertes hFas-UTR als Matrize verwendet. Die Matrize wurde Hitze-denaturiert bei 98°C für 5 min und schnell abgekühlt.

### Hybridisierungsbedingungen

Alle Reagenzien für Filterhybridisierungen mit DIG-markierten Sonden und Signaldetektion wie auch Nylonmembranen wurden erworben bei Roche Molecular Biochemicals und die Experimente wurden durchgeführt nach Ross (BioTechniques 26 (1999), 150-155). Kurz zusammengefaßt wurde DIG Easy Hyb sowohl als Prähybridisierungs- als auch als Hybridisierungslösung verwendet und enthielt 50 mg/ml gescherte und hitzedenaturierte genomische DNA aus Lachsspermien. Sowohl Prähybridisierung als auch Hybridisierung wurde bei 38°C in einem Wasserbad bei leichtem Schütteln durchgeführt. Die DIG-markierte Sonde wurde detektiert durch mit alkalischer Phosphatase-gekoppeltem anti-DIG-Antikörper und CDP-Star™ als Chemilumineszenzsubstrat. Die Expositionszeit der Autoradiographie betrug typischerweise weniger als 30 min für Filter angeordneter Genbanken und weniger als 5 min für Southern blots und Koloniefilter. Zur Wiederverwertung wurden die Filter "gestrippt", wie es vom Hersteller empfohlen wird.

### Isolierung und Charakterisierung der genomischen Clone

Die angeordnete menschliche genomische Genbank, die aus PBMC eines Kaukasiers gewonnen wurde, wurde hergestellt von P. loannou, C. Chen und B.

Zhao (Roswell Park Cancer Institute, Human Genetics Department, Buffalo, NY) (loannou, Nature Genetics 6 (1994), 84-89) und wurde erhalten vom Resourcenzentrum des deutschen Humangenomprojekts am Max-Planck-Institut für Molekulare Genetik (Berlin). Die Genbank wurde gescreent durch Hybridisierung mit der Sonde mFascin-ORF. Nach DNA-Präparation (NucleoBond™ Plasmid Kit, Clontech, Palo Alto, USA) wurden positive PAC-Clone durch Southern blot verifiziert. Zur weiteren Charakterisierung wurde die PAC-Clon-DNA gespalten und die entstehenden Fragmente nach dem Zufallsprinzip in pZero2.1 (invitrogen, Groningen, Niederlande) subcloniert.

### DNA-Sequenzierung

Die Nucleotidsequenz wurde bestimmt durch Cycle-Sequencing und Analyse auf einem PE 373A-Sequencer (Perkin-Elmer). Wegen des hohen GC-Gehalts der Fascingensequenzen, vor allem im 5'-flankierenden Bereich, der vermutlich zur Ausbildung starker Sekundärstrukturen führte, ließen sich Teilbereiche nicht nach herkömmlichen Methoden sequenzieren, was allerdings durch folgende Maßnahmen überwunden werden konnte. GC-reiche DNA-Matrizen wurden vor der Sequenzierung linearisiert durch Restriktionsspaltung und Hitzedenaturierung (98°C für 5 min). Desweiteren wurde DMSO in einer Endkonzentration von 5% hinzugefügt. Die Temperatur des Denaturierungsschritts wurde reduziert auf 95°C und die Zyklenzahl auf 30 erhöht.

### Konstruktion eines Fascin-Promotor-Photinus-Luciferase-Plasmids

Ein subcloniertes genomisches *Hind III*-Restriktionsfragment von 5,5 kb Länge enthielt 3 kb 5'-flankierende Sequenz des Fascingens, Exon 1 und ein Teil des introns 1 (Figur 1). Das putative Promotorfragment, das die 5'-flankierende Genregion und einen Teil des 5'-UTR umfaßt, wurde herausgeschnitten (Restriktionsspaltung mit *Acc I*, Auffüllen der Überhänge, und weitere Restriktionsspaltung mit *Kpn I*) und ligiert in den promotorlosen Photinus-Luciferase-Expressionsvektor pGL3-Basic (Promega, Madison, USA). Die korrekte Länge und Orientierung des Promotorkonstrukts (pFascin-3,0) wurde überprüft durch die DNA-Sequenzierung von beiden Enden (RVprimer3 und GLprimer2, Promega). Zur 5'-Deletionsclonierung wurde das Konstrukt mit *Kpn I* und *Stu I* gespalten und die Deletionsclonierung durchgeführt nach den Anweisungen des Herstellers (Amersham Pharmacia Biotech). Einige Deletionscione wurden erzeugt durch Restriktionsspaltung mit *Kpn I* und einem zweiten Restriktionsenzym, das in pFascin-3,0 schneidet. Die Integrität und Länge der Plasmid-DNA jedes Deletionsclons wurde durch Gelelektrophorese überprüft. Um die absoluten Photinus-Reporterexpressionswerte zu normalisieren auf Grund von Differenzen in der Transfektionseffektivität, wurde ein CMV-Promotor-kontrollierter Renilla-Luciferase-Expressionsvektor (pRL-CMV, Promega) verwendet als Coreporter. Plasmid-DNA wurde unter Verwendung des Qiagen™ Plasmidkits isoliert (Qiagen, Hilden). Die DNA-Konzentration wurde photometrisch bestimmt. Die Integrität der Plasmid-DNA wurde durch Gelelektrophorese überprüft.

Um die Expressionsstärke des humanen Fascin-Promotors in verschiedenen Zelltypen vergleichen zu können, wurde zur Normierung der Promotor des Haushaltsgens EF1α (Wakabayashi-Ito, J. Biol. Chem. 269 (1994), 29831-29837) verwendet. Dies sollte ausschließen, daß etwaige Zelltyp-spezifische Unterschiede in der Expression des allgemein verwendeten Referenzkonstrukts unter Kontrolle des CMV-Promotors das Ergebnis verfälschen. Hierzu wurde der EF1α-Promotor aus dem Expressionskonstrukt PEF-BOS-lacz (Mizushima, Nucleic Acids Res. 18 (1990), 5322) mittels PCR amplifiziert und in pGL3-Basic kloniert. Parallel dazu wurde der CMV-Promotor des Coreporterkonstrukts pRL-CMV durch den EF1α-Promotor ersetzt (pRL-EF1α). Transfektionsexperimente in HaCaT, SHSY-5Y und DC, bei denen beide Coreporter in Parallelansätzen getestet wurden, zeigten, daß die Expressionsstärken des EF1α- und CMV-Promotors in der gleichen Größenordnung liegen.

### Transiente Zelltransfektion

Die Transfektionen wurden durchgeführt nach der biolistischen Gentransfertechnik unter Verwendung des Helium-angetriebenen PDS-1000/He-Systems (Bio-Rad, Hercules, USA) nach Kalkbrenner (Meth. Mol. Biol. 83 (1996), 203-216). Dendritische Zellen (5x10⁵) und THP-1 Zellen (10⁶) wurden cotransfiziert mit 4,5 pMol des Testkonstrukts und 0,5 pMol des pRL-CMV. Der Abstand zwischen dem Makrocarrierhalter und dem *Transwell* (24 mm Durchmesser, 3 µm Porengröße, Corning Costar, Bodenheim) betrug 6 cm. Es wurde ein Druck von 900 psi angewendet, basierend auf Optimierungsversuchen.

Die humanen Zellinien HaCaT und SHSY-5Y wurden mittels Lipofektion transfiziert. Am Vortag wurden jeweils 5x10⁵ Zellen pro Loch einer 24-Loch-Zellkultur-Platte ausgesät. Für jede Tansfektion wurden 190 nMol Testkonstrukt und 20 nMol Coreporter (pRL-CMV) eingesetzt. Durch Zugabe denaturierter Lachssperma-DNA (Roche Diagnostics, Mannheim) wurde die Gesamt-DNA-Menge auf 1 µg normiert. Als Transfektionsreagenz wurde GenePorter (PEQLAB, Erlangen) eingesetzt. Aufgrund entsprechender Optimierungsexperimente wurden für die Transfektion der HaCaT-Zellen 7 µl GenePorter / µg DNA und für SHSY-5Y 4 µl / µg DNA eingesetzt. Die Transfektion wurde entsprechend den Empfehlungen des Herstellers durchgeführt.

### Luciferase-Assays

Es wurden Zellextrakte 24 Stunden nach der Transfektion präpariert. Die Zellen wurden pelletiert und gewaschen mit 2 ml PBS. Die Zellpellets wurden in Passive Lysis Buffer (Promega) resuspendiert in einer Konzentration von 10⁶ Zellen/ 100 µl und für 15 min bei Raumtemperatur inkubiert auf einem Taumelschüttler unter leichtem Schütteln. Die Zellysate wurden bei -20°C aufbewahrt. Die Proben wurden aufgetaut und auf Eis gestellt. 10 µl des Zellysats wurden auf Photinus- und Renilla-Luciferaseaktivität hin analysiert mit dem Dual-Luciferase™ Reporterassaysystem nach Angaben des Herstellers (Promega) in einem Turner-Luminometer TD-20/20 (Turner Design, Sunnyvale, USA). Die Luciferaseaktivität wurde für eine Dauer von 10 Sekunden gemessen. Die absoluten Werte wurden normalisiert durch Dividieren der Photinus-Luciferaseaktivität durch die Renilla-Luciferaseaktivität.

### Beispiel 1: Isolierung und genomische Organisation des menschlichen Fascingens

Durch das Screening einer humanen, PBMC-abgeleiteten genomischen PAC-Bibliothek mit der Maus-Fascin-cDNA-spezifischen Sonde mFascin-ORF wurden 16 Clone identifiziert, wobei sich 8 Clone davon als positiv im Southern Blot herausstellten. Clon RPCIP704C24766Q3/4 wurde zur Charakterisierung des menschlichen Fascin-Genlokus verwendet. Die PAC-Clon-DNA wurde jeweils mit *Hind III, Pst I* und *Sac* / restringiert und die Restriktionsfragmente nach dem Zufallsprinzip in den Vektor pZero2.1 cloniert. Ein größeres Restriktionsfragment, das einen Teil des Exons 1, das komplette Intron 1 und Exons 2 bis 4 enthält, wurde durch Doppelverdau mit *Hinc II* und *Eco RI* cloniert. Clonierte Fragmente, die mit Fascin-Sonden hybridisierten, wurden weiteranalysiert und der Sequenzierung unterzogen. Figur 1 zeigt, daß das menschliche Fascingen eine genomische Region von annähernd 13 kb abdeckt und aus 5 Exons besteht. Exon 1 umfaßt die kurze 5'nicht-translatierte Region (5'-UTR) des Gens und etwa die Hälfte der translatierten Sequenz. Intron 1 hat eine Länge von etwa 8 kb. Die kurzen Exons 2 bis 4 sind innerhalb einer Region von 800 bp gruppiert und codieren ein Drittel der translatierten mRNA. Exon 5 liegt etwa 1 kb weiter stromabwärts und enthält den verbleibenden codierenden Bereich sowie die komplette 3'-UTR des Gens. Die DNA-Sequenz des Gens ist in Figur 2 zu sehen. Die in der vorläufigen DNA-Sequenzierung ermittelten Gensequenzen wiesen zunächst noch einzelne Lücken in Intron 1 sowie eine Lücke in Exon 5 auf (SEQ ID NOs: 33, 10-15, 34 und 35), die mit der Bestimmung der Gesamtsequenz des Fascingens (SEQ ID NO:72) geschlossen wurden. Die letzten fünf Basenpaare der 3'-UTR der cDNA-Sequenz sind nicht in der genomischen Sequenz enthalten. Die Exon/intron Grenzen entsprechen der GT/AG-Regel, abgesehen von einer Abweichung der 5'-Introngrenze des Introns 4 (GC anstatt GT).

### Beispiel 2: Funktionelle Analyse der 5'-flankierenden Region

Wie es typisch ist für einen eukaryontischen Genpromotor, wurde ein Konsensus-TATA-Box-Motif (TATAAAA) in der Nähe des Transkriptionsstartpunkts identifiziert.

Um die Promotoraktivität der 5'-flankierenden Region des humanen Fascingens einzuschätzen, wurde ein Promotor-Reporter-Konstrukt (pFascin-3.0, Position 1 bis 3069 in SEQ ID NO:72 bzw. SEQ ID NO: 1) konstruiert, das die Gesamtlänge der isolierten 5'-flankierenden Region enthält sowie stromabwärts angrenzend die ersten 102 bp der 5'-nicht-translatierten Sequenz des Fascingens (Position 1 des publizierten humanen cDNA-Clons als Transkriptionsstartpunkt definiert). Ausgereifte dendritische Zellen, die Fascin stark exprimieren, wurden transient transfiziert durch einen biolistischen Gentransfer dieses Konstrukts. Während die negative Kontrolle (pGL3-Basic) nur Hintergrundluciferaseaktivität zeigt, resultierte die Transfektion mit pFascin-3.0 in einer starken Reporterexpression, die im Vergleich zu pGL3-Basic um das 101fache erhöht war (Fig. 3). Um cis-agierende Elemente in dem Promotor zu identifizieren, wurden 5'-Deletionsclone von pFascin-3.0 erzeugt (Figur 4, Position 1136 bis 3069, 1451 bis 3069, 1621 bis 3069, 1830 bis 3069, 2127 bis 3069, 2410 bis 3069, 2700 bis 3069, 2859 bis 3069 und 2915 bis 3069 in SEQ ID NO:72 bzw. SEQ ID NO: 2 bis 8, 21, 22) und in transienten Transfektionen eingesetzt. Die menschliche Monocytenzellinie THP-1 wurde als Negativkontrolle verwendet, da eine RT-PCR keine endogene Fascinexpression anzeigte (Fig. 5).
Ein 5'-Deletionskonstrukt, das vom Promotor nur 53 Basenpaare einschließlich der Consensus-TATA-Box enthält (pFascin-0.05, Position 2915 bis 3069 in SEQ ID NO:72 bzw. SEQ ID NO: 22) ist nicht ausreichend für eine Luciferaseexpression (Fig. 6). Das erste Fragment, das eine basale Promotoraktivität zeigt, ist ein 211 bp langes Fragment, das 109 bp des Promotors enthält (pFascin-0.11, Position 2859 bis 3069 in SEQ ID NO:72 bzw.SEQ ID NO: 21). Die Transfektion mit Promotorkonstrukten, die in den weiter distal gelegenen Bereich der 5'-flankierenden Sequenz reichen, resultierte in einer weiteren schrittweisen Erhöhung der Luciferaseexpression. Die höchste Reporterexpression wurde für pFascin-1.6 (Position 1451 bis 3069 in SEQ ID NO:72 bzw. SEQ ID NO: 3) detektiert, das eine 3,4-fache Aktivität verglichen mit dem Minimalpromotor (pFascin-0.11) aufwies.
Terminal ausgereifte dendritische Zellen zeichnen sich gegenüber unreifen dendritischen Zellen durch die Oberflächenexpression des Markers CD83 aus. Die Zellen einer DC-Kultur wurden in eine CD83-positive und eine CD83-negative Fraktion aufgeteilt. Während in der CD83-positiven DC-Fraktion die Reportergenexpression für die längeren Promotorkonstrukte pFascin-1.6 und pFascin-3.0 den fünffachen Wert des Basalpromotores pFascin-0.11 erreicht, wird in der CD83-negativen Zellfraktion durch Verwendung von pFascin-1.6 und pFascin-3.0 das Expressions-Niveau nicht über die Basalexpression hinaus gesteigert (Figur 7). Dieser Befund belegt, daß der Fascinpromotor vorwiegend in terminal ausgereiften, CD83-positiven dendritischen Zellen aktiv ist. Dies ist für den klinischen Einsatz besonders vorteilhaft.
Überraschenderweise weist der Promotor des menschlichen Fascingens auch eine basale Aktivität in THP-1 auf. Daraus geht hervor, daß zumindest ein unspezifisches aktivierendes Transkriptionselement in nächster Nachbarschaft 5' der TATA-Box lokalisiert ist. Jedoch nimmt im Gegensatz zu den dendritischen Zellen die Promotoraktivität in den THP-1-Zellen mit zunehmender Länge der transfizierten Konstrukte ab. Bei dem Konstrukt mit der vollen Promotorlänge ist die Reportergenexpression auf ein Drittel der Aktivität, die für das Konstrukt pFascin-0.11 detektiert wurde, reduziert. Zusammenfassend läßt sich sagen, daß innerhalb der untersuchten 5'-flankierenden Region des menschlichen Fascingens transkriptionsregulierende Elemente mit Spezifität für dendritische Zellen cooperieren, um die Expression des menschlichen Fascingens zu erhöhen. Zusätzlich enthält der Fascinpromotor Elemente, die die Transkription in Fascin-negativen Zellinien wie THP-1 spezifisch reprimieren.

Auch ein zelltypübergreifender Vergleich der Fascinpromotorstärke - im Verhältnis zu einer Positivkontrolle, die zelltypunabhängig starke Reportergenexpression vermittelt - bestätigte die DC-Spezifität des Fascinpromoters. In der Fascin-negativen Keratinozytenlinie HaCaT bewegte sich die Fascinpromotor-abhängige Reportergenaktivität mit unter zehn Prozent der Positivkontrolle lediglich auf basalem Niveau (Figur 8). Selbst in der Fascin-positiven Neuroblastomlinie SHSY-5Y erreichte die vom Fascinpromotor getriebene Reportergenexpression nur ein Fünftel der Positivkontrolle. Demgegenüber wies der Facinpromotor in den stark Fascin-positiven DC die ca. anderthalbfache Stärke der Positivkontrolle auf.

## Patentansprüche

1. Regulatorische Sequenz ausgewählt aus der Gruppe bestehend aus:
(a) der regulatorischen Sequenz, die unter SEQ ID NO:72 von Position 1 bis 3069 angegeben ist;
(b) der regulatorischen Sequenz, die in der Insertion des Klons DSM13274 enthalten ist und durch Amplifikation unter Verwendung zweier Oligonucleotide erhältlich ist, die die unter SEQ ID NOS: 36 und 37 angegebenen Sequenzen besitzen;
(c) der regulatorischen Sequenz, aus SEQ ID NO:72 von Position 1136 bis 3069, 1451 bis 3069, 1621 bis 3069, 1830 bis 3069, 2127 bis 3069, 2410 bis 3069 oder 2700 bis 3069 oder ausgewählt aus der Gruppe bestehend aus: SEQ ID NOs:2 bis 8;
(d) der regulatorischen Sequenz, die in der Insertion des Klons DSM 13274 enthalten ist und durch Amplifikation unter Verwendung eines Paars von Oligonucleotiden erhältlich ist, wobei die Sequenzen der Oligonucleotide angegeben sind unter den SEQ ID-Nummern, ausgewählt aus der Gruppe von Paaren bestehend aus: 38 und 37; 39 und 37; 40 und 37; 41 und 37; 42 und 37; 43 und 37; und 44 und 37;
(e) regulatorischen Sequenzen, die zumindest ein funktionaler Teil einer unter (a) bis (d) genannten Sequenz sind und eine für dendritische Zellen spezifische Expression bewirken; und
(f) regulatorischen Sequenzen, die mit der unter (a) angegebenen regulatorischen Sequenz hybridisieren, zu der unter (a) angegebenen Sequenz eine Sequenzidentität von mindestens 50% aufweisen und eine für dendritische Zellen spezifische Expression bewirken.

2. Regulatorische Sequenz nach Anspruch 1, die kombiniert ist mit mindestens einer der Nucleotidsequenzen,
a) ausgewählt aus der Gruppe bestehend aus: den Abschnitten von SEQ ID NO:72 von Position 3911 bis 13398, 13556 bis 13637, 13760 bis 14004, 14173 bis 15414 und 16791 bis 16951 und SEQ ID NOs:9 bis 20, oder Teilen davon; oder
b) die in der Insertion des Klons DSM13274 enthalten ist und durch Amplifikation unter Verwendung eines Paars von Oligonucleotiden erhältlich ist, wobei die Sequenzen der Oligonucleotide angegeben sind unter den SEQ ID-Nummern, ausgewählt aus der Gruppe von Paaren bestehend aus: 45 und 46; 47 und 48; 49 und 50; 51 und 52; 53 und 54; 55 und 56; 57 und 58; 59 und 60; 61 und 62; 63 und 64; 65 und 66; 67 und 68; und 45 und 60.

3. Regulatorische Sequenz nach Anspruch 1 oder 2, die aus Mensch stammt.

4. Rekombinantes Nucleinsäuremolekül enthaltend die regulatorische Sequenz nach einem der Ansprüche 1 bis 3 in Kombination mit mindestens einer Nucleotidsequenz, wobei die Nucleotidsequenz aus einer anderen Spezies stammt als die regulatorische Sequenz, die Nucleotidsequenz natürlicherweise im Vergleich zur regulatorischen Sequenz an einer anderen Position innerhalb des Genoms lokalisiert ist oder die Nucleotidsequenz im Vergleich zur natürlichen Vorlage mutiert oder chemisch modifiziert ist.

5. Vektor enthaltend die regulatorische Sequenz nach einem der Ansprüche 1 bis 3 oder das rekombinante Nucleinsäuremolekül nach Anspruch 4.

6. Rekombinantes Nucleinsäuremolekül nach Anspruch 4 oder Vektor nach Anspruch 5, der zusätzlich eine zu exprimierende Nucleotidsequenz enthält, wobei die Expression der Nucleotidsequenz von der regulatorischen Sequenz kontrolliert wird.

7. Rekombinantes Nucleinsäuremolekül oder Vektor nach Anspruch 6, wobei die Nucleotidsequenz ein Antigen codiert.

8. Rekombinantes Nucleinsäuremolekül oder Vektor nach Anspruch 7, wobei das Antigen tumor- oder pathogenspezifisch ist oder an der Bildung von Creutzfeldt-Jakob-Plaques oder Alzheimer-Plaques beteiligt ist.

9. Rekombinantes Nucleinsäuremolekül oder Vektor nach Anspruch 7, wobei das Antigen ein Autoantigen oder ein Transplantationsantigen ist.

10. Rekombinantes Nucleinsäuremolekül oder Vektor nach Anspruch 7, wobei das Antigen ein Allergen ist.

11. Rekombinantes Nucleinsäuremolekül oder Vektor nach Anspruch 6, wobei die Nucleotidsequenz ein Protein codiert, das eine Immunantwort reguliert.

12. Rekombinantes Nucleinsäuremolekül oder Vektor nach Anspruch 11, wobei das Protein ein Cytokin oder ein co-stimulatorisches Molekül ist.

13. Rekombinantes Nucleinsäuremolekül oder Vektor nach Anspruch 11, wobei die Regulation eine Hemmung ist.

14. Rekombinantes Nucleinsäuremolekül oder Vektor nach Anspruch 11, 12 oder 13, wobei das Protein IL-10 oder TGF-β ist.

15. Rekombinantes Nucleinsäuremolekül oder Vektor nach Anspruch 11 oder 12, wobei die Regulation eine Steigerung der Immunantwort ist.

16. Rekombinantes Nucleinsäuremolekül oder Vektor nach Anspruch 11, 12 oder 15, wobei das Protein IL-2, IL-4, IL-12, IL-15, IL-18, IFN-gamma, IFN-alpha, DC-CK1, MDC oder GM-CSF ist.

17. Rekombinantes Nucleinsäuremolekül oder Vektor nach Anspruch 11, 12 oder 15, wobei das Protein ein Mitglied der B7-Familie, ICOS-Ligand oder CD40 ist.

18. Rekombinantes Nucleinsäuremolekül oder Vektor nach Anspruch 6, wobei die Nucleotidsequenz für ein Apoptose-induzierendes Molekül codiert.

19. Rekombinantes Nucleinsäuremolekül oder Vektor nach Anspruch 18, wobei das Apoptose-induzierende Molekül zur TNF-Superfamilie gehört.

20. Rekombinantes Nucleinsäuremolekül oder Vektor nach Anspruch 6, wobei die Nucleotidsequenz eine Antisense-Sequenz ist oder ein Ribozym exprimiert.

21. Rekombinantes Nucleinsäuremolekül oder Vektor nach Anspruch 20, wobei die Antisense-Sequenz oder das Ribozym spezifisch ist für eine mRNA, die ein Cytokin oder ein co-stimulatorisches Molekül codiert.

22. Rekombinantes Nucleinsäuremolekül oder Vektor nach Anspruch 6, wobei die Nucleotidsequenz einen Transkriptionsfaktor codiert.

23. Rekombinantes Nucleinsäuremolekül oder Vektor nach Anspruch 6, der zusätzlich eine zweite zu exprimierende Nucleotidsequenz enthält, wobei eine Nucleotidsequenz ein Antigen codiert und die zweite Nucleotidsequenz ein Protein codiert, das eine Immunantwort reguliert.

24. Vektor nach einem der Ansprüche 5 bis 23, der ein Virus ist.

25. Vektor nach einem der Ansprüche 6 bis 24, der zur Gentherapie oder DNA-Vakzinierung geeignet ist.

26. Rekombinantes Nucleinsäuremolekül oder Vektor nach Anspruch 6, wobei die Nucleotidsequenz ein Reportergen ist.

27. Verfahren zur Herstellung von genetisch modifizierten Wirtszellen, **dadurch gekennzeichnet, daß** man Wirtszellen mit einem Vektor nach einem der Ansprüche 5 bis 26 transfiziert und die transfizierten Wirtszellen in einem Kulturmedium kultiviert.

28. Wirtszelle, die genetisch modifiziert ist mit dem rekombinanten Nucleinsäuremolekül nach einem der Ansprüche 4, 6 bis 23 und 26 oder dem Vektor nach einem der Ansprüche 5 bis 26 oder erhältlich nach dem Verfahren nach Anspruch 27.

29. Wirtszelle nach Anspruch 28, die eine dendritische Zelle ist.

30. Wirtszelle nach Anspruch 28 oder 29, die aus Mensch stammt.

31. Nucleinsäurefragment mit einer Länge von mindestens 15 Nucleotiden, das spezifisch mit einem Strang einer regulatorischen Sequenz nach einem der Ansprüche 1 bis 3 unter stringenten Bedingungen hybridisiert, wobei die Sequenz des Nucleinsäurefragments zu der unter Anspruch 1(a) angegebenen Sequenz eine Sequenzidentiät von mindestens 50% aufweist.

32. Verfahren zur Antigen-spezifischen Stimulierung von T-Zellen in vitro, enthaltend die Schritte:
(a) Transfektion von dendritischen Zellen mit einem Vektor nach einem der Ansprüche 7 bis 10, allein oder in Kombination mit einem Vektor nach einem der Ansprüche 11, 12, 15, 16, 17, 20 und 22, oder mit einem Vektor nach Anspruch 23;
(b) Cokultivierung der durch Schritt (a) gewonnenen transfizierten dendritischen Zellen mit T-Zellen; und
(c) Detektion der Aktivierung der T-Zellen aus Schritt (b).

33. Verfahren zur Herstellung eines Arzneimittels umfassend die Schritte (a) bis (c) nach Anspruch 32 und zusätzlich den Schritt
(d) Formulierung eines Arzneimittels durch Versetzen der durch den Schritt (c) gewonnenen stimulierten T-Zellen mit einem pharmazeutisch verträglichen Träger.

34. Verfahren zur Herstellung von T-Zell-stimulierenden dendritischen Zellen in vitro, enthaltend die Schritte:
(a) Transfektion von dendritischen Zellen mit einem Vektor nach einem der Ansprüche 7 bis 10, allein oder in Kombination mit einem Vektor nach einem der Ansprüche 11 bis 22 oder mit einem Vektor nach Anspruch 23; und
(b) Kultivierung der transfizierten dendritischen Zellen in einem geeigneten Medium und/oder Detektion der T-Zell-stimulierenden Aktivität.

35. Verfahren zur Herstellung eines Arzneimittels umfassend die Schritte (a) und (b) nach Anspruch 34 und zusätzlich den Schritt
(c) Formulierung eines Arzneimittels durch Versetzen der durch den Schritt (b) gewonnenen T-Zell-stimulierenden dendritischen Zellen mit einem pharmazeutisch verträglichen Träger.

36. Verfahren nach einem der Ansprüche 32 bis 35, wobei die dendritischen Zellen aus Mensch stammen.

37. Arzneimittel umfassend das rekombinante Nucleinsäuremolekül nach einem der Ansprüche 6 bis 23, den Vektor nach einem der Ansprüche 6 bis 25, die Wirtszelle nach einem der Ansprüche 28 bis 30, Antigen-spezifisch stimulierte T-Zellen, erhältlich nach dem Verfahren nach Anspruch 32, oder T-Zell-stimulierende dendritische Zellen, erhältlich nach dem Verfahren nach Anspruch 34, und optional einen pharmazeutisch verträglichen Träger.

38. Arzneimittel nach Anspruch 37, das ein Impfstoff ist.

39. Verwendung des rekombinanten Nucleinsäuremoleküls nach einem der Ansprüche 6 bis 8 und 10, des Vektors nach einem der Ansprüche 6 bis 8, 10, 24 und 25, allein oder in Kombination mit einem rekombinanten Nucleinsäuremolekül oder Vektor nach einem der Ansprüche 11, 12, 14 bis 17 und 22, der Wirtszelle nach einem der Ansprüche 28 bis 30, des rekombinanten Nucleinsäuremoleküls oder Vektors nach Anspruch 23, von Antigen-spezifisch stimulierten T-Zellen, erhältlich nach dem Verfahren nach Anspruch 32, oder T-Zell-stimulierenden dendritischen Zellen, erhältlich nach dem Verfahren nach Anspruch 34, zur Herstellung eines Arzneimittels zur Impfung gegen Viren, Bakterien, Pilze, Parasiten, Tumoren, Allergene, Creutzfeldt-Jakob-Plaques, oder Alzheimer-Plaques oder zur gentherapeutischen Behandlung von Tumoren oder viralen, bakteriellen oder parasitischen Infektionen oder von Allergien.

40. Verwendung des rekombinanten Nucleinsäuremoleküls nach einem der Ansprüche 6, 7, 9 und 10 oder des Vektors nach einem der Ansprüche 6, 7, 9, 10, 24 und 25, allein oder in Kombination mit einem rekombinanten Nucleinsäuremolekül oder Vektor, nach einem der Ansprüche 11, 13, 14, 18 bis 22, des Nucleinsäuremoleküls oder Vektors nach Anspruch 23, oder der Wirtszelle nach einem der Ansprüche 28 bis 30 zur Herstellung eines Arzneimittels zur Behandlung von Autoimmunerkrankungen, Transplantatabstoßung oder Allergien.

41. Verwendung des rekombinanten Nucleinsäuremoleküls nach Anspruch 18 oder 19 oder des Vektors nach einem der Ansprüche 18, 19, 24 und 25 zur Herstellung eines Arzneimittels zur Vermeidung der Abstoßung von Transplantaten und von Autoimmunreaktionen.

42. Verwendung der regulatorischen Sequenz nach einem der Ansprüche 1 bis 3, des rekombinanten Nucleinsäuremoleküls nach einem der Ansprüche 4, 6 bis 23 oder des Vektors nach einem der Ansprüche 5 bis 25 zur Herstellung eines Arzneimittels zur Immuntherapie durch spezifische Expression von Antigenen oder immunregulatorischen Proteinen in dendritischen Zellen.

43. Verwendung der regulatorischen Sequenz nach einem der Ansprüche 1 bis 3, des rekombinanten Nucleinsäuremoleküls nach Anspruch 4, 6 oder 26 oder des Vektors nach Anspruch 5, 6 oder 26 zur Identifizierung und Isolierung von cis-Elementen aus der regulatorischen Sequenz, die eine für dendritische Zellen spezifische Expression vermitteln.

44. Verwendung der regulatorischen Sequenz nach einem der Ansprüche 1 bis 3, des rekombinanten Nucleinsäuremoleküls nach Anspruch 4, 6 oder 26 oder des Vektors nach Anspruch 5, 6 oder 26 zur Bestimmung des Reifegrads von dendritischen Zellen.

45. Verwendung der regulatorischen Sequenz nach einem der Ansprüche 1 bis 3, des rekombinanten Nucleinsäuremoleküls nach Anspruch 4, 6 oder 26 oder des Vektors nach Anspruch 5, 6 oder 26 zur Identifizierung und Isolierung von Faktoren, die eine Expression spezifisch für dendritische Zellen vermitteln.

46. Verwendung der regulatorischen Sequenz nach Anspruch 1 oder Teilen davon zur Herstellung eines Arzneimittels zur Immuntherapie durch Inhibierung der Primärstimulation von T-Zellen, wobei die regulatorische Sequenz oder Teile davon in dendritischen Zellen als Transkriptionsfaktor-Bindungsstellen dienen, die Transkriptionsfaktoren blockieren.

## Claims

1. A regulatory sequence selected from the group consisting of:
(a) the regulatory sequence indicated under SEQ ID NO:72 from position 1 to 3069;
(b) the regulatory sequence contained in the insertion of clone DSM13274 and obtainable by amplification using two oligonucleotides which have the sequences indicated under SEQ ID NOS: 36 and 37;
(c) the regulatory sequence of SEQ ID NO:72 from position 1136 to 3069, 1451 to 3069, 1621 to 3069, 1830 to 3069, 2127 to 3069, 2410 to 3069 or 2700 to 3069 or selected from the group consisting of: SEQ ID NOS: 2 to 8;
(d) the regulatory sequence contained in the insertion of clone DSM 13274 and obtainable by amplification using a pair of oligonucleotides, the sequences of the oligonucleotides being indicated under the SEQ ID Numbers selected from the group of pairs consisting of: 38 and 37; 39 and 37; 40 and 37; 41 and 37; 42 and 37; 43 and 37; and 44 and 37;
(e) regulatory sequences being at least a functional part of a sequence mentioned under (a) to (d) and causing a dendritic cell-specific expression; and
(f) regulatory sequences hybridizing with the regulatory sequence indicated under (a), having a sequence identity of at least 50% to the sequence defined under (a) and causing a dendritic cell-specific expression.

2. The regulatory sequence according to claim 1, which is combined with at least one of the nucleotide sequences,
a) selected from the group consisting of: the sections of SEQ ID NO:72 from position 3911 to 13398, 13556 to 13637, 13760 to 14004, 14173 to 15414 and 16791 to 16951 and SEQ ID NOS:9 to 20, or parts thereof; or
b) which is contained in the insertion of clone DSM13274 and is obtainable by amplification using a pair of oligonucleotides, the sequences of the oligonucleotides being disclosed under the SEQ ID Numbers selected from the group of pairs consisting of: 45 and 46; 47 and 48; 49 and 50; 51 and 52; 53 and 54; 55 and 56; 57 and 58; 59 and 60; 61 and 62; 63 and 64; 65 and 66; 67 and 68; and 45 and 60.

3. The regulatory sequence according to claim 1 to 2 which is of human origin.

4. A recombinant nucleic acid molecule containing the regulatory sequence according to any one of claims 1 to 3 in combination with at least one nucleotide sequence, the nucleotide sequence being derived from another species than the regulatory sequence, the nucleotide sequence, in comparison with the regulatory sequence, being naturally located at another position within the genome, or the nucleotide sequence being mutated or chemically modified compared to its natural model.

5. A vector containing the regulatory sequence according to any one of claims 1 to 3 or the recombinant nucleic acid molecule according to claim 4.

6. The recombinant nucleic acid molecule according to claim 4 or the vector according to claim 5, which additionally contains a nucleotide sequence to be expressed, wherein the expression of the nucleotide sequence is controlled by the regulatory sequence.

7. The recombinant nucleic acid molecule or the vector according to claim 6, wherein the nucleotide sequence encodes an antigen.

8. The recombinant nucleic acid molecule or the vector according to claim 7, wherein the antigen is tumor-specific or pathogen-specific or participates in the formation of Creutzfeldt-Jakob plaques or Alzheimer plaques.

9. The recombinant nucleic acid molecule or the vector according to claim 7, wherein the antigen is an autoantigen or a transplantation antigen.

10. The recombinant nucleic acid molecule or the vector according to claim 7, wherein the antigen is an allergen.

11. The recombinant nucleic acid molecule or the vector according to claim 6, wherein the nucleotide sequence encodes a protein which regulates an immune response.

12. The recombinant nucleic acid molecule or the vector according to claim 11, wherein the protein is a cytokine or a co-stimulatory molecule.

13. The recombinant nucleic acid molecule or the vector according to claim 11, wherein the regulation is an inhibition.

14. The recombinant nucleic acid molecule or the vector according to claim 11, 12 or 13, wherein the protein is IL-10 or TGF-β.

15. The recombinant nucleic acid molecule or the vector according to claim 11 or 12, wherein the regulation is an increase of the immune response.

16. The recombinant nucleic acid molecule or the vector according to claim 11, 12 or 15, wherein the protein is IL-2, IL-4, IL-12, IL-15, IL-18, IFN-gamma, IFN-alpha, DC-CK1, MDC or GM-CSF.

17. The recombinant nucleic acid molecule or the vector according to claim 11, 12 or 15, wherein the protein is a member of the B7 family, ICOS ligand or CD40.

18. The recombinant nucleic acid molecule or the vector according to claim 6, wherein the nucleotide sequence encodes an apoptosis-inducing molecule.

19. The recombinant nucleic acid molecule or the vector according to claim 18, wherein the apoptosis-inducing molecule belongs to the TNF superfamily.

20. The recombinant nucleic acid molecule or the vector according to claim 6, wherein the nucleotide sequence is an antisense sequence or expresses a ribozyme.

21. The recombinant nucleic acid molecule or the vector according to claim 20, wherein the antisense sequence or the ribozyme is specific for an mRNA encoding a cytokine or a co-stimulatory molecule.

22. The recombinant nucleic acid molecule or the vector according to claim 6, wherein the nucleotide sequence encodes a transcription factor.

23. The recombinant nucleic acid molecule or the vector according to claim 6 which additionally contains a second nucleotide sequence to be expressed, wherein one nucleotide sequence encodes an antigen and the second nucleotide sequence encodes a protein which regulates an immune response.

24. The vector according to any one of claims 5 to 23, which is a virus.

25. The vector according to any one of claims 6 to 24, which is suitable for gene therapy or DNA vaccination.

26. The recombinant nucleic acid molecule or the vector according to claim 6, wherein the nucleotide sequence is a reporter gene.

27. A method for preparing genetically modified host cells, **characterised in that** the host cells are transfected with a vector according to any one of claims 5 to 26 and the transfected host cells are cultured in a culture medium.

28. A host cell which is genetically modified with the recombinant nucleic acid molecule according to any one of claims 4, 6 to 23 and 26 or the vector according to any one of claims 5 to 26, or obtainable by the method according to claim 27.

29. The host cell according to claim 28, which is a dendritic cell.

30. The host cell according to claim 28 or 29 which is of human origin.

31. A nucleic acid fragment having a length of at least 15 nucleotides which specifically hybridises with a strand of a regulatory sequence according to any one of claims 1 to 3 under stringent conditions, the sequence of the nucleic acid fragment having a sequence identity of at least 50% to the sequence indicated under claim 1(a).

32. A method for the antigen-specific stimulation of T cells in vitro, comprising the steps of:
(a) transfecting dendritic cells with a vector according to any one of claims 7 to 10, alone or in combination with a vector according to any one of claims 11, 12, 15, 16, 17, 20 and 22, or with a vector according to claim 23;
(b) co-culturing the transfected dendritic cells obtained in step (a) with T-cells; and
(c) detecting the activation of the T cells of step (b).

33. A method for preparing a pharmaceutical composition, which comprises steps (a) to (c) according to claim 32 and additional the step of
(d) formulating a pharmaceutical composition by mixing the stimulated T-cells obtained in step (c) with a pharmaceutically acceptable carrier.

34. A method for the in vitro preparation of T cell-stimulating dendritic cells, comprising the steps of:
(a) transfecting dendritic cells with a vector according to any one of claims 7 to 10, alone or in combination with a vector according to any one of claims 11 or 22, or with a vector according to claim 23; and
(b) culturing the transfected dendritic cells in a suitable medium and/or detecting the T-cell stimulating activity.

35. A method for preparing a pharmaceutical composition, which comprises steps (a) and (b) according to claim 34 and additionally the step of
(c) formulating a pharmaceutical composition by mixing the T cell-stimulating dendritic cells obtained in step (b) with a pharmaceutical acceptable carrier.

36. The method according to any one of claims 32 to 35, wherein the dendritic cells are of human origin.

37. A pharmaceutical composition comprising the recombinant nucleic acid molecule according to any one of claims 6 to 23, the vector according to any one of claims 6 to 25, the host cell according to any one of claims 28 to 30, antigen-specific stimulated T-cells obtainable by the method according to claim 32, or T cell-stimulating dendritic cells obtainable by the method according to claim 34, and optionally a pharmaceutically acceptable carrier.

38. The pharmaceutical composition according to claim 37, which is a vaccine.

39. Use of the recombinant nucleic acid molecule according to any one of claims 6 to 8 and 10, the vector according to any one of claims 6 to 8, 10, 24 and 25, alone or in combination with a recombinant nucleic acid molecule or vector according to any one of claims 11, 12, 14 to 17 and 22, the host cell according to any one of claims 28 to 30, the recombinant nucleic acid molecule or vector according to claim 23, antigen-specific stimulated T-cells obtainable by the method according to claim 32, or T cell-stimulating dendritic cells obtainable by the method according to claim 34, for preparing a pharmaceutical composition for the vaccination against viruses, bacteria, fungi, parasites, tumors, allergens, Creutzfeldt-Jakob plaques or Alzheimer plaques or for the gene therapy treatment of tumors or viral, bacterial or parasitic infections or allergies.

40. Use of the recombinant nucleic acid molecule according to any one of claims 6, 7, 9 and 10 or the vector according to any one of claims 6, 7, 9, 10, 24 and 25, alone or in combination with a recombinant nucleic acid molecule or a vector according to any one of claims 11, 13, 14, 18 to 22, of the nucleic acid molecule or the vector according to claim 23, or of the host cell according to any one of claims 28 to 30, for preparing a pharmaceutical composition for the treatment of autoimmune diseases, graft rejection or allergies.

41. Use of the recombinant nucleic acid molecule according to claim 18 or 19 or the vector according to any one of claims 18, 19, 24 and 25 for preparing a pharmaceutical composition for preventing graft rejection and autoimmune reactions.

42. Use of the regulatory sequence according to any one of claims 1 to 3, the recombinant nucleic acid molecule according to any one of claims 4, 6 to 23 or the vector according to any one of claims 5 to 25 for preparing a pharmaceutical composition for immune therapy by specific expression of antigens or immunoregulatory proteins in dendritic cells.

43. Use of the regulatory sequence according to any one of claims 1 to 3, the recombinant nucleic acid molecule according to claim 4, 6 or 26 or the vector according to claim 5, 6 or 26 for identifying and isolating cis-elements from the regulatory sequence, which mediate an expression specific to dendritic cells.

44. Use of the regulatory sequence according to any one of claims 1 to 3, the recombinant nucleic acid molecule according to claim 4, 6 or 26 or the vector according to claim 5, 6 or 26 for determining the degree of maturation of dendritic cells.

45. Use of the regulatory sequence according to any one of claims 1 to 3, the recombinant nucleic acid molecule according to claim 4, 6 or 26 or the vector according to claim 5, 6 or 26 for identifying and isolating factors which mediate an expression specific to dendritic cells.

46. Use of the regulatory sequence according to claim 1 or parts thereof for preparing a pharmaceutical composition for immune therapy by inhibition of the primary stimulation of T-cells, the regulatory sequence or parts thereof serving in dendritic cells as transcription factor binding sites which block transcription factors.

## Revendications

1. Séquence régulatrice sélectionnée dans le groupe se composant de :
(a) la séquence régulatrice qui est enregistrée sous SEQ ID No : 72 de position 1 à 3069 ;
(b) la séquence régulatrice qui est contenue dans l'insertion du clone DSM13274 et qui est disponible par l'amplification en utilisant deux oligonucléotides lesquels possèdent les séquences enregistrées sous SEQ ID Nos : 36 et 37 ;
(c) la séquence régulatrice se composant de SEQ ID No : 72 positions 1136 à 3069, 1451 à 3069, 1621 à 3069, 1830 à 3069, 2127 à 3069, 2410 à 3069 ou 2700 à 3069 ou sélectionnée dans le groupe se composant de : SEQ ID N° : 2 à 8 ;
(d) la séquence régulatrice qui est contenue dans l'insertion du clone DSM13274 et qui est disponible par l'amplification d'une paire d'oligonucléotides, les séquences d'oligonucléotides étant enregistrées sous les numéros de SEQ ID sélectionnées dans le groupe des paires se composant de : 38 et 37 ; 39 et 37 ; 40 et 37 ; 41 et 37 ; 42 et 37 ; 43 et 37 ; et 44 et 37 ;
(e) des séquences régulatrices qui représentent au moins une partie fonctionnelle d'une séquence citée sous (a) à (d) et qui exercent une expression spécifique aux cellules dendritiques ; et
(f) des séquences régulatrices, qui s'hybrident avec la séquence régulatrice mentionnée sous (a), qui présentent une identité de séquence par rapport à la séquence mentionnée sous (a) d'au moins 50 % et qui exercent une expression spécifique aux cellules dendritiques.

2. Séquence régulatrice selon la revendication 1, qui est combinée avec au moins l'une des séquences nucléotidiques,
(a) sélectionnée dans le groupe se composant : des segments de SEQ ID No : 72 de position 3911 à 13398, 13556 à 13637, 13760 à 14004, 14173 à 15414 et 16791 à 16951 et SEQ ID Nos : 9 à 20, ou de partie de ceux-ci ; ou
(b) qui est contenue dans l'insertion du clone DSM13274 et qui est disponible par l'amplification d'une paire d'oligonucléotides, les séquences d'oligonucléotides étant enregistrées sous les numéros de SEQ ID sélectionnées dans le groupe des paires se composant de : 45 et 46 ; 47 et 48 ; 49 et 50 ; 51 et 52 ; 53 et 54 ; 55 et 56 ; 57 et 58 ; 59 et 60 ; 61 et 62 ; 63 et 64 ; 65 et 66 ; 67 et 68 ; et 45 et 60.

3. Séquence régulatrice selon la revendication 1 ou la revendication 2, qui provient de l'homme.

4. Molécule d'acide nucléique recombinante contenant la séquence régulatrice selon l'une quelconque des revendications 1 à 3 en combinaison avec au moins une séquence nucléotidique, la séquence nucléotidique provenant d'une autre espèce que la séquence régulatrice, la séquence nucléotidique étant localisée naturellement, en comparaison des séquences régulatrices, au niveau d'une autre position à l'intérieur du génome ou la séquence nucléotidique étant mutée en comparaison du modèle naturel ou modifiée chimiquement.

5. Vecteur contenant la séquence régulatrice selon l'une quelconque des revendications 1 à 3 ou molécule d'acide nucléique recombinant selon la revendication 4.

6. Molécule d'acide nucléique recombinante selon la revendication 4 ou vecteur selon la revendication 5, qui contient en plus une séquence nucléotidique à exprimer l'expression de la séquence nucléotidique étant contrôlée par la séquence régulatrice.

7. Molécule d'acide nucléique recombinante ou vecteur selon la revendication 6, dans lesquels la séquence nucléotidique code pour un antigène.

8. Molécule d'acide nucléique recombinante ou vecteur selon la revendication 7, dans lesquels l'antigène est spécifique d'une tumeur ou d'un pathogène et prend part à la formation des plaques de Creutzfeldt-Jakob ou d'Alzheimer.

9. Molécule d'acide nucléique recombinante ou vecteur selon la revendication 7, dans lesquels l'antigène est un auto-antigène ou un antigène de transplantation.

10. Molécule d'acide nucléique recombinante ou vecteur selon la revendication 7, dans lesquels l'antigène est un allergène.

11. Molécule d'acide nucléique recombinante ou vecteur selon la revendication 6, dans lesquels la séquence nucléotidique code pour une protèine, qui régule une réponse immunitaire.

12. Molécule d'acide nucléique recombinante ou vecteur selon la revendication 11, dans lesquels la protéine est une cytokine ou une molécule co-simulatrice.

13. Molécule d'acide nucléique recombinante ou vecteur selon la revendication 11, dans lesquels la régulation est une inhibition.

14. Molécule d'acide nucléique recombinante ou vecteur selon la revendication 11, 12 ou 13, dans lesquels la protéine est IL-10 ou TGF-β.

15. Molécule d'acide nucléique recombinante ou vecteur selon les revendications 11 et 12, dans lesquels la régulation est une augmentation de la réponse immunitaire.

16. Molécule d'acide nucléique recombinante ou vecteur selon la revendication 11, 12 ou 15, dans lesquels la protéine est IL-2, IL-4, IL-12, IL-15, IL-18, IFN-gamma, IFN-alpha, DC-CK1, MDC ou GM-CSF.

17. Molécule d'acide nucléique recombinante ou vecteur selon la revendication 11, 12 ou 15, dans lesquels la protéine est un membre de la famille B7, un ligand ICOS ou CD40.

18. Molécule d'acide nucléique recombinante ou vecteur selon la revendication 6, dans lesquels la séquence nucléotidique code pour une molécule induisant l'apoptose.

19. Molécule d'acide nucléique recombinante ou vecteur selon la revendication 18, dans lesquels la molécule induisant l'apoptose appartient à la superfamille TNF.

20. Molécule d'acide nucléique recombinante ou vecteur selon la revendication 6, dans lesquels la séquence nucléotidique est une séquence antisens ou exprime un ribozyme.

21. Molécule d'acide nucléique recombinante ou vecteur selon la revendication 20, dans lesquels la séquence antisens ou le ribozyme est spécifique à l'ARNm, qui code pour une cytokine ou une molécule co-simulatrice.

22. Molécule d'acide nucléique recombinante ou vecteur selon la revendication 6, dans lesquels la séquence code pour un facteur de transcription.

23. Molécule d'acide nucléique recombinante ou vecteur selon la revendication 6, qui contient en plus une deuxième séquence nucléotidique à exprimer, moyennant quoi la séquence nucléotidique code pour un antigène et la deuxième séquence nucléotidique code pour une protéine qui régule la réponse immunitaire.

24. Vecteur selon l'une quelconque des revendications 5 à 23, qui est un virus.

25. Vecteur selon l'une quelconque des revendications 6 à 24, qui convient pour une thérapie génique ou pour une vaccination ADN.

26. Molécule d'acide nucléique recombinante ou vecteur selon la revendication 6, dans lesquels la séquence nucléotidique est un gène rapporteur.

27. Procédé pour la préparation de cellules hôtes génétiquement modifiées, **caractérisé en ce que** l'on transfecte les cellules hôtes avec un vecteur selon une quelconque des revendications 5 à 26 et on cultive les cellules hôtes transfectées dans un milieu de culture.

28. Cellule hôte qui est modifiée génétiquement avec la molécule d'acide nucléique recombinante selon l'une quelconque des revendications 4, 6 à 23 et 26 ou le vecteur selon l'une quelconque des revendications 5 à 26 ou obtenue selon le procédé de la revendication 27.

29. Cellule hôte selon la revendication 28, qui est une cellule dendritique.

30. Cellule hôte selon la revendication 28 ou 29, qui provient de l'homme.

31. Fragment d'acide nucléique présentant une longueur d'au moins 15 nucléotides, qui s'hybride de manière spécifique avec un brin d'une séquence régulatrice selon l'une quelconque des revendications 1 à 3 dans des conditions stringentes, dans lequel la séquence de fragment d'acide nucléique présente pour une séquence mentionnée sous la revendication 1(a) une identité de séquence d'au moins 50 %.

32. Procédé pour la stimulation spécifique à l'antigène des cellules T in vitro, contenant les étapes consistant à :
(a) transfecter les cellules dendritiques avec un vecteur selon l'une quelconque des revendications 7 à 10, seul ou en combinaison avec un vecteur selon l'une quelconque des revendications 11, 12, 15, 16, 17, 20 et 22, ou avec un vecteur selon la revendication 23 ;
(b) co-cultiver les cellules dendritiques transfectées obtenues dans l'étape (a) avec des cellules T ; et
(c) détecter l'activité des cellules T à partir de l'étape (b).

33. Procédé pour la fabrication d'un médicament comprenant les étapes (a) à (c) selon la revendication 32 et en plus l'étape (d) consistant à formuler un médicament en mélangeant des cellules T stimulées obtenues dans l'étape (c) avec un support pharmaceutiquement compatible.

34. Procédé pour la préparation des cellules dendritiques stimulant les cellules T in vitro, comprenant les étapes consistant à :
(a) transfecter les cellules dendritiques avec un vecteur selon l'une quelconque des revendications 7 à 10, seul ou en combinaison avec un vecteur selon l'une quelconque des revendications 11 à 22 ou avec un vecteur selon la revendication 23 ; et
(b) cultiver les cellules dendritiques transfectées dans un milieu approprié et/ou détecter l'activité stimulant les cellules T.

35. Procédé pour la fabrication d'un médicament comprenant les étapes (a) et (b) selon la revendication 34 et en plus l'étape (c) consistant à formuler un médicament en mélangeant des cellules dendritiques stimulant les cellules T obtenues dans l'étape (b) avec un vecteur pharmaceutiquement compatible.

36. Procédé selon l'une quelconque des revendications 32 à 35, dans lequel les cellules dendritiques proviennent de l'homme.

37. Médicament comprenant la molécule d'acide nucléique recombinante selon l'une quelconque des revendications 6 à 23, le vecteur selon l'une quelconque des revendications 6 à 25, les cellules hôtes selon l'une quelconque des revendications 28 à 30, les cellules T stimulées de façon spécifique à l'antigène, obtenues d'après le procédé selon la revendication 32, ou des cellules dendritiques stimulant la cellule T, obtenues d'après le procédé selon la revendication 34, et éventuellement un vecteur pharmaceutiquement compatible.

38. Médicament selon la revendication 37, qui est un vaccin.

39. Utilisation de la molécule d'acide nucléique recombinante d'après l'une quelconque des revendications 6 à 8 et 10, du vecteur selon l'une quelconque des revendications 6 à 8, 10, 24 et 25, seul ou en combinaison avec une molécule d'acide nucléique recombinante ou un vecteur selon l'une quelconque des revendications 11, 12, 14 à 17 et 22, de la cellule hôte selon l'une quelconque des revendications 28 à 30, de la molécule d'acide nucléique recombinant ou du vecteur selon la revendication 23, des cellules T stimulant spécifiquement l'antigène, obtenues d'après le procédé selon la revendication 32, ou des cellules dendritiques stimulant les cellules T, obtenues d'après le procédé selon la revendication 34, pour la fabrication d'un médicament pour la vaccination contre des virus, des bactéries, des champignons, des parasites, des tumeurs, des allergènes, des plaques de Creutzfeldt-Jakob, ou des plaques d'Alzheimer ou pour le traitement par thérapie génique des tumeurs ou des infections virales, bactériennes ou parasitaires ou des allergies.

40. Utilisation de la molécule d'acide nucléique recombinante selon l'une quelconque des revendications 6, 7, 9 et 10 ou du vecteur selon l'une quelconque des revendications 6, 7, 9, 10, 24 et 25, seul ou en combinaison avec une molécule d'acide nucléique recombinante ou un vecteur, selon l'une quelconque des revendications 11, 13, 14, 18 à 22, d'une molécule d'acide nucléique ou vecteur selon la revendication 23, ou des cellules hôtes selon l'une quelconque des revendications 28 à 30 pour la fabrication d'un médicament destiné à traiter des maladies auto-immunes, des rejets des greffons ou des allergies.

41. Utilisation de la molécule d'acide nucléique recombinante selon la revendication 18 ou 19 ou du vecteur selon l'une quelconque des revendications 18, 19, 24 et 25 pour la fabrication d'un médicament destiné à éviter le rejet des greffons et les réactions auto-immunes.

42. Utilisation de la séquence régulatrice selon l'une quelconque des revendications 1 à 3, de la molécule d'acide nucléique recombinante selon l'une quelconque des revendications 4, 6 à 23 ou du vecteur selon l'une des revendications 5 à 25 pour la fabrication d'un médicament destiné à l'immunothérapie par l'expression spécifique des antigènes ou des protéines immunorégulatrices dans des cellules dendritiques.

43. Utilisation de la séquence régulatrice selon l'une quelconque des revendications 1 à 3, de la molécule d'acide nucléique recombinante selon la revendication 4, 6 ou 26 ou du vecteur selon la revendication 5, 6 ou 26 pour l'identification et l'isolement des éléments cis à partir de la séquence régulatrice, qui déterminent une expression spécifique pour les cellules dendritiques.

44. Utilisation de la séquence régulatrice selon l'une quelconque des revendications 1 à 3, de la molécule d'acide nucléique recombinant selon la revendication 4, 6 ou 26 ou du vecteur selon la revendication 5, 6 ou 26 pour déterminer le degré de maturité des cellules dendritiques.

45. Utilisation de la séquence régulatrice selon l'une quelconque des revendications 1 à 3, de la molécule d'acide nucléique recombinante selon la revendication 4, 6 ou 26 ou du vecteur selon la revendication 5, 6 ou 26 pour l'identification et l'isolement de facteurs, qui déterminent une expression spécifique pour les cellules dendritiques.

46. Utilisation de la séquence régulatrice selon la revendication 1 ou des parties de celle-ci pour la fabrication d'un médicament destiné à l'immunothérapie par inhibition de la stimulation, primaire des cellules T, la séquence régulatrice ou partie de celle-ci servant dans des cellules dendritiques de positions de liaison du facteur de transcription, bloquant les facteurs de transcription.


### SEQUENCE LISTING

<110> RESKE-KUNZ, A.B.
   ROSS, Xiaolan
   ROSS, Ralf
   BROS, Matthias
<120> Regulatorische Sequenz zur spezifischen Expression in dendritischen Zellen und deren Anwendung
<130> D 2299 PCT
<140>
   <141>
<150> P 100 01 169.1-41
   <151> 2000-01-13
<150> P 100 10 188.7-
   <151> 2000-03-02
<160> 72
<170> PatentIn Ver. 2.1
<210> 1
   <211> 3069
   <212> DNA
   <213> Homo sapiens
<400> 1
   aagcttctag aaggggtata ttttgccccc aggccccaaa tcctggtctt tggactacag 60
   tcaagaccta gcacagggct caggcctaaa aaaaagctgt tgaggagggt gtgaggctgc 120
   aacgttgcgg tgagaagggg gtccccaggg agggcgcagc aggaagcccc agggaagtgc 180
   ctgggagagg gaggttgtgc agccagaagg agcagcccgc agcctttggt tgttgactcc 240
   tgctttgtaa gtggcagtct ggttgggtag gacagggtcc gagtcctcac tcagggaact 300
   gagtccaggg tgagctgagc agcccttcct ggtggcctca ctttcccctg cagagcctgc 360
   tgcatggtgt ccagtggcca gcctgggagg agctcaggac tggcctcacc tcgtgccacg 420
   ccctcgtacc aaggtggctc agatggcacc tgggttcccg aagggcccag gaacacagcg 480
   tccatgtccc catccttccc cggagggact tggggtgggg cctgcaggaa ggatcatgtg 540
   acttgttcaa gggcagcttg tcctgccccg gacacagagg tgcccatcgt aagcgagatg 600
   caggcaaggt gaggacaggg catggtgccg agcaggaatg attttcgaaa atgctactct 660
   gtggtcgggc acggtggctc actcctgtaa tcccagtact ttgggaggcc caggcgggca 720
   gatcatgagg tcaggagttc aagaccagcc tggccaacat ggtgaaaccc cttctctact 780
   gaaaatacaa aaaactaccc gggcgtggtg gtggatgcct gtaatcccag ctactcagga 840
   ggctgaggca ggagaatcgc ttgaaccctg gaggtgaagg ttgcaatgag ccgagattgc 900
   accactgcac tccgacctgg gcgacagaga aagactccgt ctgaaaaaca aacaaacaaa 960
   accccgagat tctcttttcc cccgtccgga gctctatggc catctggagc tcgttctgtc 1020
   cacaaggaca catttcctgg cagcagctgt ggaccagggc tcactcactc acatctgtac 1080
   ccaatctaga gcaggacaaa cacctatcac ctgcactggc aatggacaga ggacagtggc 1140
   agccccatca ccagaggcat tcaagccaag gcattttcta tcgtctattt atctatctat 1200
   ctatctatct atctatctat ctatctatct atctatctag atgagatctt gctatgttgc 1260
   ccaggttgaa ctcctggcct caagcgatcc tcctgcttca gcctcccaaa gtgttgggat 1320
   ttcaggtgtg aaccactgta cctggctgga gtgcagtggc actgtcatag ctcactgcag 1380
   cctccacctc ctgggctcaa ggaatcctct ttcctcagcc tcctgagtag ctgggaccac 1440
   aggcatgcac catcacaccc agctaaacct tgattttttc catagtatat ggctcagggc 1500
   agagcaaaga agcacaaaaa catgttggct tgcggttgag ggctgatggg agggggttct 1560
   ggccaaagcc caaaattaac agccacaacg tcagtgtctg tgggaggggt tgccaggggc 1620
   gtgcgggttc tggggctcaa ggccctgccg gtaaacccat ttgaagcagg atggcaagaa 1680
   ggtgacacca tcttcccccg cgcactgaaa gcccctggct gggattgcct ggggcagaca 1740
   caggctcgga ccagccccag caatcccagt ttatcagcga gccggctgag ggcccggagt 1800
   tatctcagtg cccggcctga gaccttgtgg gcagcctgtg gtcatgctgg cattccaggg 1860
   gccttttggc atgtggggaa tgtccaggaa aagcctcagc cttcggtgag gcgcagaaaa 1920
   gggaagtgtc cctagagggg gtgggtgagg gcgtgggagg tggtgtctgc agggaatgtc 1980
   ccctttgggg gaggaggatg gagggttggg attctgagga tggggggggg ggctgtagcc 2040
   agcaccatgt ccctcctgtg tgaccagctc agagtcccat gaaattgggg cttgggaggg 2100
   gaagggacac tggcctggga accagagacc tgggctggtc tggctcacag ttgctggacc 2160
   tctgtgatcc gtgtcaaaaa acgagaacac caattcctgt cctgcccact caccaccagg 2220
   tgggacctga accctggcat cgccagcatt gggaatgtcg gccactgact caaccactct 2280
   cccggagacc tatttgggcc acccgaggcg ggtgcctggg ccacacggag gggtcctggt 2340
   ggtcttcagg gcagcggctg tggggctgaa gcctcaagga accacatctc tgcataggag 2400
   ggccaggctg cagggcctcg ggagacaacc tagttgggcg tgttggggtc tgtgggtccc 2460
   aggtcctggc ctcaccgggt ccccaccgcg ctgtcagctc ccagcctctt tccctcgtct 2520
   gcctctgggc ttctgtaagg ctatgtgctc caaggccacc tcctccaggc agccctcaga 2580
   cccccacctt cgcccagtac cgatctgcac cgtggtctct gaagtctcca tcgtgacctc 2640
   aaacctcgct cgtccttgct cctagcgagg cttggggtcg gggtgtccga ggtgggggac 2700
   atccgggggg gttaggtggc tggcgcgggg agccggggtt gtgaggggtg atgtcctcag 2760
   gcggcggcgc tgcggggtgc ggcgaggaca ccggtggggt gagagcaccg gcggggcagc 2820
   agcgggggcc gcagcgccgg gtccctcggc ccggggcccc tcccgcgcgg agccaggggc 2880
   gggacagggg ggcgtggcct ggtggcgctg acgtcacctc gcctataaaa tgtccggggc 2940
   gccgctagct gggctttgtg gagcgctgcg gagggtgcgt gcgggccgcg gcagccgaac 3000
   aaaggagcag gggcgccgcc gcagggaccc gccacccacc tcccggggcc gcgcagcggc 3060
   ctctcgtct                                                        3069
<210> 2
   <211> 1934
   <212> DNA
   <213> Homo sapiens
<400> 2
   gtggcagccc catcaccaga ggcattcaag ccaaggcatt ttctatcgtc tatttatcta 60
   tctatctatc tatctatcta tctatctatc tatctatcta tctagatgag atcttgctat 120
   gttgcccagg ttgaactcct ggcctcaagc gatcctcctg cttcagcctc ccaaagtgtt 180
   gggatttcag gtgtgaacca ctgtacctgg ctggagtgca gtggcactgt catagctcac 240
   tgcagcctcc acctcctggg ctcaaggaat cctctttcct cagcctcctg agtagctggg 300
   accacaggca tgcaccatca cacccagcta aaccttgatt ttttccatag tatatggctc 360
   agggcagagc aaagaagcac aaaaacatgt tggcttgcgg ttgagggctg atgggagggg 420
   gttctggcca aagcccaaaa ttaacagcca caacgtcagt gtctgtggga ggggttgcca 480
   ggggcgtgcg ggttctgggg ctcaaggccc tgccggtaaa cccatttgaa gcaggatggc 540
   aagaaggtga caccatcttc ccccgcgcac tgaaagcccc tggctgggat tgcctggggc 600
   agacacaggc tcggaccagc cccagcaatc ccagtttatc agcgagccgg ctgagggccc 660
   ggagttatct cagtgcccgg cctgagacct tgtgggcagc ctgtggtcat gctggcattc 720
   caggggcctt ttggcatgtg gggaatgtcc aggaaaagcc tcagccttcg gtgaggcgca 780
   gaaaagggaa gtgtccctag agggggtggg tgagggcgtg ggaggtggtg tctgcaggga 840
   atgtcccctt tgggggagga ggatggaggg ttgggattct gaggatgggg gggggggctg 900
   tagccagcac catgtccctc ctgtgtgacc agctcagagt cccatgaaat tggggcttgg 960
   gaggggaagg gacactggcc tgggaaccag agacctgggc tggtctggct cacagttgct 1020
   ggacctctgt gatccgtgtc aaaaaacgag aacaccaatt cctgtcctgc ccactcacca 1080
   ccaggtggga cctgaaccct ggcatcgcca gcattgggaa tgtcggccac tgactcaacc 1140
   actctcccgg agacctattt gggccacccg aggcgggtgc ctgggccaca cggaggggtc 1200
   ctggtggtct tcagggcagc ggctgtgggg ctgaagcctc aaggaaccac atctctgcat 1260
   aggagggcca ggctgcaggg cctcgggaga caacctagtt gggcgtgttg gggtctgtgg 1320
   gtcccaggtc ctggcctcac cgggtcccca ccgcgctgtc agctcccagc ctctttccct 1380
   cgtctgcctc tgggcttctg taaggctatg tgctccaagg ccacctcctc caggcagccc 1440
   tcagaccccc accttcgccc agtaccgatc tgcaccgtgg tctctgaagt ctccatcgtg 1500
   acctcaaacc tcgctcgtcc ttgctcctag cgaggcttgg ggtcggggtg tccgaggtgg 1560
   gggacatccg ggggggttag gtggctggcg cggggagccg gggttgtgag gggtgatgtc 1620
   ctcaggcggc ggcgctgcgg ggtgcggcga ggacaccggt ggggtgagag caccggcggg 1680
   gcagcagcgg gggccgcagc gccgggtccc tcggcccggg gcccctcccg cgcggagcca 1740
   ggggcgggac aggggggcgt ggcctggtgg cgctgacgtc acctcgccta taaaatgtcc 1800
   ggggcgccgc tagctgggct ttgtggagcg ctgcggaggg tgcgtgcggg ccgcggcagc 1860
   cgaacaaagg agcaggggcg ccgccgcagg gacccgccac ccacctcccg gggccgcgca 1920
   gcggcctctc gtct 1934
<210> 3
   <211> 1619
   <212> DNA
   <213> Homo sapiens
<400> 3
   catcacaccc agctaaacct tgattttttc catagtatat ggctcagggc agagcaaaga 60
   agcacaaaaa catgttggct tgcggttgag ggctgatggg agggggttct ggccaaagcc 120
   caaaattaac agccacaacg tcagtgtctg tgggaggggt tgccaggggc gtgcgggttc 180
   tggggctcaa ggccctgccg gtaaacccat ttgaagcagg atggcaagaa ggtgacacca 240
   tcttcccccg cgcactgaaa gcccctggct gggattgcct ggggcagaca caggctcgga 300
   ccagccccag caatcccagt ttatcagcga gccggctgag ggcccggagt tatctcagtg 360
   cccggcctga gaccttgtgg gcagcctgtg gtcatgctgg cattccaggg gccttttggc 420
   atgtggggaa tgtccaggaa aagcctcagc cttcggtgag gcgcagaaaa gggaagtgtc 480
   cctagagggg gtgggtgagg gcgtgggagg tggtgtctgc agggaatgtc ccctttgggg 540
   gaggaggatg gagggttggg attctgagga tggggggggg ggctgtagcc agcaccatgt 600
   ccctcctgtg tgaccagctc agagtcccat gaaattgggg cttgggaggg gaagggacac 660
   tggcctggga accagagacc tgggctggtc tggctcacag ttgctggacc tctgtgatcc 720
   gtgtcaaaaa acgagaacac caattcctgt cctgcccact caccaccagg tgggacctga 780
   accctggcat cgccagcatt gggaatgtcg gccactgact caaccactct cccggagacc 840
   tatttgggcc acccgaggcg ggtgcctggg ccacacggag gggtcctggt ggtcttcagg 900
   gcagcggctg tggggctgaa gcctcaagga accacatctc tgcataggag ggccaggctg 960
   cagggcctcg ggagacaacc tagttgggcg tgttggggtc tgtgggtccc aggtcctggc 1020
   ctcaccgggt ccccaccgcg ctgtcagctc ccagcctctt tccctcgtct gcctctgggc 1080
   ttctgtaagg ctatgtgctc caaggccacc tcctccaggc agccctcaga cccccacctt 1140
   cgcccagtac cgatctgcac cgtggtctct gaagtctcca tcgtgacctc aaacctcgct 1200
   cgtccttgct cctagcgagg cttggggtcg gggtgtccga ggtgggggac atccgggggg 1260
   gttaggtggc tggcgcgggg agccggggtt gtgaggggtg atgtcctcag gcggcggcgc 1320
   tgcggggtgc ggcgaggaca ccggtggggt gagagcaccg gcggggcagc agcgggggcc 1380
   gcagcgccgg gtccctcggc ccggggcccc tcccgcgcgg agccaggggc gggacagggg 1440
   ggcgtggcct ggtggcgctg acgtcacctc gcctataaaa tgtccggggc gccgctagct 1500
   gggctttgtg gagcgctgcg gagggtgcgt gcgggccgcg gcagccgaac aaaggagcag 1560
   gggcgccgcc gcagggaccc gccacccacc tcccggggcc gcgcagcggc ctctcgtct  1619
<210> 4
   <211> 1449
   <212> DNA
   <213> Homo sapiens
<400> 4
   gtgcgggttc tggggctcaa ggccctgccg gtaaacccat ttgaagcagg atggcaagaa 60
   ggtgacacca tcttcccccg cgcactgaaa gcccctggct gggattgcct ggggcagaca 120
   caggctcgga ccagccccag caatcccagt ttatcagcga gccggctgag ggcccggagt 180
   tatctcagtg cccggcctga gaccttgtgg gcagcctgtg gtcatgctgg cattccaggg 240
   gccttttggc atgtggggaa tgtccaggaa aagcctcagc cttcggtgag gcgcagaaaa 300
   gggaagtgtc cctagagggg gtgggtgagg gcgtgggagg tggtgtctgc agggaatgtc 360
   ccctttgggg gaggaggatg gagggttggg attctgagga tggggggggg ggctgtagcc 420
   agcaccatgt ccctcctgtg tgaccagctc agagtcccat gaaattgggg cttgggaggg 480
   gaagggacac tggcctggga accagagacc tgggctggtc tggctcacag ttgctggacc 540
   tctgtgatcc gtgtcaaaaa acgagaacac caattcctgt cctgcccact caccaccagg 600
   tgggacctga accctggcat cgccagcatt gggaatgtcg gccactgact caaccactct 660
   cccggagacc tatttgggcc acccgaggcg ggtgcctggg ccacacggag gggtcctggt 720
   ggtcttcagg gcagcggctg tggggctgaa gcctcaagga accacatctc tgcataggag 780
   ggccaggctg cagggcctcg ggagacaacc tagttgggcg tgttggggtc tgtgggtccc 840
   aggtcctggc ctcaccgggt ccccaccgcg ctgtcagctc ccagcctctt tccctcgtct 900
   gcctctgggc ttctgtaagg ctatgtgctc caaggccacc tcctccaggc agccctcaga 960
   cccccacctt cgcccagtac cgatctgcac cgtggtctct gaagtctcca tcgtgacctc 1020
   aaacctcgct cgtccttgct cctagcgagg cttggggtcg gggtgtccga ggtgggggac 1080
   atccgggggg gttaggtggc tggcgcgggg agccggggtt gtgaggggtg atgtcctcag 1140
   gcggcggcgc tgcggggtgc ggcgaggaca ccggtggggt gagagcaccg gcggggcagc 1200
   agcgggggcc gcagcgccgg gtccctcggc ccgggccccc tcccgcgcgg agccaggggc 1260
   gggacagggg ggcgtggcct ggtggcgctg acgtcacctc gcctataaaa tgtccggggc 1320
   gccgctagct gggctttgtg gagcgctgcg gagggtgcgt gcgggccgcg gcagccgaac 1380
   aaaggagcag gggcgccgcc gcagggaccc gccacccacc tcccggggcc gcgcagcggc 1440
   ctctcgtct                                                       1449
<210> 5
   <211> 1240
   <212> DNA
   <213> Homo sapiens
<400> 5
   ggcagcctgt ggtcatgctg gcattccagg ggccttttgg catgtgggga atgtccagga 60
   aaagcctcag ccttcggtga ggcgcagaaa agggaagtgt ccctagaggg ggtgggtgag 120
   ggcgtgggag gtggtgtctg cagggaatgt cccctttggg ggaggaggat ggagggttgg 180
   gattctgagg atgggggggg gggctgtagc cagcaccatg tccctcctgt gtgaccagct 240
   cagagtccca tgaaattggg gcttgggagg ggaagggaca ctggcctggg aaccagagac 300
   ctgggctggt ctggctcaca gttgctggac ctctgtgatc cgtgtcaaaa aacgagaaca 360
   ccaattcctg tcctgcccac tcaccaccag gtgggacctg aaccctggca tcgccagcat 420
   tgggaatgtc ggccactgac tcaaccactc tcccggagac ctatttgggc cacccgaggc 480
   gggtgcctgg gccacacgga ggggtcctgg tggtcttcag ggcagcggct gtggggctga 540
   agcctcaagg aaccacatct ctgcatagga gggccaggct gcagggcctc gggagacaac 600
   ctagttgggc gtgttggggt ctgtgggtcc caggtcctgg cctcaccggg tccccaccgc 660
   gctgtcagct cccagcctct ttccctcgtc tgcctctggg cttctgtaag gctatgtgct 720
   ccaaggccac ctcctccagg cagccctcag acccccacct tcgcccagta ccgatctgca 780
   ccgtggtctc tgaagtctcc atcgtgacct caaacctcgc tcgtccttgc tcctagcgag 840
   gcttggggtc ggggtgtccg aggtggggga catccggggg ggttaggtgg ctggcgcggg 900
   gagccggggt tgtgaggggt gatgtcctca ggcggcggcg ctgcggggtg cggcgaggac 960
   accggtgggg tgagagcacc ggcggggcag cagcgggggc cgcagcgccg ggtccctcgg 1020
   cccgggcccc ctcccgcgcg gagccagggg cgggacaggg gggcgtggcc tggtggcgct 1080
   gacgtcacct cgcctataaa atgtccgggg cgccgctagc tgggctttgt ggagcgctgc 1140
   ggagggtgcg tgcgggccgc ggcagccgaa caaaggagca ggggcgccgc cgcagggacc 1200
   cgccacccac ctcccggggc cgcgcagcgg cctctcgtct                      1240
<210> 6
   <211> 943
   <212> DNA
   <213> Homo sapiens
<400> 6
   gacctgggct ggtctggctc acagttgctg gacctctgtg atccgtgtca aaaaacgaga 60
   acaccaattc ctgtcctgcc cactcaccac caggtgggac ctgaaccctg gcatcgccag 120
   cattgggaat gtcggccact gactcaacca ctctcccgga gacctatttg ggccacccga 180
   ggcgggtgcc tgggccacac ggaggggtcc tggtggtctt cagggcagcg gctgtggggc 240
   tgaagcctca aggaaccaca tctctgcata ggagggccag gctgcagggc ctcgggagac 300
   aacctagttg ggcgtgttgg ggtctgtggg tcccaggtcc tggcctcacc gggtccccac 360
   cgcgctgtca gctcccagcc tctttccctc gtctgcctct gggcttctgt aaggctatgt 420
   gctccaaggc cacctcctcc aggcagccct cagaccccca ccttcgccca gtaccgatct 480
   gcaccgtggt ctctgaagtc tccatcgtga cctcaaacct cgctcgtcct tgctcctagc 540
   gaggcttggg gtcggggtgt ccgaggtggg ggacatccgg gggggttagg tggctggcgc 600
   ggggagccgg ggttgtgagg ggtgatgtcc tcaggcggcg gcgctgcggg gtgcggcgag 660
   gacaccggtg gggtgagagc accggcgggg cagcagcggg ggccgcagcg ccgggtccct 720
   cggcccgggg cccctcccgc gcggagccag gggcgggaca ggggggcgtg gcctggtggc 780
   gctgacgtca cctcgcctat aaaatgtccg gggcgccgct agctgggctt tgtggagcgc 840
   tgcggagggt gcgtgcgggc cgcggcagcc gaacaaagga gcaggggcgc cgccgcaggg 900
   acccgccacc cacctcccgg ggccgcgcag cggcctctcg tct                  943
<210> 7
   <211> 660
   <212> DNA
   <213> Homo sapiens
<400> 7
   gcagggcctc gggagacaac ctagttgggc gtgttggggt ctgtgggtcc caggtcctgg 60
   cctcaccggg tccccaccgc gctgtcagct cccagcctct ttccctcgtc tgcctctggg 120
   cttctgtaag gctatgtgct ccaaggccac ctcctccagg cagccctcag acccccacct 180
   tcgcccagta ccgatctgca ccgtggtctc tgaagtctcc atcgtgacct caaacctcgc 240
   tcgtccttgc tcctagcgag gcttggggtc ggggtgtccg aggtggggga catccggggg 300
   ggttaggtgg ctggcgcggg gagccggggt tgtgaggggt gatgtcctca ggcggcggcg 360
   ctgcggggtg cggcgaggac accggtgggg tgagagcacc ggcggggcag cagcgggggc 420
   cgcagcgccg ggtccctcgg cccggggccc ctcccgcgcg gagccagggg cgggacaggg 480
   gggcgtggcc tggtggcgct gacgtcacct cgcctataaa atgtccgggg cgccgctagc 540
   tgggctttgt ggagcgctgc ggagggtgcg tgcgggccgc ggcagccgaa caaaggagca 600
   ggggcgccgc cgcagggacc cgccacccac ctcccggggc cgcgcagcgg cctctcgtct 660
<210> 8
   <211> 370
   <212> DNA
   <213> Homo sapiens
<400> 8
   catccggggg ggttaggtgg ctggcgcggg gagccggggt tgtgaggggt gatgtcctca 60
   ggcggcggcg ctgcggggtg cggcgaggac accggtgggg tgagagcacc ggcggggcag 120
   cagcgggggc cgcagcgccg ggtccctcgg cccggggccc ctcccgcgcg gagccagggg 180
   cgggacaggg gggcgtggcc tggtggcgct gacgtcacct cgcctataaa atgtccgggg 240
   cgccgctagc tgggctttgt ggagcgctgc ggagggtgcg tgcgggccgc ggcagccgaa 300
   caaaggagca ggggcgccgc cgcagggacc cgccacccac ctcccggggc cgcgcagcgg 360
   cctctcgtct                                                        370
<210> 9
   <211> 191
   <212> DNA
   <213> Homo sapiens
<400> 9
   gtgagtgggg acgctgcccc cgcctctcct ggtccgtgca caaagcgcac cccacccgcg 60
   cccctccagc ctcccgccct ttctcgctcg cggcgccgct gcggtccgga gcactgccca 120
   ttgcgccccc gctaggcacg cgggctaccc cgcctggagg gggcgaggag tggggctttg 180
   cccatccgcg g                                                      191
<210> 10
   <211> 1896
   <212> DNA
   <213> Homo sapiens
<400> 10
   gtgtcagccc ttccccccag cccctcctcc ggcgtctgcc ccgcgctcga ggccgcggcc 60
   tttgtgagca ggggggcggg tcgcctcgac tgggtcctcc ctcgccccct ttgtcctgct 120
   ccatctctgc agatgggaaa accagatgcg gcggggcggg ggaggggatc ggcttttgcg 180
   gttcacccct gcagaggagc cccccgcgcc gcccccgggc ccagggctcg ggtcccgagg 240
   ttccccagga ggcggtctcc ctcctcgtcg ccgcggcccg ggaacggcgt ggcgcggatg 300
   gcggccctcc aggcaccccg cccttcgccc gccgcgcgcg tctccaggcc ggtgcgctga 360
   gctccgctcc gcgggcgacc gagggcggct tcagcgcgag ccgggagacc ccaggccagc 420
   tccctcggga agcccctacc ctctggtgaa cccatcccct agggcgctcg ccgggaacaa 480
   ttgactgcaa ctcgatccgt cccctccggg gcctcctgca ggcacggcta tttgcgaccg 540
   gcctgtgcgc cactctttcc cttctttctc agattctccc cgaggggctt tctcttcctt 600
   ttggcgtcct cttccactcc tggcggagaa gctgttcctt gcttgcagac agaggccccg 660
   ctggagggag gggcgtgggg ggatcttttt ccctggatag gaagtcggac cccaggcctt 720
   ggagatggct ggacgggagc ccagtttgtg tcctcatccc tcctcttgga aggagccctc 780
   ctgacggccc ccctcttggc tgtgggctct gcaggagggg gaaagacccc ccatggcagt 840
   gggggtgagg gtggaggcct gggtggggta atggccattt tgtgcctgaa gtttgctacc 900
   ttaagtccca gagaggtgaa gctgcttgtc atgggtcaca cagcaagtga ccacaaggaa 960
   gcagcatgca gaagtgggtg catttggctc cagaaacccc gtttcctgtt tcccacagcg 1020
   catggcgggc agagctcctc acctgatcag caggcattga gccctactaa ggggacctgc 1080
   tgagccatga gtgaaccagt ggggttcacg ttctcttggg gacattacga agtgtcaagg 1140
   aaaaggcagc gggtacaggt tattagatgg ctgagtcctt tctgagcagg acatttgagc 1200
   tggcctgaag gatgaataga aggtggcagg tatggggggg gtgctgtgtg gaccagcgtt 1260
   gtaggctgtg ggtgtctcct gtgcaaaggt cttatggcag gaaggtacag agaaagccca 1320
   gtgtggctgg gaggaagctt gtgaggtctg ggccacggga ccttgggtga gagcttagtg 1380
   cagggctatg gtccttgcct tggggttgca gccacagcct cccgtgggga agaagtcgca 1440
   gaagtaggtg gccttggccc tgggggccag gaagttgcag agagcctgag ggtgtttttg 1500
   gcgagcctgg gcagataact cccctctcct gagaagcttg ctgggggcgt ctggtgtgtg 1560
   attcaggctg atagggtggg aggaaccaga aattgcagcc aggagaggtg gtggtgatgg 1620
   ctcagcttgg agttgaaagg gatgtgggac ggcgcggggg tgggggtact tgggggccga 1680
   ggttggggct cctctggcct cagatcttgg ggtccttatc tcgtttctat gtcagccaac 1740
   tcccgggggg cttttgggaa ctggagtctg caggggtggg cgtggggggc gtgtctgcag 1800
   aggccatttt ggacgggcct gcgtggcaag ggggaggcgt ctgcttccga cacacatgct 1860
   ggggaacgcc agccaggaag gggaggatcc gagctc                           1896
<210> 11
   <211> 356
   <212> DNA
   <213> Homo sapiens
<400> 11
   gggcccggtg ggggcagggt gctccccttt gaaaaacgct gcgccctgct cttcagcctc 60
   agtttcccca tctgtaaagt gtgcattcca ggcctccttg ggctggccca gagctgccct 120
   gggcaggggc tttcagccct tcacaccaga ggctgggtca gctgagtgag tgctcctttg 180
   tcctcccgcc atgccccagg ctcctccctg ctccccagga tccacaccta ccctggcccc 240
   gaccctgggc catgcctcac cacacttcct gtcttctctc acatctctca catctgggag 300
   tcctctcccg ccagcctgtg cttgcatctg gggatccatg ccagggatag gacctg     356
<210> 12
   <211> 550
   <212> DNA
   <213> Homo sapiens
<400> 12
   ctgcagtgag ccgagatcgt gccactgcac tccagcctgg gcaacagaac gagaccctgt 60
   cttaaaaaaa ttttttttgg cctggtgcgg tggctcacgc ctgtaatgcc agcactttgg 120
   gagtccaagg tgggtggatc acctggaggt cgggagtttg agaccagtct gaccaacatg 180
   gagaaacccc atctctacta aaaacacaaa aattagccgg gcgtggtggc gcatgcctgt 240
   aatcccagct actctggagg ctgaggcagg agaatccctt gaacccggga ggcggaagtt 300
   gcagtgagcc gagatcgcac cattgcactc cagcctgggc aacaagagtg aaactccatc 360
   tcaaaaaaca aaacaaaaca aaaaaaacaa ccaaaacaaa caaacaaaaa actgtaatta 420
   aaataattaa aagaaaaaag ttaaaaaaaa aaaaaagaga ggatggctca aaggctggaa 480
   acagggaagg ccactgtgag gggaggacag gcagggctag acctctctgg aaggaggagg 540
   gagaggtacc                                                        550
<210> 13
   <211> 1426
   <212> DNA
   <213> Homo sapiens
<400> 13
   gggactgagt cgtggtacag atggcggcag ttgtgtggcc ctggggccca ggaatggctg 60
   ggaagctccc tttcttctct ggcttggggg atgaggttag tgtaaatttc atggagttgc 120
   caggaggatt aaggccgacg tgcattcaca cgtggccccg tggctgccgc cagttaagcc 180
   ctgcgcagcc attggaagtc tcattgagaa ggagcctccc ggcttcctgc atttgttcca 240
   ggcgggctgg taagcgccct gcttatttgc aaggctgtgt gaggacgagc cctgtaaacc 300
   ctaggacgag gctgtgctaa tgtggtttct gttccactgt ctccccttct ccttttgccc 360
   ctctttctgg ggaacctgga gcccctgatg ccctctgttt tttcctgctg ctggggtgga 420
   cctagaacct ctccctccat cctttctgct gctggggtgg gcctgcagaa gactgtcctc 480
   ttacctgagt ccctaacttc cctccgatca gcgggggctt cagcggagcc ccagcgcatt 540
   ggacaggtgg tgctctgtgg ccggcacagg ccagggccca gggtgcggcc cctgcctgta 600
   ggtagacatg caggcccttc tgagcagaat gagggggcgg tgagggcagc tgtgcgggtg 660
   tggctggcct gacggctccc tgcagccctg cggcttctgt gcagtggggg tggggcgggc 720
   cagacctttg cagggcctct tggctggtgg agggctgagt gagcagaggc cccagccctc 780
   gtgttccctg gggtgtggcc ttaggatggt cccggcaccc tggggaccca gcccctgctc 840
   accttatcct cctcccgtgc ttggctgggg tgtggtggct gagccagccc acccagtgcg 900
   gtggatgctc gtcggagggc agagggtggg ctaccggctt aaggggcccc agggaacctg 960
   gggggtggag cccaaggggt tccaagaagg ggcggggcca ggaacccata gacaagaggg 1020
   tggggcaggg aaagggcgtg gcaagagggc cacccacctc cggtccagag agccagccag 1080
   acccttactt tctcttgctg tgtgacctta ggcaaggaca tgccaccacc ggccttagtc 1140
   tccctctttg tgaagtagga tgaagatccc cacctgtgga gcagatgtgg ggctgcatgg 1200
   ttgggtcagg atggaacagg atggggaggc cgggcgtggt ggcttacccc tgtaatccca 1260
   gcactttggg aggcaaaggt gggaggactg cttgagtcct ggagtttggg caacatagcg 1320
   agaccacccc catctctaca aaataatgtt aaagttagcc aggtgtagtg gtgagtgcct 1380
   gtggtcccac ctactgggga gactgaggca agaggatcct ttgagc                1426
<210> 14
   <211> 477
   <212> DNA
   <213> Homo sapiens
<400> 14
   ctgcagcctc cgcctcctgg gttcaagcaa ttctcctgcg tcagcctccc gagcagctgg 60
   gattacaggc gcccggcacc acccccagct aatttttttt tttttgagac agagtctcgc 120
   attgtcgccc aggctggagt gcagtggcgc gatcttggct cactgcaagc tccacctccc 180
   gggttcacgc cattctcctg cctcagcctc ccgagtagca gggactacag gcgcccgcca 240
   ccgtgcccgg ctaatttttt ttttttgtat tattaataga gacgggtttc accgtgttag 300
   gatgatctcg atctcctgac ctcgtgatcc gcctgcctcg gcctcccaaa gtgctgggat 360
   tacaggcatg agccaccttg cctggtcctt ttttttgtat ttttagtaga gatgaggttt 420
   caccatgttg gccaggctgg tctcaaactc ctgacctcat gatccgcccg cctcggc    477
<210> 15
   <211> 473
   <212> DNA
   <213> Homo sapiens
<400> 15
   ctgcagaaca cccctgtgtt ggcaaataca tctctttctt tttgattcag ggtcttgctc 60
   tgttgcccag gttggagtgc agtggcacaa tcatagctca ctgtagcctc gacttcctgg 120
   gctcaagtga tcctcccacc tcagcctccc aaatagctga gactacaggc acacaccacc 180
   acatcaagtt aatttttttt tctatttggt acagacaggg tctggctatg ttgcctagga 240
   tggtcttgaa ctcccggcct cacatgatcc tcctaactca gcctcccaaa gtgctgaggt 300
   tacagacaca agccaccatg tgcccagcca gtgaacccac aactttctag ctgttttttt 360
   cttatacata agaagcatgc gggttgccac ggcggctcac gcctgtaatc ccagcacttt 420
   gggaagctga ggcgggtgga tcacctgagg tcatgagttc aagaccagcc ttg        473
<210> 16
   <211> 311
   <212> DNA
   <213> Homo sapiens
<400> 16
   caatcctcct gccttgggcc tcccaaagtg ttgcgatcac aggcatgagc ccctgtccct 60
   ggcccctatt atttctctaa ctggggaaag tcatgtggga acagatgtag cttgccttgg 120
   cctctgaccg gccctgcctg cgttcctggg tgctctctgc tgcttctcat gtgtgccact 180
   gtggggactc ggccgcccac cccaccccgt ggtgttacct tgcgtgtgta gttctgtgag 240
   ctcagggcta tggtctgcca gaactagggg gcgtggggcc ccagtaccag cccaaggcct 300
   cctctctgca g                                                      311
<210> 17
   <211> 82
   <212> DNA
   <213> Homo sapiens
<400> 17
   gtgagtgcct cgctcccacc tgtcaccgcc cccaccacct tgcctgggct accccgcctg 60
   accctgtccc gccatccccc ag                                          82
<210> 18
   <211> 245
   <212> DNA
   <213> Homo sapiens
<400> 18
   gtaacactaa agccccagtt ccctggagcc gtcctggagt cctggagggt ctggccatgc 60
   cgtggtcact tggtagcccc agccaaggcc tgctctgtgc tgggcatccc cccggactgg 120
   ccccgcactg tcctaccctg gggactgctg tgtgacccca gctcctggcc ctccctctct 180
   ggtcacccca gcctccaccc cactccctgc caggaggctc actgactccc ctctttctgg 240
   gacag                                                             245
<210> 19
   <211> 1242
   <212> DNA
   <213> Homo sapiens
<400> 19
   gcaggttctc ctgtgggcag ctgctgggca gggaacccct cggtcggggc tggggtcagt 60
   gctgcgggga gcgccctctg catccacact ggaccctggc ttggctcagg gccattccag 120
   gccctaaagg gacaggtgtc tgatggccac cagggggctc tgggatgcaa gcagcccctt 180
   tccctcttgt ctgtgtggtt ggggggactt accttgccca cctgacagag aggtgtgtgg 240
   aggggagagc agggagggaa ggagaccagg aaagggaggg aggagagcag gggaggggag 300
   agccgggaag agaggagagc aggggtgggg aggtttctgg aaagggtgtg caaggggagg 360
   acgcgcctcg gttatgggac tggagcccct tcccaggagg acccccaaca atccagaggt 420
   gcctgttagg attcagaaca tggttttttt gtttgttttt ttgagactca ctccctcacc 480
   ctggctggac ttgcagtggc gttatctcgg ctcactgcaa cctctgcctc ctgggttcaa 540
   gtgattctcc tgcctcagcc tcccaagtag ctgggattac aggtgtgcac caccacgcct 600
   ggctaatttt tatattttta aaatttatta tttatttatt ttgagaccca gtctggagtg 660
   cagtggcgtt atctcggctc tctgcaacct ctgcctcctg ggttcaagcg attctcctgc 720
   ctcagcctcc cgagtagctg ggactatgtg tgggagccac catgcctggc taattttttt 780
   gtatttttca tagagacggg tttcaccatg ttgtccaggc tggtcttgaa ttcgtggcct 840
   caagtgatcc gcccacctca gcctcccaca gtgctgggtt tataggtgtg agccaccaca 900
   cccggctaat tgttttgtat ttttagtaga gacggagctt cactatgttg gcaaggctgg 960
   ctcgaactcc tgacctcaag tgatccgccc acctcagcct cccaaagtgc tgggattaca 1020
   ggcgtgagcc actgcggccg agcagaacac gttctaggac ccttgttcat gtgtccatca 1080
   tggacaggag gacgtgcggg ccatagggac cctggctcat tccggagccg ggactggagg 1140
   gtggggcgtc acccttggga acacccgtgc ccaccctccg ctgcccaggg taggggtggg 1200
   gagccaggct ttgggcccca cttgataaag tcccctcccc ag                   1242
<210> 20
   <211> 161
   <212> DNA
   <213> Homo sapiens
<400> 20
   agccccaggc cggcctgtgt gtgtcttggg gctgaggtgg gtgggggggc tgaggtgggt 60
   gggagggctg gcgggacagg taggcgccct ggctccccag ctcagtgctg ggagtgtgca 120
   gtgggaggga ggccgtggct ccagtgggtg ctccggagct c                    161
<210> 21
   <211> 211
   <212> DNA
   <213> Homo sapiens
<400> 21
   cctcccgcgc ggagccaggg gcgggacagg ggggcgtggc ctggtggcgc tgacgtcacc 60
   tcgcctataa aatgtccggg gcgccgctag ctgggctttg tggagcgctg cggagggtgc 120
   gtgcgggccg cggcagccga acaaaggagc aggggcgccg ccgcagggac ccgccaccca 180
   cctcccgggg ccgcgcagcg gcctctcgtc t                               211
<210> 22
   <211> 155
   <212> DNA
   <213> Homo sapiens
<400> 22
   cacctcgcct ataaaatgtc cggggcgccg ctagctgggc tttgtggagc gctgcggagg 60
   gtgcgtgcgg gccgcggcag ccgaacaaag gagcaggggc gccgccgcag ggacccgcca 120
   cccacctccc ggggccgcgc agcggcctct cgtct 155
<210> 23
   <211> 20
   <212> DNA
   <213> Artificial Sequence
<220>
   <223> Description of Artificial Sequence: artificial sequence
<400> 23
   gctgacctgc tggattacat 20
<210> 24
   <211> 22
   <212> DNA
   <213> Artificial Sequence
<220>
   <223> Description of Artificial Sequence: artificial sequence
<400> 24
   cattatagtc aagggcatat cc 22
<210> 25
   <211> 20
   <212> DNA
   <213> Artificial Sequence
<220>
   <223> Description of Artificial Sequence: artificial sequence
<400> 25
   ggcaagcctg gctgtagtag 20
<210> 26
   <211> 21
   <212> DNA
   <213> Artificial Sequence
<220>
   <223> Description of Artificial Sequence: artificial sequence
<400> 26
   ccagagtgag atgcatgttg g 21
<210> 27
   <211> 2772
   <212> DNA
   <213> Homo sapiens
<220>
   <221> CDS
   <222> (112)..(1590)
<400> 27
   gcggagggtg cgtgcgggcc gcggcagccg aacaaaggag caggggcgcc gccgcaggga 60
   cccgccaccc acctcccggg gccgcgcagc ggcctctcgt ctactgccac c atg acc  117
                                                            Met Thr
                                                             1
   gcc aac ggc aca gcc gag gcg gtg cag atc cag ttc ggc ctc atc aac   165
   Ala Asn Gly Thr Ala Glu Ala Val Gln Ile Gln Phe Gly Leu Ile Asn
           5                   10                  15
   tgc ggc aac aag tac ctg acg gcc gag gcg ttc ggg ttc aag gtg aac   213
   Cys Gly Asn Lys Tyr Leu Thr Ala Glu Ala Phe Gly Phe Lys Val Asn
       20                  25                  30
   gcg tcc gcc agc agc ctg aag aag aag cag atc tgg acg ctg gag cag   261
   Ala Ser Ala Ser Ser Leu Lys Lys Lys Gln Ile Trp Thr Leu Glu Gln
   35                  40                  45                  50
   ccc cct gac gag gcg ggc agc gcg gcc gtg tgc ctg cgc agc cac ctg   309
   Pro Pro Asp Glu Ala Gly Ser Ala Ala Val Cys Leu Arg Ser His Leu
                   55                  60                  65
   ggc cgc tac ctg gcg gcg gac aag gac ggc aac gtg acc tgc gag cgc   357
   Gly Arg Tyr Leu Ala Ala Asp Lys Asp Gly Asn Val Thr Cys Glu Arg
               70                  75                  80
   gag gtg ccc ggt ccc gac tgc cgt ttc ctc atc gtg gcg cac gac gac   405
   Glu Val Pro Gly Pro Asp Cys Arg Phe Leu Ile Val Ala His Asp Asp
           85                  90                  95
   ggt cgc tgg tcg ctg cag tcc gag gcg cac cgg cgc tac ttc ggc ggc   453
   Gly Arg Trp Ser Leu Gln Ser Glu Ala His Arg Arg Tyr Phe Gly Gly
       100                 105                 110
   acc gag gac cgc ctg tcc tgc ttc gcg cag acg gtg tcc ccc gcc gag   501
   Thr Glu Asp Arg Leu Ser Cys Phe Ala Gln Thr Val Ser Pro Ala Glu
   115                 120                 125                 130
   aag tgg agc gtg cac atc gcc atg cac cct cag gtc aac atc tac agt   549
   Lys Trp Ser Val His Ile Ala Met His Pro Gln Val Asn Ile Tyr Ser
                   135                 140                 145
   gtc acc cgt aag cgc tac gcg cac ctg agc gcg cgg ccg gcc gac gag    597
   Val Thr Arg Lys Arg Tyr Ala His Leu Ser Ala Arg Pro Ala Asp Glu
               150                 155                 160
   atc gcc gtg gac cgc gac gtg ccc tgg ggc gtc gac tcg ctc atc acc    645
   Ile Ala Val Asp Arg Asp Val Pro Trp Gly Val Asp Ser Leu Ile Thr
           165                 170                 175
   ctc gcc ttc cag gac cag cgc tac agc gtg cag acc gcc gac cac cgc    693
   Leu Ala Phe Gln Asp Gln Arg Tyr Ser Val Gln Thr Ala Asp His Arg
       180                 185                 190
   ttc ctg cgc cac gac ggg cgc ctg gtg gcg cgc ccc gag ccg gcc act    741
   Phe Leu Arg His Asp Gly Arg Leu Val Ala Arg Pro Glu Pro Ala Thr
   195                 200                 205                 210
   ggc tac acg ctg gag ttc cgc tcc ggc aag gtg gcc ttc cgc gac tgc    789
   Gly Tyr Thr Leu Glu Phe Arg Ser Gly Lys Val Ala Phe Arg Asp Cys
                   215                 220                 225
   gag ggc cgt tac ctg gcg ccg tcg ggg ccc agc ggc acg ctc aag gcg    837
   Glu Gly Arg Tyr Leu Ala Pro Ser Gly Pro Ser Gly Thr Leu Lys Ala
               230                 235                 240
   ggc aag gcc acc aag gtg ggc aag gac gag ctc ttt gct ctg gag cag    885
   Gly Lys Ala Thr Lys Val Gly Lys Asp Glu Leu Phe Ala Leu Glu Gln
           245                 250                 255
   agc tgc gcc cag gtc gtg ctg cag gcg gcc aac gag agg aac gtg tcc    933
   Ser Cys Ala Gln Val Val Leu Gln Ala Ala Asn Glu Arg Asn Val Ser
       260                 265                 270
   acg cgc cag ggt atg gac ctg tct gcc aat cag gac gag gag acc gac    981
   Thr Arg Gln Gly Met Asp Leu Ser Ala Asn Gln Asp Glu Glu Thr Asp
   275                 280                 285                 290
   cag gag acc ttc cag ctg gag atc gac cgc gac acc aaa aag tgt gcc    1029
   Gln Glu Thr Phe Gln Leu Glu Ile Asp Arg Asp Thr Lys Lys Cys Ala
                   295                 300                 305
   ttc cgt acc cac acg ggc aag tac tgg acg ctg acg gcc acc ggg ggc    1077
   Phe Arg Thr His Thr Gly Lys Tyr Trp Thr Leu Thr Ala Thr Gly Gly
               310                 315                 320
   gtg cag tcc acc gcc tcc agc aag aat gcc agc tgc tac ttt gac atc    1125
   Val Gln Ser Thr Ala Ser Ser Lys Asn Ala Ser Cys Tyr Phe Asp Ile
           325                 330                 335
   gag tgg cgt gac cgg cgc atc aca ctg agg gcg tcc aat ggc aag ttt    1173
   Glu Trp Arg Asp Arg Arg Ile Thr Leu Arg Ala Ser Asn Gly Lys Phe
       340                 345                 350
   gtg acc tcc aag aag aat ggg cag ctg gcc gcc tcg gtg gag aca gca    1221
   Val Thr Ser Lys Lys Asn Gly Gln Leu Ala Ala Ser Val Glu Thr Ala
   355                 360                 365                 370
   ggg gac tca gag ctc ttc ctc atg aag ctc atc aac cgc ccc atc atc    1269
   Gly Asp Ser Glu Leu Phe Leu Met Lys Leu Ile Asn Arg Pro Ile Ile
                   375                 380                 385
   gtg ttc cgc ggg gag cat ggc ttc atc ggc tgc cgc aag gtc acg ggc   1317
   Val Phe Arg Gly Glu His Gly Phe Ile Gly Cys Arg Lys Val Thr Gly
               390                 395                 400
   acc ctg gac gcc aac cgc tcc agc tat gac gtc ttc cag ctg gag ttc   1365
   Thr Leu Asp Ala Asn Arg Ser Ser Tyr Asp Val Phe Gln Leu Glu Phe
           405                 410                 415
   aac gat ggc gcc tac aac atc aaa gac tcc aca ggc aaa tac tgg acg   1413
   Asn Asp Gly Ala Tyr Asn Ile Lys Asp Ser Thr Gly Lys Tyr Trp Thr
       420                 425                 430
   gtg ggc agt gac tcc gcg gtc acc agc agc ggc gac act cct gtg gac   1461
   Val Gly Ser Asp Ser Ala Val Thr Ser Ser Gly Asp Thr Pro Val Asp
   435                 440                 445                 450
   ttc ttc ttc gag ttc tgc gac tat aac aag gtg gcc atc aag gtg ggc   1509
   Phe Phe Phe Glu Phe Cys Asp Tyr Asn Lys Val Ala Ile Lys Val Gly
                   455                 460                 465
   ggg cgc tac ctg aag ggc gac cac gca ggc gtc ctg aag gcc tcg gcg   1557
   Gly Arg Tyr Leu Lys Gly Asp His Ala Gly Val Leu Lys Ala Ser Ala
               470                 475                 480
   gaa acc gtg gac ccc gcc tcg ctc tgg gag tac tagggccggc ccgtccttcc 1610
   Glu Thr Val Asp Pro Ala Ser Leu Trp Glu Tyr
           485                 490
   ccgcccctgc ccacatggcg gctcctgcca accctccctg ctaacccctt ctccgccagg 1670
   tgggctccag ggcgggaggc aagccccctt gcctttcaaa ctggaaaccc cagagaaaac 1730
   ggtgccccca cctgtcgccc ctatggactc cccactctcc cctccgcccg ggttccctac 1790
   tcccctcggg tcagcggctg cggcctggcc ctgggaggga tttcagatgc ccctgccctc 1850
   ttgtctgcca cggggcgagt ctggcacctc tttcttctga cctcagacgg ctctgagcct 1910
   tatttctctg gaagcggcta agggacggtt gggggctggg agccctgggc gtgtagtgta 1970
   actggaatct tttgcctctc ccagccacct cctcccagcc ccccaggaga gctgggcaca 2030
   tgtcccaagc ctgtcagtgg ccctccctgg tgcactgtcc ccgaaacccc tgcttgggaa 2090
   gggaagctgt cgggagggct aggactgacc cttgtggtgt ttttttgggt ggtggctgga 2150
   aacagcccct ctcccacgtg ggagaggctc agcctggctc ccttccctgg agcggcaggg 2210
   cgtgacggcc acagggtctg cccgctgcac gttctgccaa ggtggtggtg gcgggcgggt 2270
   aggggtgtgg gggccgtctt cctcctgtct ctttcctttc accctagcct gactggaagc 2330
   agaaaatgac caaatcagta ttttttttaa tgaaatatta ttgctggagg cgtcccaggc 2390
   aagcctggct gtagtagcga gtgatctggc ggggggcgtc tcagcaccct ccccaggggg 2450
   tgcatctcag ccccctcttt ccgtccttcc cgtccagccc cagccctggg cctgggctgc 2510
   cgacacctgg gccagagccc ctgctgtgat tggtgctccc tgggcctccc gggtggatga 2570
   agccaggcgt cgccccctcc gggagccctg gggtgagccg ccggggcccc cctgctgcca 2630
   gcctcccccg tccccaacat gcatctcact ctgggtgtct tggtctttta ttttttgtaa 2690
   gtgtcatttg tataactcta aacgcccatg atagtagctt caaactggaa atagcgaaat 2750
   aaaataactc agtctgcgcg tg 2772
<210> 28
   <211> 493
   <212> PRT
   <213> Homo sapiens
<400> 28
   Met Thr Ala Asn Gly Thr Ala Glu Ala Val Gln Ile Gln Phe Gly Leu
   1 5 10 15
   Ile Asn Cys Gly Asn Lys Tyr Leu Thr Ala Glu Ala Phe Gly Phe Lys
   20 25 30
   Val Asn Ala Ser Ala Ser Ser Leu Lys Lys Lys Gln Ile Trp Thr Leu
   35 40 45
   Glu Gln Pro Pro Asp Glu Ala Gly Ser Ala Ala Val Cys Leu Arg Ser
   50 55 60
   His Leu Gly Arg Tyr Leu Ala Ala Asp Lys Asp Gly Asn Val Thr Cys
   65 70 75 80
   Glu Arg Glu Val Pro Gly Pro Asp Cys Arg Phe Leu Ile Val Ala His
   85 90 95
   Asp Asp Gly Arg Trp Ser Leu Gln Ser Glu Ala His Arg Arg Tyr Phe
   100 105 110
   Gly Gly Thr Glu Asp Arg Leu Ser Cys Phe Ala Gln Thr Val Ser Pro
   115 120 125
   Ala Glu Lys Trp Ser Val His Ile Ala Met His Pro Gln Val Asn Ile
   130 135 140
   Tyr Ser Val Thr Arg Lys Arg Tyr Ala His Leu Ser Ala Arg Pro Ala
   145 150 155 160
   Asp Glu Ile Ala Val Asp Arg Asp Val Pro Trp Gly Val Asp Ser Leu
   165 170 175
   Ile Thr Leu Ala Phe Gln Asp Gln Arg Tyr Ser Val Gln Thr Ala Asp
   180 185 190
   His Arg Phe Leu Arg His Asp Gly Arg Leu Val Ala Arg Pro Glu Pro
   195 200 205
   Ala Thr Gly Tyr Thr Leu Glu Phe Arg Ser Gly Lys Val Ala Phe Arg
   210 215 220
   Asp Cys Glu Gly Arg Tyr Leu Ala Pro Ser Gly Pro Ser Gly Thr Leu
   225 230 235 240
   Lys Ala Gly Lys Ala Thr Lys Val Gly Lys Asp Glu Leu Phe Ala Leu
   245 250 255
   Glu Gln Ser Cys Ala Gln Val Val Leu Gln Ala Ala Asn Glu Arg Asn
   260 265 270
   Val Ser Thr Arg Gln Gly Met Asp Leu Ser Ala Asn Gln Asp Glu Glu
           275                 280                 285
   Thr Asp Gln Glu Thr Phe Gln Leu Glu Ile Asp Arg Asp Thr Lys Lys
       290                 295                 300
   Cys Ala Phe Arg Thr His Thr Gly Lys Tyr Trp Thr Leu Thr Ala Thr
   305                 310                 315                 320
   Gly Gly Val Gln Ser Thr Ala Ser Ser Lys Asn Ala Ser Cys Tyr Phe
                   325                 330                 335
   Asp Ile Glu Trp Arg Asp Arg Arg Ile Thr Leu Arg Ala Ser Asn Gly
               340                 345                 350
   Lys Phe Val Thr Ser Lys Lys Asn Gly Gln Leu Ala Ala Ser Val Glu
           355                 360                 365
   Thr Ala Gly Asp Ser Glu Leu Phe Leu Met Lys Leu Ile Asn Arg Pro
       370                 375                 380
   Ile Ile Val Phe Arg Gly Glu His Gly Phe Ile Gly Cys Arg Lys Val
   385                 390                 395                 400
   Thr Gly Thr Leu Asp Ala Asn Arg Ser Ser Tyr Asp Val Phe Gln Leu
                   405                 410                 415
   Glu Phe Asn Asp Gly Ala Tyr Asn Ile Lys Asp Ser Thr Gly Lys Tyr
               420                 425                 430
   Trp Thr Val Gly Ser Asp Ser Ala Val Thr Ser Ser Gly Asp Thr Pro
           435                 440                 445
   Val Asp Phe Phe Phe Glu Phe Cys Asp Tyr Asn Lys Val Ala Ile Lys
       450                 455                 460
   Val Gly Gly Arg Tyr Leu Lys Gly Asp His Ala Gly Val Leu Lys Ala
   465                 470                 475                 480
   Ser Ala Glu Thr Val Asp Pro Ala Ser Leu Trp Glu Tyr
                   485                 490
<210> 29
   <211> 2634
   <212> DNA
   <213> Mus musculus
<220>
   <221> CD5
   <222> (79)..(1557)
<400> 29
   gagctaagcc gtgttgaaca aaggaggtcg ggcacagcta tccaagctcc cggggccacc 60
   gggccgccct ccgccacc atg acc gcc aac ggc acg gca gag gct gtg cag   111
                       Met Thr Ala Asn Gly Thr Ala Glu Ala Val Gln
                        1               5                  10
   att cag ttc ggg ctc atc agc tgc ggc aac aag tac ctg aca gcc gag   159
   Ile Gln Phe Gly Leu Ile Ser Cys Gly Asn Lys Tyr Leu Thr Ala Glu
               15                  20                  25
   gcg ttc ggg ttc aag gtg aac gca tcc gct agt agc ttg aaa aag aag    207
   Ala Phe Gly Phe Lys Val Asn Ala Ser Ala Ser Ser Leu Lys Lys Lys
           30                  35                  40
   cag atc tgg acg ctg gag caa cct ccc gat gag gcg ggc agc gcg gcc    255
   Gln Ile Trp Thr Leu Glu Gln Pro Pro Asp Glu Ala Gly Ser Ala Ala
       45                  50                  55
   gtg tgt ctg cgc acg cac ctg ggt cgc tac ctg gcc gcc gac aag gac    303
   Val Cys Leu Arg Thr His Leu Gly Arg Tyr Leu Ala Ala Asp Lys Asp
   60                  65                  70                  75
   ggc aac gtg acc tgc gag cgc gag gtg ccc gac ggc gac tgc cgc ttt    351
   Gly Asn Val Thr Cys Glu Arg Glu Val Pro Asp Gly Asp Cys Arg Phe
                   80                  85                  90
   ctc gtc gtg gcg cac gac gac ggc cgc tgg tcg ctg cag tcc gag gct    399
   Leu Val Val Ala His Asp Asp Gly Arg Trp Ser Leu Gln Ser Glu Ala
               95                  100                 105
   cac cgg cgc tac ttt ggc ggc acc gag gac cgc ctg tcc tgc ttc gcg    447
   His Arg Arg Tyr Phe Gly Gly Thr Glu Asp Arg Leu Ser Cys Phe Ala
           110                 115                 120
   cag agc gtg tcg ccg gcc gag aag tgg agc gtg cac atc gcc atg cac    495
   Gln Ser Val Ser Pro Ala Glu Lys Trp Ser Val His Ile Ala Met His
       125                 130                 135
   ccg cag gtt aac atc tac agc gtt acc cgc aag cgc tac gcg cat ctg    543
   Pro Gln Val Asn Ile Tyr Ser Val Thr Arg Lys Arg Tyr Ala His Leu
   140                 145                 150                 155
   agc gcg cgg ccg gcc gac gag atc gcg gta gac cgc gac gtg cct tgg    591
   Ser Ala Arg Pro Ala Asp Glu Ile Ala Val Asp Arg Asp Val Pro Trp
                   160                 165                 170
   ggc gtc gac tcg ctc atc acc ttg gcc ttc cag gac caa cgc tac agt    639
   Gly Val Asp Ser Leu Ile Thr Leu Ala Phe Gln Asp Gln Arg Tyr Ser
               175                 180                 185
   gtg cag acg tcc gac cac cgc ttc ctg cgc cac gac ggg cgc ctt gtg    687
   Val Gln Thr Ser Asp His Arg Phe Leu Arg His Asp Gly Arg Leu Val
           190                 195                 200
   gca cgg ccg gag ccc gcc acg ggc ttc acg ctg gag ttc cgc tcc ggc    735
   Ala Arg Pro Glu Pro Ala Thr Gly Phe Thr Leu Glu Phe Arg Ser Gly
       205                 210                 215
   aag gtg gcc ttt cgc gac tgc gaa ggt cgc tac ctg gct ccg tcc ggg    783
   Lys Val Ala Phe Arg Asp Cys Glu Gly Arg Tyr Leu Ala Pro Ser Gly
   220                 225                 230                 235
   ccc agc ggc acc ctc aag gct ggc aag gcc acc aag gtg ggc aaa gat    831
   Pro Ser Gly Thr Leu Lys Ala Gly Lys Ala Thr Lys Val Gly Lys Asp
                   240                 245                 250
   gag ctc ttc gcc ctg gaa cag agc tgc gct gag gtg gtg ctg cag gcg    879
   Glu Leu Phe Ala Leu Glu Gln Ser Cys Ala Glu Val Val Leu Gln Ala
               255                 260                 265
   gcc aac gag ggg aac gtg tcc acg cgc cag gga atg gac ctg tca gcc    927
   Ala Asn Glu Gly Asn Val Ser Thr Arg Gln Gly Met Asp Leu Ser Ala
           270                 275                 280
   aat cag gat gaa gag acc gat cag gag acc ttc cag ctg gag atc gac    975
   Asn Gln Asp Glu Glu Thr Asp Gln Glu Thr Phe Gln Leu Glu Ile Asp
       285                 290                 295
   cgc gac aca aga aag tgt gcc ttt cgc acc cac acg ggc aag tac tgg   1023
   Arg Asp Thr Arg Lys Cys Ala Phe Arg Thr His Thr Gly Lys Tyr Trp
   300                 305                 310                 315
   aca ctg acg gcg acc gga ggt gtg caa tcc act gcg tcc acc aag aac   1071
   Thr Leu Thr Ala Thr Gly Gly Val Gln Ser Thr Ala Ser Thr Lys Asn
                   320                 325                 330
   gcc agc tgc tac ttt gac atc gag tgg tgt gac cgc cgg atc act ctg   1119
   Ala Ser Cys Tyr Phe Asp Ile Glu Trp Cys Asp Arg Arg Ile Thr Leu
               335                 340                 345
   aga gcc tcc aac ggc aag ttt gtg acc gcc aag aaa aat ggc cac gtg   1167
   Arg Ala Ser Asn Gly Lys Phe Val Thr Ala Lys Lys Asn Gly His Val
           350                 355                 360
   gcc gcc tcg gtg gag aca gca ggg gac tcg gaa ctc ttc ctc atg aag   1215
   Ala Ala Ser Val Glu Thr Ala Gly Asp Ser Glu Leu Phe Leu Met Lys
       365                 370                 375
   ctg att aac cgc ccc atc att gcg ttc cgg ggg gaa cac ggg ttc att   1263
   Leu Ile Asn Arg Pro Ile Ile Ala Phe Arg Gly Glu His Gly Phe Ile
   380                 385                 390                 395
   gcg tgc cgc aag gtc acg ggc act ctg gat gcc aac cgt tcc agt tac   1311
   Ala Cys Arg Lys Val Thr Gly Thr Leu Asp Ala Asn Arg Ser Ser Tyr
                   400                 405                 410
   gat gtc ttc cag ttg gaa ttc aat gac ggc gcc tac aac atc aaa gac   1359
   Asp Val Phe Gln Leu Glu Phe Asn Asp Gly Ala Tyr Asn Ile Lys Asp
               415                 420                 425
   tcc acg ggc aag tac tgg acg gtg ggt agt gat tcc tcg gtc acc agc   1407
   Ser Thr Gly Lys Tyr Trp Thr Val Gly Ser Asp Ser Ser Val Thr Ser
           430                 435                 440
   agc agc gac acc cct gtg gat ttc ttc ctt gag ttc tgt gac tac aat   1455
   Ser Ser Asp Thr Pro Val Asp Phe Phe Leu Glu Phe Cys Asp Tyr Asn
       445                 450                 455
   aag gtg gct ctc aag gtg ggc ggc cgc tac ctg aag ggg gac cac gct   1503
   Lys Val Ala Leu Lys Val Gly Gly Arg Tyr Leu Lys Gly Asp His Ala
   460                 465                 470                 475
   ggg gtc ctg aag gcc tgc gcg gag act atc gac ccc gcc tca ctc tgg   1551
   Gly Val Leu Lys Ala Cys Ala Glu Thr Ile Asp Pro Ala Ser Leu Trp
                   480                 485                 490
   gag tac tagggccacc tgcctctgca gccgctctcg tcagtcctcc tgttatcctt    1607
   Glu Tyr
   actcatcggg tggcctgcag caggtggcaa accccttgcc tttcaaactg gaaacccaag 1667
   agaaaacggt gcccttgctg tcaccctctg tggacccctt ttccctaact cactgctccc 1727
   catgggtcgg tggctgcaga ctgtccccag gaggactctg gttccctctg tccccttctt 1787
   tccatgggga actctggcac ctttcttctg acctcagtca actctgagcc ttatttcccc 1847
   ccaggaagtg gcctaggaga agctacaggg cctagggact taccctgagc ttgtaactgg 1907
   aagaccccgt ccctatcccc gctcccgccc ccaccccacc ccacccctgc tctggcccca 1967
   gcctctggag gccagccttt tggcgggact gaagccgggc atggccaacc ttgcccacaa 2027
   gtgtttttct ggatcttggc tggaaggcag tctgtcccat cctgcagtgt ttgggcctgg 2087
   ctctttgact caaagctagc taggtggcac tccgtgtcgc tcctgcacat tctggaaggg 2147
   gcgggcctct cacccacctc attccttttc cccctggcct gactggaagc agaaaaatga 2207
   ccaaatcagt attttttttt tttctttaag gaaatgttac tgttgaaagg ccctaggcaa 2267
   gcctgccctg ttggttgtag tcgtgagtgg tcttgggggg agatgcttgg ctcctgtccc 2327
   tgcctcccca gcggttccct ccctccctcc tgcctgacca ccccagctct ggctctgtga 2387
   ttggtgctcc acgtctccag acacctcggg gctcctgggc ggagaaagcc gatgtgcccc 2447
   tccctgggag ccctgagtaa acctcagggg gccctttccc aatcacccct ccaccgaccc 2507
   ctcaacacca tgcatctcac tctgggtgta ctcgctcaca tttatttttt tgtaactgtc 2567
   atttctataa ctctgaagac ccatgatagt aagctttgaa ctggaaaata aagtaaaatc 2627
   aagtctg 2634
<210> 30
   <211> 493
   <212> PRT
   <213> Mus musculus
<400> 30
   Met Thr Ala Asn Gly Thr Ala Glu Ala Val Gln Ile Gln Phe Gly Leu
    1               5                  10                  15
   Ile Ser Cys Gly Asn Lys Tyr Leu Thr Ala Glu Ala Phe Gly Phe Lys
               20                  25                  30
   Val Asn Ala Ser Ala Ser Ser Leu Lys Lys Lys Gln Ile Trp Thr Leu
           35                  40                  45
   Glu Gln Pro Pro Asp Glu Ala Gly Ser Ala Ala Val Cys Leu Arg Thr
       50                  55                  60
   His Leu Gly Arg Tyr Leu Ala Ala Asp Lys Asp Gly Asn Val Thr Cys
   65                  70                  75                  80
   Glu Arg Glu Val Pro Asp Gly Asp Cys Arg Phe Leu Val Val Ala His
                   85                  90                  95
   Asp Asp Gly Arg Trp Ser Leu Gln Ser Glu Ala His Arg Arg Tyr Phe
               100                 105                 110
   Gly Gly Thr Glu Asp Arg Leu Ser Cys Phe Ala Gln Ser Val Ser Pro
           115                 120                 125
   Ala Glu Lys Trp Ser Val His Ile Ala Met His Pro Gln Val Asn Ile
       130                 135                 140
   Tyr Ser Val Thr Arg Lys Arg Tyr Ala His Leu Ser Ala Arg Pro Ala
   145                 150                 155                 160
   Asp Glu Ile Ala Val Asp Arg Asp Val Pro Trp Gly Val Asp Ser Leu
                   165                 170                 175
   Ile Thr Leu Ala Phe Gln Asp Gln Arg Tyr Ser Val Gln Thr Ser Asp
               180                 185                 190
   His Arg Phe Leu Arg His Asp Gly Arg Leu Val Ala Arg Pro Glu Pro
           195                 200                 205
   Ala Thr Gly Phe Thr Leu Glu Phe Arg Ser Gly Lys Val Ala Phe Arg
       210                 215                 220
   Asp Cys Glu Gly Arg Tyr Leu Ala Pro Ser Gly Pro Ser Gly Thr Leu
   225                 230                 235                 240
   Lys Ala Gly Lys Ala Thr Lys Val Gly Lys Asp Glu Leu Phe Ala Leu
                   245                 250                 255
   Glu Gln Ser Cys Ala Glu Val Val Leu Gln Ala Ala Asn Glu Gly Asn
               260                 265                 270
   Val Ser Thr Arg Gln Gly Met Asp Leu Ser Ala Asn Gln Asp Glu Glu
           275                 280                 285
   Thr Asp Gln Glu Thr Phe Gln Leu Glu Ile Asp Arg Asp Thr Arg Lys
       290                 295                 300
   Cys Ala Phe Arg Thr His Thr Gly Lys Tyr Trp Thr Leu Thr Ala Thr
   305                 310                 315                 320
   Gly Gly Val Gln Ser Thr Ala Ser Thr Lys Asn Ala Ser Cys Tyr Phe
                   325                 330                 335
   Asp Ile Glu Trp Cys Asp Arg Arg Ile Thr Leu Arg Ala Ser Asn Gly
               340                 345                 350
   Lys Phe Val Thr Ala Lys Lys Asn Gly His Val Ala Ala Ser Val Glu
           355                 360                 365
   Thr Ala Gly Asp Ser Glu Leu Phe Leu Met Lys Leu Ile Asn Arg Pro
       370                 375                 380
   Ile Ile Ala Phe Arg Gly Glu His Gly Phe Ile Ala Cys Arg Lys Val
   385                 390                 395                 400
   Thr Gly Thr Leu Asp Ala Asn Arg Ser Ser Tyr Asp Val Phe Gln Leu
                   405                 410                 415
   Glu Phe Asn Asp Gly Ala Tyr Asn Ile Lys Asp Ser Thr Gly Lys Tyr
               420                 425                 430
   Trp Thr Val Gly Ser Asp Ser Ser Val Thr Ser Ser Ser Asp Thr Pro
           435                 440                 445
   Val Asp Phe Phe Leu Glu Phe Cys Asp Tyr Asn Lys Val Ala Leu Lys
       450                 455                 460
   Val Gly Gly Arg Tyr Leu Lys Gly Asp His Ala Gly Val Leu Lys Ala
   465                 470                 475                 480
   Cys Ala Glu Thr Ile Asp Pro Ala Ser Leu Trp Glu Tyr
   485 490
<210> 31
   <211> 20
   <212> DNA
   <213> Artificial Sequence
<220>
   <223> Description of Artificial Sequence: artificial sequence
<400> 31
   gccaccatga ccgccaacgg 20
<210> 32
   <211> 20
   <212> DNA
   <213> Artificial Sequence
<220>
   <223> Description of Artificial Sequence: artificial sequence
<400> 32
   tgtgtcgcgg tcgatctcca 20
<210> 33
   <211> 4100
   <212> DNA
   <213> Homo sapiens
<400> 33
   aagcttctag aaggggtata ttttgccccc aggccccaaa tcctggtctt tggactacag 60
   tcaagaccta gcacagggct caggcctaaa aaaaagctgt tgaggagggt gtgaggctgc 120
   aacgttgcgg tgagaagggg gtccccaggg agggcgcagc aggaagcccc agggaagtgc 180
   ctgggagagg gaggttgtgc agccagaagg agcagcccgc agcctttggt tgttgactcc 240
   tgctttgtaa gtggcagtct ggttgggtag gacagggtcc gagtcctcac tcagggaact 300
   gagtccaggg tgagctgagc agcccttcct ggtggcctca ctttcccctg cagagcctgc 360
   tgcatggtgt ccagtggcca gcctgggagg agctcaggac tggcctcacc tcgtgccacg 420
   ccctcgtacc aaggtggctc agatggcacc tgggttcccg aagggcccag gaacacagcg 480
   tccatgtccc catccttccc cggagggact tggggtgggg cctgcaggaa ggatcatgtg 540
   acttgttcaa gggcagcttg tcctgccccg gacacagagg tgcccatcgt aagcgagatg 600
   caggcaaggt gaggacaggg catggtgccg agcaggaatg attttcgaaa atgctactct 660
   gtggtcgggc acggtggctc actcctgtaa tcccagtact ttgggaggcc caggcgggca 720
   gatcatgagg tcaggagttc aagaccagcc tggccaacat ggtgaaaccc cttctctact 780
   gaaaatacaa aaaactaccc gggcgtggtg gtggatgcct gtaatcccag ctactcagga 840
   ggctgaggca ggagaatcgc ttgaaccctg gaggtgaagg ttgcaatgag ccgagattgc 900
   accactgcac tccgacctgg gcgacagaga aagactccgt ctgaaaaaca aacaaacaaa 960
   accccgagat tctcttttcc cccgtccgga gctctatggc catctggagc tcgttctgtc 1020
   cacaaggaca catttcctgg cagcagctgt ggaccagggc tcactcactc acatctgtac 1080
   ccaatctaga gcaggacaaa cacctatcac ctgcactggc aatggacaga ggacagtggc 1140
   agccccatca ccagaggcat tcaagccaag gcattttcta tcgtctattt atctatctat 1200
   ctatctatct atctatctat ctatctatct atctatctag atgagatctt gctatgttgc 1260
   ccaggttgaa ctcctggcct caagcgatcc tcctgcttca gcctcccaaa gtgttgggat 1320
   ttcaggtgtg aaccactgta cctggctgga gtgcagtggc actgtcatag ctcactgcag 1380
   cctccacctc ctgggctcaa ggaatcctct ttcctcagcc tcctgagtag ctgggaccac 1440
   aggcatgcac catcacaccc agctaaacct tgattttttc catagtatat ggctcagggc 1500
   agagcaaaga agcacaaaaa catgttggct tgcggttgag ggctgatggg agggggttct 1560
   ggccaaagcc caaaattaac agccacaacg tcagtgtctg tgggaggggt tgccaggggc 1620
   gtgcgggttc tggggctcaa ggccctgccg gtaaacccat ttgaagcagg atggcaagaa 1680
   ggtgacacca tcttcccccg cgcactgaaa gcccctggct gggattgcct ggggcagaca 1740
   caggctcgga ccagccccag caatcccagt ttatcagcga gccggctgag ggcccggagt 1800
   tatctcagtg cccggcctga gaccttgtgg gcagcctgtg gtcatgctgg cattccaggg 1860
   gccttttggc atgtggggaa tgtccaggaa aagcctcagc cttcggtgag gcgcagaaaa 1920
   gggaagtgtc cctagagggg gtgggtgagg gcgtgggagg tggtgtctgc agggaatgtc 1980
   ccctttgggg gaggaggatg gagggttggg attctgagga tggggggggg ggctgtagcc 2040
   agcaccatgt ccctcctgtg tgaccagctc agagtcccat gaaattgggg cttgggaggg 2100
   gaagggacac tggcctggga accagagacc tgggctggtc tggctcacag ttgctggacc 2160
   tctgtgatcc gtgtcaaaaa acgagaacac caattcctgt cctgcccact caccaccagg 2220
   tgggacctga accctggcat cgccagcatt gggaatgtcg gccactgact caaccactct 2280
   cccggagacc tatttgggcc acccgaggcg ggtgcctggg ccacacggag gggtcctggt 2340
   ggtcttcagg gcagcggctg tggggctgaa gcctcaagga accacatctc tgcataggag 2400
   ggccaggctg cagggcctcg ggagacaacc tagttgggcg tgttggggtc tgtgggtccc 2460
   aggtcctggc ctcaccgggt ccccaccgcg ctgtcagctc ccagcctctt tccctcgtct 2520
   gcctctgggc ttctgtaagg ctatgtgctc caaggccacc tcctccaggc agccctcaga 2580
   cccccacctt cgcccagtac cgatctgcac cgtggtctct gaagtctcca tcgtgacctc 2640
   aaacctcgct cgtccttgct cctagcgagg cttggggtcg gggtgtccga ggtgggggac 2700
   atccgggggg gttaggtggc tggcgcgggg agccggggtt gtgaggggtg atgtcctcag 2760
   gcggcggcgc tgcggggtgc ggcgaggaca ccggtggggt gagagcaccg gcggggcagc 2820
   agcgggggcc gcagcgccgg gtccctcggc ccggggcccc tcccgcgcgg agccaggggc 2880
   gggacagggg ggcgtggcct ggtggcgctg acgtcacctc gcctataaaa tgtccggggc 2940
   gccgctagct gggctttgtg gagcgctgcg gagggtgcgt gcgggccgcg gcagccgaac 3000
   aaaggagcag gggcgccgcc gcagggaccc gccacccacc tcccggggcc gcgcagcggc 3060
   ctctcgtcta ctgccaccat gaccgccaac ggcacagccg aggcggtgca gatccagttc 3120
   ggcctcatca actgcggcaa caagtacctg acggccgagg cgttcgggtt caaggtgaac 3180
   gcgtccgcca gcagcctgaa gaagaagcag atctggacgc tggagcagcc ccctgacgag 3240
   gcgggcagcg cggccgtgtg cctgcgcagc cacctgggcc gctacctggc ggcggacaag 3300
   gacggcaacg tgacctgcga gcgcgaggtg cccggtcccg actgccgttt cctcatcgtg 3360
   gcgcacgacg acggtcgctg gtcgctgcag tccgaggcgc accggcgcta cttcggcggc 3420
   accgaggacc gcctgtcctg cttcgcgcag acgggtcccc cgccgagaag tggagcgtgc 3480
   acatcgccat gcaccctcag gtcaacatct acagcgtcac ccgtaagcgc tacgcgcacc 3540
   tgagcgcgcg gccggccgac gagatcgccg tggaccgcga cgtgccctgg ggcgtcgact 3600
   cgctcatcac cctcgccttc caggaccagc gctacagcgt gcagaccgcc gaccaccgct 3660
   tcctgcgcca cgacgggcgc ctggtggcgc gccccgagcc ggccactggc tacacgctgg 3720
   agttccgctc cggcaaggtg gccttccgcg actgcgaggg ccgttacctg gcgccgtcgg 3780
   ggcccagcgg cacgctcaag gcgggcaagg ccaccaaggt gggcaaggac gagctctttg 3840
   ctctggagca gagctgcgcc caggtcgtgc tgcaggcggc caacgagagg aacgtgtcca 3900
   cgcgccaggg tgagtgggga cgctgccccc gcctctcctg gtccgtgcac aaagcgcacc 3960
   ccacccgcgc ccctccagcc tcccgccctt tctcgctcgc ggcgccgctg cggtccggag 4020
   cactgcccat tgcgcccccg ctaggcacgc gggctacccc gcctggaggg ggcgaggagt 4080
   ggggctttgc ccatccgcgg 4100
<210> 34
   <211> 2843
   <212> DNA
   <213> Homo sapiens
<400> 34
   caatcctcct gccttgggcc tcccaaagtg ttgcgatcac aggcatgagc ccctgtccct 60
   ggcccctatt atttctctaa ctggggaaag tcatgtggga acagatgtag cttgccttgg 120
   cctctgaccg gccctgcctg cgttcctggg tgctctctgc tgcttctcat gtgtgccact 180
   gtggggactc ggccgcccac cccaccccgt ggtgttacct tgcgtgtgta gttctgtgag 240
   ctcagggcta tggtctgcca gaactagggg gcgtggggcc ccagtaccag cccaaggcct 300
   cctctctgca ggtatggacc tgtctgccaa tcaggacgag gagaccgacc aggagacctt 360
   ccagctggag atcgaccgcg acaccaaaaa gtgtgccttc cgtacccaca cgggcaagta 420
   ctggacgctg acggccaccg ggggcgtgca gtccaccgcc tccagcaagt gagtgcctcg 480
   ctcccacctg tcaccgcccc caccaccttg cctgggctac cccgcctgac cctgtcccgc 540
   catcccccag gaatgccagc tgctactttg acatcgagtg gcgtgaccgg cgcatcacac 600
   tgagggcgtc caatggcaag tttgtgacct ccaagaagaa tgggcagctg gccgcctcgg 660
   tggagacagc aggtaacact aaagccccag ttccctggag ccgtcctgga gtcctggagg 720
   gtctggccat gccgtggtca cttggtagcc ccagccaagg cctgctctgt gctgggcatc 780
   cccccggact ggccccgcac tgtcctaccc tggggactgc tgtgtgaccc cagctcctgg 840
   ccctccctct ctggtcaccc cagcctccac cccactccct gccaggaggc tcactgactc 900
   ccctctttct gggacagggg actcagagct cttcctcatg aagctcatca accgccccat 960
   catcgtgttc cgcggggagc atggcttcat cggctgccgc aaggtcacgg gcaccctgga 1020
   cgccaaccgc tccagctatg acgtcttcca gctggagttc aacgatggcg cctacaacat 1080
   caaaggcagg ttctcctgtg ggcagctgct gggcagggaa cccctcggtc ggggctgggg 1140
   tcagtgctgc ggggagcgcc ctctgcatcc acactggacc ctggcttggc tcagggccat 1200
   tccaggccct aaagggacag gtgtctgatg gccaccaggg ggctctggga tgcaagcagc 1260
   ccctttccct cttgtctgtg tggttggggg gacttacctt gcccacctga cagagaggtg 1320
   tgtggagggg agagcaggga gggaaggaga ccaggaaagg gagggaggag agcaggggag 1380
   gggagagccg ggaagagagg agagcagggg tggggaggtt tctggaaagg gtgtgcaagg 1440
   ggaggacgcg cctcggttat gggactggag ccccttccca ggaggacccc caacaatcca 1500
   gaggtgcctg ttaggattca gaacatggtt tttttgtttg tttttttgag actcactccc 1560
   tcaccctggc tggacttgca gtggcgttat ctcggctcac tgcaacctct gcctcctggg 1620
   ttcaagtgat tctcctgcct cagcctccca agtagctggg attacaggtg tgcaccacca 1680
   cgcctggcta atttttatat ttttaaaatt tattatttat ttattttgag acccagtctg 1740
   gagtgcagtg gcgttatctc ggctctctgc aacctctgcc tcctgggttc aagcgattct 1800
   cctgcctcag cctcccgagt agctgggact atgtgtggga gccaccatgc ctggctaatt 1860
   tttttgtatt tttcatagag acgggtttca ccatgttgtc caggctggtc ttgaattcgt 1920
   ggcctcaagt gatccgccca cctcagcctc ccacagtgct gggtttatag gtgtgagcca 1980
   ccacacccgg ctaattgttt tgtattttta gtagagacgg agcttcacta tgttggcaag 2040
   gctggctcga actcctgacc tcaagtgatc cgcccacctc agcctcccaa agtgctggga 2100
   ttacaggcgt gagccactgc ggccgagcag aacacgttct aggacccttg ttcatgtgtc 2160
   catcatggac aggaggacgt gcgggccata gggaccctgg ctcattccgg agccgggact 2220
   ggagggtggg gcgtcaccct tgggaacacc cgtgcccacc ctccgctgcc cagggtaggg 2280
   gtggggagcc aggctttggg ccccacttga taaagtcccc tccccagact ccacaggcaa 2340
   atactggacg gtgggcagtg actccgcggt caccagcagc ggcgacactc ctgtggactt 2400
   cttcttcgag ttctgcgact ataacaaggt ggccatcaag gtgggcgggc gctacctgaa 2460
   gggcgaccac gcaggcgtcc tgaaggcctc ggcggaaacc gtggaccccg cctcgctctg 2520
   ggagtactag ggccggcccg tccttccccg cccctgccca catggcggct cctgccaacc 2580
   ctccctgcta accccttctc cgccaggtgg gctccagggc gggaggcaag cccccttgcc 2640
   tttcaaactg gaaaccccag agaaaacggt gcccccacct gtcgccccta tggactcccc 2700
   actctcccct ccgcccgggt tccctactcc cctcgggtca gcggctgcgg cctggccctg 2760
   ggagggattt cagatgcccc tgccctcttg tctgccacgg ggcgagtctg gcacctcttt 2820
   cttctgacct cagacggctc tga                                         2843
<210> 35
   <211> 934
   <212> DNA
   <213> Homo sapiens
<400> 35
   ccacctcctc ccagcccccc aggagagctg ggcacatgtc ccaagcctgt cagtggccct 60
   ccctggtgca ctgtccccga aacccctgct tgggaaggga agctgtcggg tgggctagga 120
   ctgacccttg tggtgttttt ttgggtggtg gctggaaaca gcccctctcc cacgtggcag 180
   aggctcagcc tggctccctt ccctggagcg gcagggcgtg acggccacag ggtctgcccg 240
   ctgcacgttc tgccaaggtg gtggtggcgg gcgggtaggg gtgtgggggc cgtcttcctc 300
   ctgtctcttt cctttcaccc tagcctgact ggaagcagaa aatgaccaaa tcagtatttt 360
   ttttaatgaa atattattgc tggaggcgtc ccaggcaagc ctggctgtag tagcgagtga 420
   tctggcgggg ggcgtctcag caccctcccc agggggtgca tctcagcccc ctctttccgt 480
   ccttcccgtc cagccccagc cctgggcctg ggctgccgac acctgggcca gagcccctgc 540
   tgtgattggt gctccctggg cctcccgggt ggatgaagcc aggcgtcgcc ccctccggga 600
   gccctggggt gagccgccgg ggcccccctg ctgccagcct cccccgtccc caacatgcat 660
   ctcactctgg gtgtcttggt cttttatttt ttgtaagtgt catttgtata actctaaacg 720
   cccatgatag tagcttcaaa ctggaaatag cgaaataaaa taactcagtc tgcagcccca 780
   ggccggcctg tgtgtgtctt ggggctgagg tgggtggggg ggctgaggtg ggtgggaggg 840
   ctggcgggac aggtaggcgc cctggctccc cagctcagtg ctgggagtgt gcagtgggag 900
   ggaggccgtg gctccagtgg gtgctccgga gctc                              934
<210> 36
   <211> 20
   <212> DNA
   <213> Artificial Sequence
<220>
   <223> Description of Artificial Sequence: artificial sequence
<400> 36
   aagcttctag aaggggtata                                              20
<210> 37
   <211> 20
   <212> DNA
   <213> Artificial Sequence
<220>
   <223> Description of Artificial Sequence: artificial sequence
<400> 37
   agacgagagg ccgctgcgcg                                              20
<210> 38
   <211> 20
   <212> DNA
   <213> Artificial Sequence
<220>
   <223> Description of Artificial Sequence: artificial sequence
<400> 38
   gtggcagccc catcaccaga                                              20
<210> 39
   <211> 20
   <212> DNA
   <213> Artificial Sequence
<220>
   <223> Description of Artificial Sequence: artificial sequence
<400> 39
   catcacaccc agctaaacct 20
<210> 40
   <211> 20
   <212> DNA
   <213> Artificial Sequence
<220>
   <223> Description of Artificial Sequence: artificial sequence
<400> 40
   gtgcgggttc tggggctcaa 20
<210> 41
   <211> 20
   <212> DNA
   <213> Artificial Sequence
<220>
   <223> Description of Artificial Sequence: artificial sequence
<400> 41
   ggcagcctgt ggtcatgctg 20
<210> 42
   <211> 20
   <212> DNA
   <213> Artificial Sequence
<220>
   <223> Description of Artificial Sequence: artificial sequence
<400> 42
   gacctgggct ggtctggctc 20
<210> 43
   <211> 20
   <212> DNA
   <213> Artificial Sequence
<220>
   <223> Description of Artificial Sequence: artificial sequence
<400> 43
   gcagggcctc gggagacaac 20
<210> 44
   <211> 20
   <212> DNA
   <213> Artificial Sequence
<220>
   <223> Description of Artificial Sequence: artificial sequence
<400> 44
   catccggggg ggttaggtgg 20
<210> 45
   <211> 18
   <212> DNA
   <213> Artificial Sequence
<220>
   <223> Description of Artificial Sequence: artificial sequence
<400> 45
   gtgagtgggg acgctgcc 18
<210> 46
   <211> 18
   <212> DNA
   <213> Artificial Sequence
<220>
   <223> Description of Artificial Sequence: artificial sequence
<400> 46
   ccgcggatgg gcaaagcc 18
<210> 47
   <211> 20
   <212> DNA
   <213> Artificial Sequence
<220>
   <223> Description of Artificial Sequence: artificial sequence
<400> 47
   gtgtcagccc ttccccccag 20
<210> 48
   <211> 20
   <212> DNA
   <213> Artificial Sequence
<220>
   <223> Description of Artificial Sequence: artificial sequence
<400> 48
   gagctcggat cctccccttc 20
<210> 49
   <211> 20
   <212> DNA
   <213> Artificial Sequence
<220>
   <223> Description of Artificial Sequence: artificial sequence
<400> 49
   gggcccggtg ggggcagggt 20
<210> 50
   <211> 20
   <212> DNA
   <213> Artificial Sequence
<220>
   <223> Description of Artificial Sequence: artificial sequence
<400> 50
   caggtcctat ccctggcatg 20
<210> 51
   <211> 20
   <212> DNA
   <213> Artificial Sequence
<220>
   <223> Description of Artificial Sequence: artificial sequence
<400> 51
   ctgcagtgag ccgagatcgt 20
<210> 52
   <211> 20
   <212> DNA
   <213> Artificial Sequence
<220>
   <223> Description of Artificial Sequence: artificial sequence
<400> 52
   ggtacctctc cctcctcctt 20
<210> 53
   <211> 20
   <212> DNA
   <213> Artificial Sequence
<220>
   <223> Description of Artificial Sequence: artificial sequence
<400> 53
   gggactgagt cgtggtacag 20
<210> 54
   <211> 20
   <212> DNA
   <213> Artificial Sequence
<220>
   <223> Description of Artificial Sequence: artificial sequence
<400> 54
   gctcaaagga tcctcttgcc 20
<210> 55
   <211> 20
   <212> DNA
   <213> Artificial Sequence
<220>
   <223> Description of Artificial Sequence: artificial sequence
<400> 55
   ctgcagcctc cgcctcctgg 20
<210> 56
   <211> 20
   <212> DNA
   <213> Artificial Sequence
<220>
   <223> Description of Artificial Sequence: artificial sequence
<400> 56
   gccgaggcgg gcggatcatg 20
<210> 57
   <211> 20
   <212> DNA
   <213> Artificial Sequence
<220>
   <223> Description of Artificial Sequence: artificial sequence
<400> 57
   ctgcagaaca cccctgtgtt 20
<210> 58
   <211> 20
   <212> DNA
   <213> Artificial Sequence
<220>
   <223> Description of Artificial Sequence: artificial sequence
<400> 58
   caaggctggt cttgaactca 20
<210> 59
   <211> 20
   <212> DNA
   <213> Artificial Sequence
<220>
   <223> Description of Artificial Sequence: artificial sequence
<400> 59
   caatcctcct gccttgggcc 20
<210> 60
   <211> 20
   <212> DNA
   <213> Artificial Sequence
<220>
   <223> Description of Artificial Sequence: artificial sequence
<400> 60
   ctgcagagag gaggccttgg 20
<210> 61
   <211> 20
   <212> DNA
   <213> Artificial Sequence
<220>
   <223> Description of Artificial Sequence: artificial sequence
<400> 61
   gtgagtgcct cgctcccacc 20
<210> 62
   <211> 20
   <212> DNA
   <213> Artificial Sequence
<220>
   <223> Description of Artificial Sequence: artificial sequence
<400> 62
   ctgggggatg gcgggacagg 20
<210> 63
   <211> 20
   <212> DNA
   <213> Artificial Sequence
<220>
   <223> Description of Artificial Sequence: artificial sequence
<400> 63
   gtaacactaa agccccagtt 20
<210> 64
   <211> 20
   <212> DNA
   <213> Artificial Sequence
<220>
   <223> Description of Artificial Sequence: artificial sequence
<400> 64
   ctgtcccaga aagaggggag 20
<210> 65
   <211> 20
   <212> DNA
   <213> Artificial Sequence
<220>
   <223> Description of Artificial Sequence: artificial sequence
<400> 65
   gcaggttctc ctgtgggcag 20
<210> 66
   <211> 20
   <212> DNA
   <213> Artificial Sequence
<220>
   <223> Description of Artificial Sequence: artificial sequence
<400> 66
   ctggggaggg gactttatca 20
<210> 67
   <211> 20
   <212> DNA
   <213> Artificial Sequence
<220>
   <223> Description of Artificial Sequence: artificial sequence
<400> 67
   agccccaggc cggcctgtgt 20
<210> 68
   <211> 20
   <212> DNA
   <213> Artificial Sequence
<220>
   <223> Description of Artificial Sequence: artificial sequence
<400> 68
   gagctccgga gcacccactg 20
<210> 69
   <211> 25
   <212> DNA
   <213> Artificial Sequence
<220>
   <223> Description of Artificial Sequence: artificial sequence
<400> 69
   aagcttctag aaggggtata ttttg 25
<210> 70
   <211> 19
   <212> DNA
   <213> Artificial Sequence
<220>
   <223> Description of Artificial Sequence: artificial sequence
<400> 70
   cgttcacctt gaacccgaa 19
<210> 71
   <211> 21
   <212> DNA
   <213> Artificial Sequence
<220>
   <223> Description of Artificial Sequence: artificial sequence
<400> 71
   gcagccgaac aaaggagcag g 21
<210> 72
   <211> 16951
   <212> DNA
   <213> Homo sapiens
<400> 72